# EUROPEAN PATENT APPLICATION

(11) **EP 4 755 890 A1**
(43) Date of publication of application: **10.06.2026**
(21) Application number: 24848337.2
(22) Date of filing: 01.08.2024
(51) Int. Cl.: C07D 471/14, C07D 487/04, A61K 31/519, A61P 35/00

(54) **OXO-TRICYCLIC DERIVATIVE AND PHARMACEUTICAL USE THEREOF**

(30) Priority: 01.08.2023 CN 202310958679; 11.12.2023 CN 202311691809; 16.01.2024 CN 202410061424; 31.01.2024 CN 202410135728; 10.04.2024 CN 202410427661; 27.05.2024 CN 202410663359
(71) Applicant: Haisco Pharmaceutical Group Co., Ltd., Shannan, Tibet 856000 (CN)
(72) Inventor: ZHANG, Chen, Chengdu, Sichuan 611130 (CN); ZHAO, Mingliang, Chengdu, Sichuan 611130 (CN); SU, Liqiang, Chengdu, Sichuan 611130 (CN); DENG, Hua, Chengdu, Sichuan 611130 (CN); QIAN, Meilin, Chengdu, Sichuan 611130 (CN); TANG, Pingming, Chengdu, Sichuan 611130 (CN); LI, Yao, Chengdu, Sichuan 611130 (CN); YAN, Pangke, Chengdu, Sichuan 611130 (CN)
(74) Representative: AWA Denmark A/S
(86) International application number: PCT/CN2024/109120
(87) International publication number: WO 2025/026382

(57) **Abstract**

The present invention relates to an oxo-tricyclic derivative and the pharmaceutical use thereof, in particular to a compound as shown in general formula (I), or a stereoisomer, a deuterated compound, a solvate, a prodrug, a metabolite, a pharmaceutically acceptable salt or a cocrystal thereof, an intermediate thereof, a preparation method therefor, and the use thereof in the preparation of a drug for treating diseases associated with WRN activity or expression level.

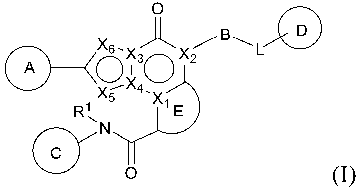

## Description

### Technical Field

The present invention relates to a compound of general formula (I), or a stereoisomer, deuterated compound, solvate, prodrug, metabolite, pharmaceutically acceptable salt, or co-crystal thereof, an intermediate thereof, a preparation method therefor, and the use thereof in the preparation of a medicament for treating a disease associated with the activity or expression level of WRN.

### Background Art

DNA mismatch repair (MMR) is a conserved mechanism that helps maintain genomic stability by eliminating errors during DNA replication, long-strand DNA repair synthesis, and recombination. Germline or somatic mutations and epigenetic alterations in MMR component genes lead to hypermutant phenotypes characterized by cancer susceptibility and high genomic instability, particularly in genomic repeat regions (called microsatellites). Microsatellites or short tandem repeats are short (1-6 base pairs), repetitive DNA sequences distributed in coding and non-coding regions, accounting for approximately 3% of the human genome. MMR deficiency leads to a phenotype called microsatellite instability (MSI), which is characterized by the accumulation of variations in repeat length in microsatellite regions (Mol. Med Today 1997: 3, 61-68). A significant proportion of MSI tumors do not respond to or develop resistance to immunotherapy and chemotherapy, suggesting that patients with MSI tumors are in need of more improved targeted and combination therapies (J. Immunother. Cancer, 2022:10, 4001).

Many articles have reported a therapeutically promising synthetic lethal relationship between RecQ-like family helicase protein (WRN) and MSI tumors (iScience 2019: 13, 488-497; and Nature 2019: 568, 551-556). WRN is a multifunctional enzyme with helicase and exonuclease activities that plays a role in various cellular processes that maintain genomic stability, including DNA replication, transcription, DNA repair, and telomere maintenance. WRN has been determined to be essential in MSI rather than microsatellite stability (MSS) cancer cell lines by CRISPR-Cas9 and RNAi screening. Further analysis has shown that WRN depletion causes cell cycle arrest, DNA damage, mitotic defects, chromosome breakage, and apoptosis in MSI cells. Furthermore, WRN depletion reduces xenograft growth and tumor formation in mice transplanted with MSI cells (npj Precision Oncology 2022: 85).

These research results indicate that the development of WRN inhibitors for the treatment of MSI tumors has application prospects. At present, there are no reports of WRN inhibitors on the market or under clinical development, and there is an urgent need for the development of WRN inhibitors.

### Summary of the Invention

It is an object of the present invention to provide a class of heterocyclic compounds or pharmaceutically acceptable salts thereof for use as WRN inhibitors. The compounds of the present invention can effectively inhibit WRN and can be used for treating diseases such as tumors. The compounds of the present invention exhibit excellent inhibitory activity against WRN helicase, as well as potent inhibitory effects on cell proliferation in HCT-116 and SW48 cells, but show poor inhibitory activity against cell proliferation in HT-29 cells. In addition, they have low toxicity and high safety profile, e.g., weak inhibitory activities against CYP enzymes and hERG channels, and also have favorable pharmacokinetic properties, such as good bioavailability and appropriate half-life.

The present invention provides a compound of general formula (I), or a stereoisomer, tautomer, deuterated compound, solvate, prodrug, metabolite, pharmaceutically acceptable salt, or co-crystal thereof,

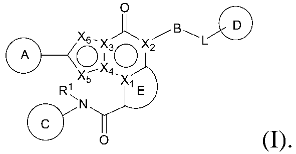

In some embodiments, -̅ -̅ -̅ represents a single bond or a double bond.

In some embodiments, the general formula (I) is general formula (Ia),

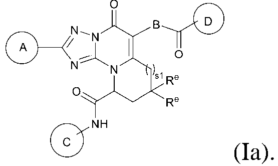

In some embodiments, the general formula (I) is general formula (Ib),

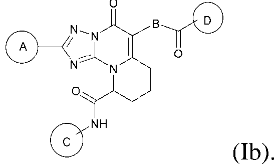

In some embodiments, ring A is selected from C₃₋₁₅ carbocycle or 4- to 15-membered heterocycle, wherein the ring A is optionally substituted with 1 to 6 R^{a}.

In some embodiments, ring A is selected from phenyl, 5- to 6-membered heteroaryl, C₃₋₆ monocyclic carbocycle, or 4- to 8-membered monocyclic heterocycle, wherein the ring A is optionally substituted with 1 to 5 R^{a}.

In some embodiments, ring A is selected from one of the following groups optionally substituted with 1 to 4 R^{a}: cyclopropyl, cyclobutyl, cyclopentyl, cyclohexyl, cyclopropenyl, cyclobutenyl, cyclopentenyl, cyclohexenyl, oxetanyl, azetidinyl, pyrrolidinyl, piperidinyl, piperazinyl, morpholinyl, tetrahydrofuryl, tetrahydropyranyl, dihydropyrrolyl, dihydropyridyl, dihydrofuryl, dihydropyranyl, phenyl, pyrrolyl, thienyl, furyl, pyrazolyl, imidazolyl, thiazolyl, isothiazolyl, oxazolyl, isoxazolyl, pyridyl, pyrimidinyl, pyrazinyl, or pyridazinyl.

In some embodiments, ring A is selected from one of the following groups optionally substituted with 1 to 3 R^{a}:

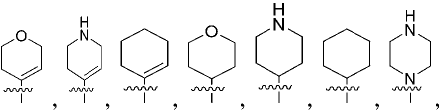

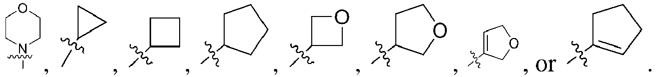

In some embodiments, ring C is selected from C₃₋₁₅ carbocycle or 4- to 15-membered heterocycle, wherein the ring C is optionally substituted with 1 to 6 R^{c}.

In some embodiments, ring C is selected from phenyl, naphthyl, benzo C₄₋₆ carbocycle, benzo 4- to 7-membered heterocyclyl, 5- to 6-membered heteroaryl, or 8- to 10-membered fused heteroaryl, wherein the ring C is optionally substituted with 1 to 5 R^{c}.

In some embodiments, ring C is selected from one of the following groups optionally substituted with 1 to 4 R^{c}: phenyl, pyrrolyl, thienyl, furyl, pyrazolyl, imidazolyl, thiazolyl, isothiazolyl, oxazolyl, isoxazolyl, pyridyl, pyrimidinyl, pyrazinyl, pyridazinyl, naphthyl, benzopyrrolyl, benzothienyl, benzofuryl, benzopyrazolyl, benzimidazolyl, benzothiazolyl, benzisothiazolyl, benzoxazolyl, benzisoxazolyl, benzopyridyl, benzopyrimidinyl, benzopyrazinyl, benzopyridazinyl, pyridopyrrolyl, pyridothienyl, pyridofuryl, pyridopyrazolyl, pyridoimidazolyl, pyridothiazolyl, pyridoisothiazolyl, pyridooxazolyl, pyridoisoxazolyl, pyridopyridyl, pyridopyrimidinyl, pyridopyrazinyl, pyridopyridazinyl,

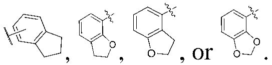

In some embodiments, ring C is selected from one of the following groups optionally substituted with 1 to 4 R^{c}: 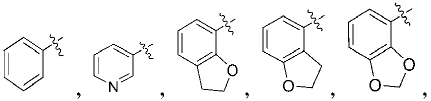 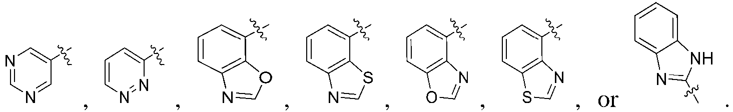 In some embodiments, ring C is selected from one of the following groups optionally substituted with 1 to 3 R^{c}: 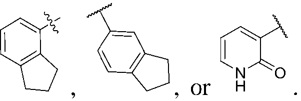 In some embodiments, ring C is selected from one of the following groups optionally substituted with 1 to 2 R^{c}:

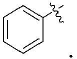

In some embodiments, ring C is selected from 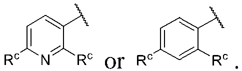 In some embodiments, ring C is

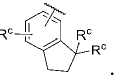

In some embodiments, ring D is selected from C₃₋₁₅ carbocycle or 4- to 15-membered heterocycle, wherein the ring D is optionally substituted with 1 to 6 R^{d}.

In some embodiments, ring D is selected from phenyl, naphthyl, benzo C₄₋₆ carbocycle, benzo 4- to 7-membered heterocyclyl, 5- to 6-membered heteroaryl, or 8- to 10-membered fused heteroaryl, wherein the ring D is optionally substituted with 1 to 5 R^{d}.

In some embodiments, ring D is selected from one of the following groups optionally substituted with 1 to 4 R^{d}: phenyl, pyrrolyl, thienyl, furyl, pyrazolyl, imidazolyl, thiazolyl, isothiazolyl, oxazolyl, isoxazolyl, pyridyl, pyrimidinyl, pyrazinyl, pyridazinyl, triazinyl, benzopyrrolyl, benzothienyl, benzofuryl, benzopyrazolyl, benzimidazolyl, benzothiazolyl, pyridopyrrolyl, pyridothienyl, pyridofuryl, pyridopyrazolyl, pyridoimidazolyl, or pyridothiazolyl.

In some embodiments, ring D is selected from one of the following groups optionally substituted with 1 to 3 R^{d}:

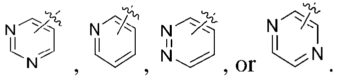

In some embodiments, 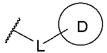 is selected from one of the following groups optionally substituted with 1 to 3 R^{d}:

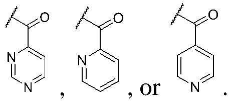

In some embodiments, ring D is selected from 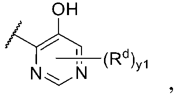 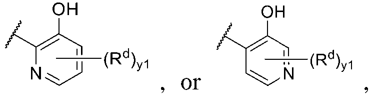 with y1 being selected from 0, 1, or 2, preferably from 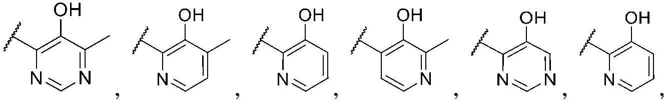 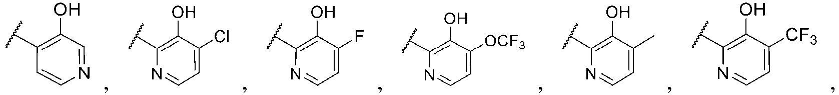 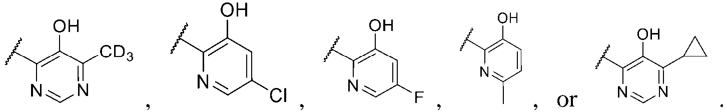 In some embodiments, ring D is

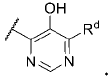

In some embodiments, ring E is selected from C₄₋₁₀ carbocycle or 4- to 10-membered heterocycle, wherein the ring E is optionally substituted with 1 to 6 R^{e}.

In some embodiments, 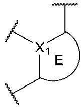 is selected from 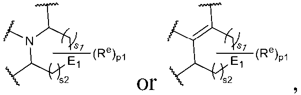 which is directly attached to X₂ at the position directly above, wherein s1 and s2 are each independently selected from 0, 1, 2, or 3; and each p1 is independently selected from 0, 1, 2, 3, or 4.

In some embodiments, each E₁ is independently selected from NR^{e}, C(R^{e})₂, O, S(=O)₂, or S.

In some embodiments, each E₁ is independently selected from NH, CH₂, O, S(=O)₂, or S.

In some embodiments, each E₁ is independently selected from NH, CH₂, C(R^{e})₂, O, S(=O)₂, or S.

In some embodiments, 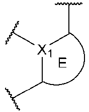 is selected from 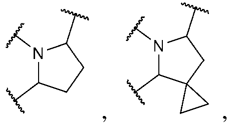 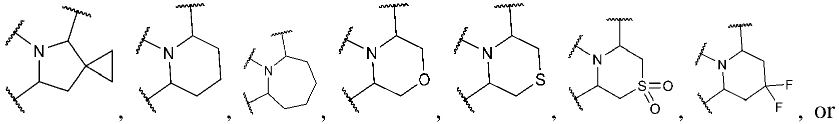 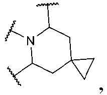 which is directly attached to X₂ at the position directly above.

In some embodiments, 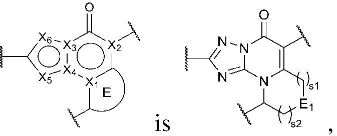 which is directly attached to ring A at the left end, wherein s1 and s2 are each independently selected from 0, 1, or 2.

In some embodiments, 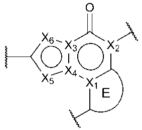 is selected from 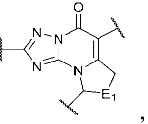 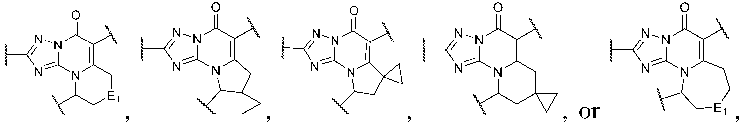 which is directly attached to ring A at the left end.

In some embodiments, 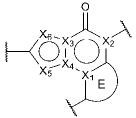 is selected from 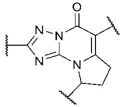 or 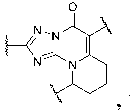 which is directly attached to ring A at the left end.

In some embodiments, X₁, X₂, X₃, X₄, X₅, and X₆ are each independently selected from N, CH, or C, wherein at least two of X₃, X₄, X₅, and X₆ are N, and at least one of X₁ and X₂ is N.

In some embodiments, 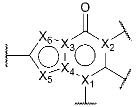 is selected from 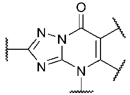 or 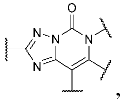 which is directly attached to ring A at the left end.

In some embodiments, B is selected from B1 or B2.

In some embodiments, B1 is selected from

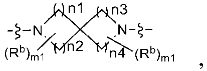

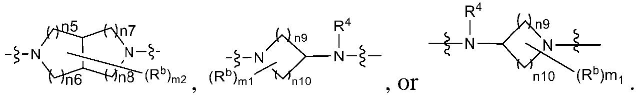

In some embodiments, B1 is selected from 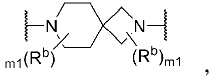 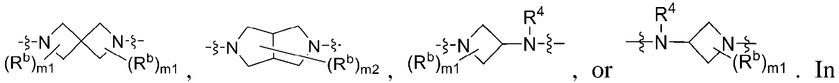 some embodiments, B1 is selected from

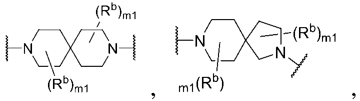

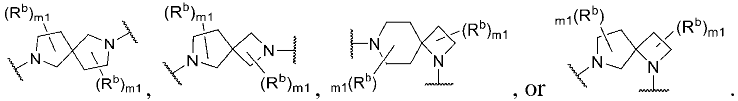

In some embodiments, B2 is selected from 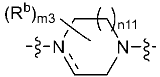 or

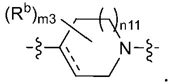

In some embodiments, B2 is

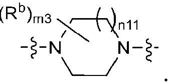

In some embodiments, B2 is selected from 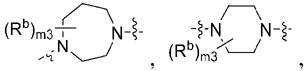 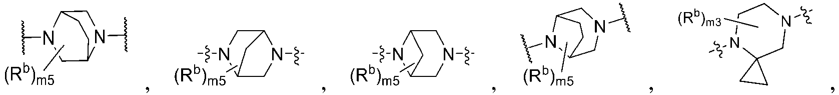 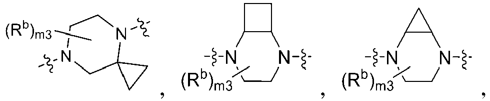 or 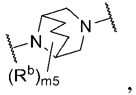 wherein m5 is selected from 0, 1, 2, 3, 4, 5, or 6.

In some embodiments, B is selected from 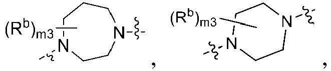 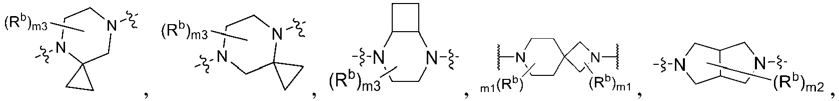 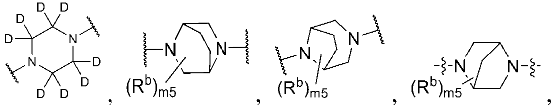 or 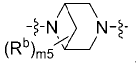 In some embodiments, B is selected from 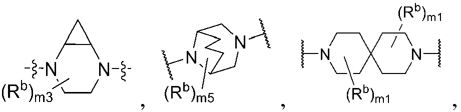 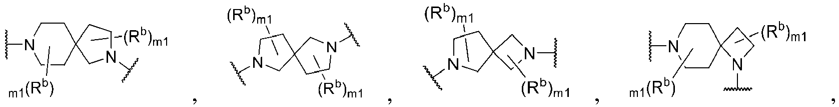 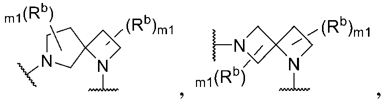 or

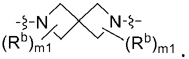

In some embodiments, B is 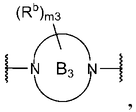 wherein B3 is selected from 5- to 7-membered heterocycloalkyl, 7- to 11-membered spiro heterocycloalkyl, 7- to 11-membered fused heterocycloalkyl, or 7- to 11-membered bridged heterocycloalkyl; and B3 is preferably selected from 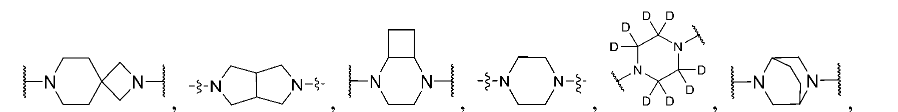 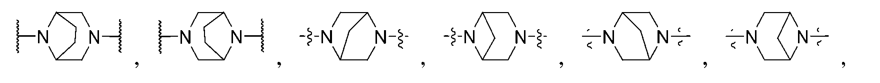 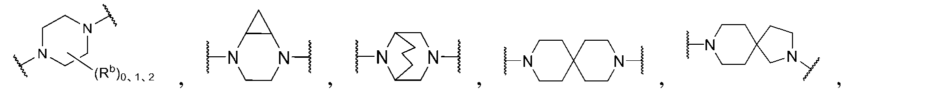 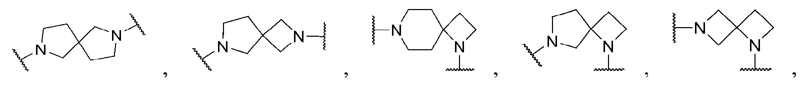 or

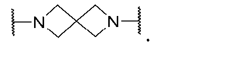

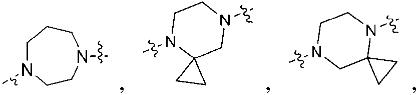

In some embodiments, B is selected from

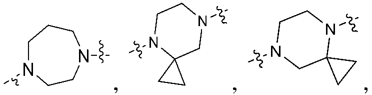

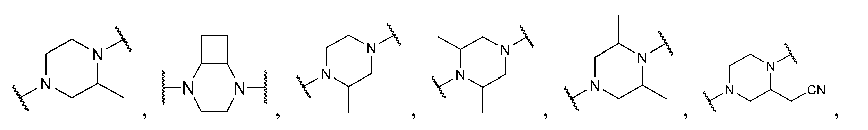

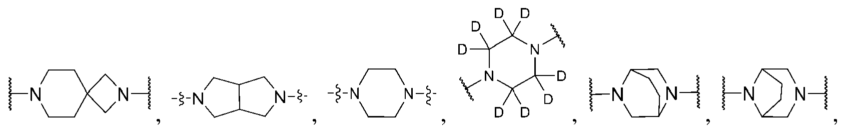

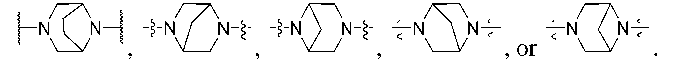

In some embodiments, B is selected from 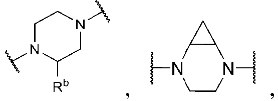 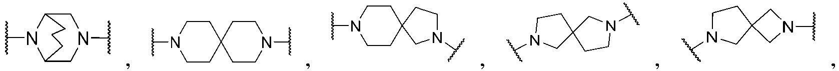 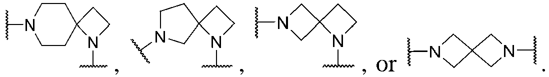 In some embodiments, B is selected from 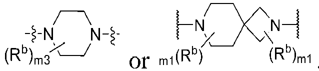 . In some embodiments, B is selected from

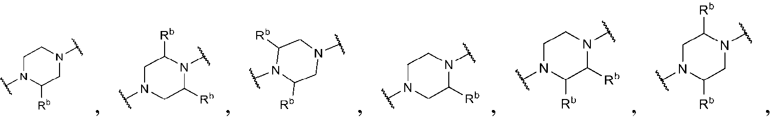

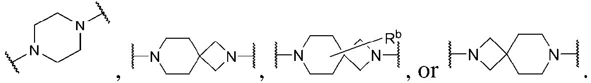

In some embodiments, n1, n2, n3, n4, n9, and n10 are each independently selected from 1, 2, 3, or 4. In some embodiments, n1, n2, n3, and n4 are each independently selected from 0, and the sum of n1, n2, n3, and n4 is greater than 2.

In some embodiments, n5, n6, n7, n8, and n11 are each independently selected from 0, 1, 2, 3, or 4.

In some embodiments, m1, m2, and m3 are each independently selected from 0, 1, 2, 3, 4, 5, 6, 7, or 8. In some embodiments, m3 is selected from 0, 1, 2, 3, or 4. In some embodiments, m3 is selected from 0, 1, or 2. In some embodiments, m1 and m2 are each independently selected from 0, 1, 2, 3, or 4. In some embodiments, each m1 is independently selected from 0 or 1.

In some embodiments, m5 is selected from 0, 1, 2, 3, or 4.

In some embodiments, L is selected from -C(=O)-, -S(=O)-, -S(=O)(=NH)-, or -S(=O)₂-.

In some embodiments, L is -C(=O)-.

In some embodiments, R¹ is selected from H, deuterium, C₁₋₆ alkyl, C₃₋₆ carbocycle, or 3- to 7-membered heterocycle, wherein the alkyl, carbocycle, or heterocycle is optionally substituted with 1 to 4 R^{k}.

In some embodiments, R¹ is selected from H, deuterium, C₁₋₄ alkyl, or C₃₋₆ carbocycle, wherein the alkyl or carbocycle is optionally substituted with 1 to 4 R^{k}.

In some embodiments, R¹ is selected from H, methyl, ethyl, or cyclopropyl.

In some embodiments, R^{a}, R^{b}, R^{c}, R^{d}, and R^{e} are each independently selected from H, deuterium, halogen, OH, =O, CN, NH₂, SF₅, COOH, CONH₂, C₁₋₆ alkyl, OC₁₋₆ alkyl, SC₁₋₆ alkyl, C₂₋₆ alkenyl, C₂₋₆ alkynyl, NHC₁₋₆ alkyl, N(C₁₋₆ alkyl)₂, - C(=O)NH-C₁₋₆ alkyl, -C(=O)N(C₁₋₆ alkyl)₂, -O-C₃₋₆ carbocycle, -O-3- to 7-membered heterocycle, -NH-C₃₋₆ carbocycle, -NH-3- to 7-membered heterocycle, - C₀₋₄ alkylene-C₃₋₆ carbocycle, or -C₀₋₄ alkylene-3- to 7-membered heterocycle, wherein the alkyl, alkylene, alkenyl, alkynyl, carbocycle, or heterocycle is optionally substituted with 1 to 4 R^{k}.

In some embodiments, R^{a}, R^{b}, R^{c}, R^{d}, and R^{e} are each independently selected from H, deuterium, halogen, OH, =O, CN, NH₂, SF₅, COOH, CONH₂, C₁₋₄ alkyl, OC₁₋₄ alkyl, SC₁₋₄ alkyl, C₂₋₄ alkenyl, C₂₋₄ alkynyl, NHC₁₋₄ alkyl, N(C₁₋₄ alkyl)₂, - C(=O)NH-C₁₋₄ alkyl, -C(=O)N(C₁₋₄ alkyl)₂, -O-C₃₋₆ carbocycle, -O-3- to 6-membered heterocycle, -NH-C₃₋₆ carbocycle, -NH-3- to 6-membered heterocycle, - C₀₋₂ alkylene-C₃₋₆ carbocycle, or -C₀₋₂ alkylene-3- to 6-membered heterocycle, wherein the alkyl, alkylene, alkenyl, alkynyl, carbocycle, or heterocycle is optionally substituted with 1 to 4 R^{k}.

In some embodiments, R^{a}, R^{b}, R^{c}, R^{d}, and R^{e} are each independently selected from H, deuterium, F, Cl, Br, I, OH, =O, CN, NH₂, SF₅, COOH, CONH₂, NHCH₃, N(CH₃)₂, or one of the following groups optionally substituted with 1 to 4 R^{k}: methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl.

In some embodiments, R^{a}, R^{b}, R^{c}, and R^{d} are each independently selected from H, deuterium, F, Cl, Br, I, OH, =O, CN, NH₂, SF₅, COOH, CONH₂, NHCH₃, N(CH₃)₂, CD₃, CHF₂, CF₃, CH₂F, OCHF₂, OCF₃, SCF₃, OCH₂F, CH₂CN, CHF₂CH₃, CHF₂CF₃, CH₂OH, methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, morpholinyl,

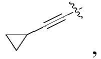

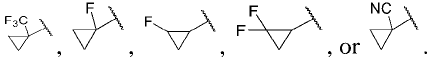

In some embodiments, R^{b} is selected from -C₁₋₄ alkylene-O-C₃₋₆ carbocycle, - C₁₋₄ alkylene-O-C₁₋₆ alkyl, -C₁₋₄ alkylene-O-C₁₋₄ alkylene-O-C₁₋₆ alkyl, or -C₁₋₄ alkylene-O-C₁₋₄ alkylene-O-C₃₋₆ carbocycle, wherein the alkyl, alkylene, or carbocycle is optionally substituted with 1 to 4 R^{k}.

In some embodiments, R^{b} is selected from -C₁₋₂ alkylene-O-C₃₋₆ carbocycle, - C₁₋₂ alkylene-O-C₁₋₄ alkyl, -C₁₋₂ alkylene-O-C₁₋₂ alkylene-O-C₁₋₄ alkyl, or -C₁₋₂ alkylene-O-C₁₋₃ alkylene-O-C₃₋₆ carbocycle, wherein the alkyl, alkylene, or carbocycle is optionally substituted with 1 to 4 R^{k}.

In some embodiments, R^{b} is selected from -methylene-O-cyclopropyl, - ethylene-O-methyl, -methylene-O-methyl, -methylene-O-ethyl, -methylene-O-ethylene-O-methyl, -methylene-O-ethylene-O-ethyl, -methylene-O-ethylene-O-propyl, -methylene-O-ethylene-O-cyclopropyl, -methylene-O-methylene-O-cyclopropyl, or -methylene-O-ethylene-O-cyclobutyl, wherein the methylene, ethylene, methyl, ethyl, propyl, cyclopropyl, or cyclobutyl is optionally substituted with 1 to 4 R^{k}.

In some embodiments, R^{b} is selected from -CH₂-O-cyclopropyl, -CH₂-OCH₃, -CH₂-O-CH₂CH₂OCH₃, -CH₂OCH₂CH₂OCH₂CH₂CH₂OCH₃, or -CH₂-O-CH₂CH₂O-cyclopropyl.

In some embodiments, each R^{e} is independently selected from H, deuterium, F, Cl, Br, I, OH, CN, NH₂, SF₅, COOH, CONH₂, NHCH₃, N(CH₃)₂, CD₃, CHF₂, CF₃, CH₂F, OCHF₂, OCF₃, SCF₃, OCH₂F, CH₂CN, methyl, ethyl, propyl, isopropyl, methoxy, ethoxy, methylthio, vinyl, ethynyl, propynyl, propargyl, cyclopropyl, cyclobutyl, azetidinyl, or oxetanyl.

In some embodiments, two R^{b} in B2, together with the atoms or ring skeletons to which they are attached, form C₃₋₆ carbocycle or 4- to 8-membered heterocycle, wherein the carbocycle or heterocycle is optionally substituted with 1 to 4 R^{k}.

In some embodiments, two R^{b} in B2, together with the atoms or ring skeletons to which they are attached, form C₃₋₆ cycloalkyl or 4- to 7-membered heterocycloalkyl, wherein the cycloalkyl or heterocycloalkyl is optionally substituted with 1 to 4 R^{k}.

In some embodiments, two R^{b} in B2, together with the carbon atoms to which they are directly attached, form cyclopropyl, cyclobutyl, cyclopentyl, cyclohexyl, azetidinyl, azacyclopentyl, or azacyclohexyl, wherein the cyclopropyl, cyclobutyl, cyclopentyl, cyclohexyl, azetidinyl, azacyclopentyl, or azacyclohexyl is optionally substituted with 1 to 4 R^{k}.

In some embodiments, two R^{b}, together with the atoms or ring skeletons to which they are attached, form C₃₋₆ carbocycle or 4- to 8-membered heterocycle, wherein the carbocycle or heterocycle is optionally substituted with 1 to 4 R^{k}.

In some embodiments, two R^{b}, together with the carbon atoms to which they are directly attached, form cyclopropyl, cyclobutyl, cyclopentyl, cyclohexyl, azetidinyl, azacyclopentyl, or azacyclohexyl, wherein the cyclopropyl, cyclobutyl, cyclopentyl, cyclohexyl, azetidinyl, azacyclopentyl, or azacyclohexyl is optionally substituted with 1 to 4 R^{k}.

In some embodiments, two R^{e}, together with the atoms to which they are attached, form C₃₋₆ carbocycle or 4- to 8-membered heterocycle, wherein the carbocycle or heterocycle is optionally substituted with 1 to 4 R^{k}.

In some embodiments, two R^{e}, together with the carbon atoms to which they are directly attached, form cyclopropyl, cyclobutyl, cyclopentyl, cyclohexyl, azetidinyl, azacyclopentyl, azacyclohexyl, or oxetanyl, wherein the cyclopropyl, cyclobutyl, cyclopentyl, cyclohexyl, azetidinyl, azacyclopentyl, azacyclohexyl, or oxetanyl is optionally substituted with 1 to 4 R^{k}.

In some embodiments, each R^{k} is independently selected from deuterium, halogen, =O , CN, OH, SH, NO₂, COOH, CONH₂, NH₂, SF₅, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocycle, -O-3- to 7-membered heterocycle, -NH-C₃₋₆ carbocycle, -NH-3- to 7-membered heterocycle, -C₁₋₄ alkylene-C₃₋₆ carbocycle, -C₁₋₄ alkylene-3- to 7-membered heterocycle, C₃₋₆ carbocycle, or 3- to 7-membered heterocycle, wherein the alkyl, alkylene, alkenyl, alkynyl, carbocycle, or heterocycle is optionally substituted with 1 to 4 substituents selected from deuterium, halogen, CN, OH, NH₂, C₁₋₆ alkyl, or C₁₋₆ alkoxy.

In some embodiments, each R^{k} is independently selected from deuterium, halogen, =O , CN, OH, SH, NO₂, COOH, CONH₂, NH₂, SF₅, C₁₋₄ alkyl, OC₁₋₄ alkyl, SC₁₋₄ alkyl, C₂₋₄ alkenyl, C₂₋₄ alkynyl, NHC₁₋₄ alkyl, N(C₁₋₄ alkyl)₂, -O-C₃₋₆ carbocycle, -O-3- to 6-membered heterocycle, -NH-C₃₋₆ carbocycle, -NH-3- to 6-membered heterocycle, -C₁₋₂ alkylene-C₃₋₆ carbocycle, -C₁₋₂ alkylene-3- to 6-membered heterocycle, C₃₋₆ carbocycle, or 3- to 6-membered heterocycle, wherein the alkyl, alkylene, alkenyl, alkynyl, carbocycle, or heterocycle is optionally substituted with 1 to 4 substituents selected from deuterium, halogen, CN, OH, NH₂, C₁₋₆ alkyl, or C₁₋₆ alkoxy.

In some embodiments, each R^{k} is independently selected from deuterium, F, Cl, Br, I, =O, CN, OH, SH, NO₂, COOH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl, wherein the methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl is optionally substituted with 1 to 4 substituents selected from deuterium, F, Cl, Br, I, CN, OH, NH₂, C₁₋₄ alkyl, or C₁₋₄ alkoxy.

In some embodiments, each R^{k} is independently selected from deuterium, F, Cl, Br, I, =O, CN, OH, SH, NO₂, COOH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl, wherein the methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl is optionally substituted with 1 to 4 substituents selected from deuterium, F, Cl, Br, I, CN, OH, NH₂, methyl, ethyl, methoxy, or ethoxy.

In some embodiments, each R^{k} is independently selected from deuterium, F, Cl, Br, I, =O, CN, OH, SH, NH₂, NO₂, SF₅, COOH, CONH₂, NH(CH₃), NH(CH₂CH₃), N(CH₃)₂, N(CH₂CH₃)₂, CD₃, OCD₃, CF₃, CH₂F, CHF₂, CH₂OH, OCF₃, OCHF₂, OCH₂F, methyl, ethyl, vinyl, ethynyl, methoxy, ethoxy, methylthio, -O-cyclopropyl, -NH-cyclopropyl, -CH₂-cyclopropyl, -CH₂-cyclobutyl, -CH₂-cyclopentyl, -CH₂-cyclohexyl, cyclopropyl, cyclobutyl, cyclopentyl, or cyclohexyl.

As a first embodiment of the present invention, in the compound represented by general formula (I) described above, or the stereoisomer, tautomer, deuterated compound, solvate, prodrug, metabolite, pharmaceutically acceptable salt, or co-crystal thereof,
ring A is selected from C₃₋₁₅ carbocycle or 4- to 15-membered heterocycle, wherein the ring A is optionally substituted with 1 to 6 R^{a};
ring C is selected from C₃₋₁₅ carbocycle or 4- to 15-membered heterocycle, wherein the ring C is optionally substituted with 1 to 6 R^{c};
ring D is selected from C₃₋₁₅ carbocycle or 4- to 15-membered heterocycle, wherein the ring D is optionally substituted with 1 to 6 R^{d};
ring E is selected from C₄₋₁₀ carbocycle or 4- to 10-membered heterocycle, wherein the ring E is optionally substituted with 1 to 6 R^{e};
B is selected from B1 or B2, wherein
B1 is selected from 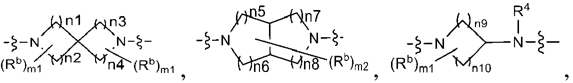 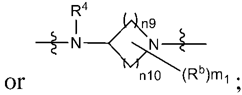 and
B2 is selected from 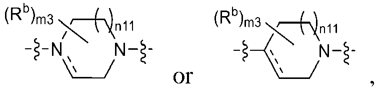 wherein -̅ -̅ -̅ represents a single bond or a double bond;
n1, n2, n3, n4, n9, and n10 are each independently selected from 1, 2, 3, or 4; or n1, n2, n3, and n4 are each independently selected from 0, and the sum of n1, n2, n3, and n4 is greater than 2;
n5, n6, n7, n8, and n11 are each independently selected from 0, 1, 2, 3, or 4; and
m1, m2, and m3 are each independently selected from 0, 1, 2, 3, 4, 5, 6, 7, or 8;
L is selected from -C(=O)-, -S(=O)-, -S(=O)(=NH)-, or -S(=O)₂-;
X₁, X₂, X₃, X₄, X₅, and X₆ are each independently selected from N, CH, or C, wherein at least two of X₃, X₄, X₅, and X₆ are N, and at least one of X₁ and X₂ is N;
R¹ is selected from H, deuterium, C₁₋₆ alkyl, C₃₋₆ carbocycle, or 3- to 7-membered heterocycle, wherein the alkyl, carbocycle, or heterocycle is optionally substituted with 1 to 4 R^{k};
R^{a}, R^{b}, R^{c}, R^{d}, and R^{e} are each independently selected from H, deuterium, halogen, OH, =O, CN, NH₂, SF₅, COOH, CONH₂, C₁₋₆ alkyl, OC₁₋₆ alkyl, SC₁₋₆ alkyl, C₂₋₆ alkenyl, C₂₋₆ alkynyl, NHC₁₋₆ alkyl, N(C₁₋₆ alkyl)₂, -C(=O)NH-C₁₋₆ alkyl, -C(=O)N(C₁₋₆ alkyl)₂, -O-C₃₋₆ carbocycle, -O-3- to 7-membered heterocycle, -NH-C₃₋₆ carbocycle, -NH-3- to 7-membered heterocycle, -C₀₋₄ alkylene-C₃₋₆ carbocycle, or -C₀₋₄ alkylene-3- to 7-membered heterocycle, wherein the alkyl, alkylene, alkenyl, alkynyl, carbocycle, or heterocycle is optionally substituted with 1 to 4 R^{k};
or R^{b} is selected from -C₁₋₄ alkylene-O-C₃₋₆ carbocycle, -C₁₋₄ alkylene-O-C₁₋₆ alkyl, -C₁₋₄ alkylene-O-C₁₋₄ alkylene-O-C₁₋₆ alkyl, or -C₁₋₄ alkylene-O-C₁₋₄ alkylene-O-C₃₋₆ carbocycle, wherein the alkyl, alkylene, or carbocycle is optionally substituted with 1 to 4 R^{k}; or
alternatively, two R^{b} in B2, together with the atoms or ring skeletons to which they are attached, form C₃₋₆ carbocycle or 4- to 8-membered heterocycle, wherein the carbocycle or heterocycle is optionally substituted with 1 to 4 R^{k}; and
alternatively, two R^{e}, together with the atoms to which they are attached, form C₃₋₆ carbocycle or 4- to 8-membered heterocycle, wherein the carbocycle or heterocycle is optionally substituted with 1 to 4 R^{k}; and
each R^{k} is independently selected from deuterium, halogen, =O, CN, OH, SH, NO₂, COOH, CONH₂, NH₂, SF₅, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocycle, -O-3- to 7-membered heterocycle, -NH-C₃₋₆ carbocycle, -NH-3- to 7-membered heterocycle, - C₁₋₄ alkylene-C₃₋₆ carbocycle, -C₁₋₄ alkylene-3- to 7-membered heterocycle, C₃₋₆ carbocycle, or 3- to 7-membered heterocycle, wherein the alkyl, alkylene, alkenyl, alkynyl, carbocycle, or heterocycle is optionally substituted with 1 to 4 substituents selected from deuterium, halogen, CN, OH, NH₂, C₁₋₆ alkyl, or C₁₋₆ alkoxy.

As a second embodiment of the present invention, in the compound represented by general formula (I) described below, or the stereoisomer, tautomer, deuterated compound, solvate, prodrug, metabolite, pharmaceutically acceptable salt, or co-crystal thereof,
ring A is selected from phenyl, 5- to 6-membered heteroaryl, C₃₋₆ monocyclic carbocycle, or 4- to 8-membered monocyclic heterocycle, wherein the ring A is optionally substituted with 1 to 5 R^{a};
ring C is selected from phenyl, naphthyl, benzo C₄₋₆ carbocycle, benzo 4- to 7-membered heterocyclyl, 5- to 6-membered heteroaryl, or 8- to 10-membered fused heteroaryl, wherein the ring C is optionally substituted with 1 to 5 R^{c};
ring D is selected from phenyl, naphthyl, benzo C₄₋₆ carbocycle, benzo 4- to 7-membered heterocyclyl, 5- to 6-membered heteroaryl, or 8- to 10-membered fused heteroaryl, wherein the ring D is optionally substituted with 1 to 5 R^{d}; 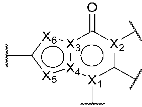
is selected from 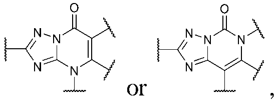 which is directly attached to ring A at the left end; 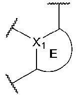
is selected from 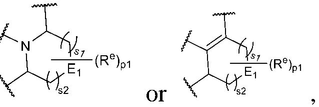 which is directly attached to X₂ at the position directly above, wherein s1 and s2 are each independently selected from 0, 1, 2, or 3;
each pl is independently selected from 0, 1, 2, 3, or 4; and
each E₁ is independently selected from NR^{e}, C(R^{e})₂, O, S(=O)₂, or S;
R¹ is selected from H, deuterium, C₁₋₄ alkyl, or C₃₋₆ carbocycle, wherein the alkyl or carbocycle is optionally substituted with 1 to 4 R^{k};
alternatively, two R^{e}, together with the atoms to which they are attached, form C₃₋₆ carbocycle or 4- to 8-membered heterocycle, wherein the carbocycle or heterocycle is optionally substituted with 1 to 4 R^{k}; and
the remaining definitions are the same as those in the first embodiment of the present invention.

As a third embodiment of the present invention, in the compound represented by general formula (I) described above, or the stereoisomer, tautomer, deuterated compound, solvate, prodrug, metabolite, pharmaceutically acceptable salt, or co-crystal thereof,
B2 is 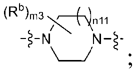
R^{a}, R^{b}, R^{c}, R^{d}, and R^{e} are each independently selected from H, deuterium, halogen, OH, =O, CN, NH₂, SF₅, COOH, CONH₂, C₁₋₄ alkyl, OC₁₋₄ alkyl, SC₁₋₄ alkyl, C₂₋₄ alkenyl, C₂₋₄ alkynyl, NHC₁₋₄ alkyl, N(C₁₋₄ alkyl)₂, -C(=O)NH-C₁₋₄ alkyl, -C(=O)N(C₁₋₄ alkyl)₂, -O-C₃₋₆ carbocycle, -O-3- to 6-membered heterocycle, -NH-C₃₋₆ carbocycle, -NH-3- to 6-membered heterocycle, -C₀₋₂ alkylene-C₃₋₆ carbocycle, or -C₀₋₂ alkylene-3- to 6-membered heterocycle, wherein the alkyl, alkylene, alkenyl, alkynyl, carbocycle, or heterocycle is optionally substituted with 1 to 4 R^{k};
or R^{b} is selected from -C₁₋₂ alkylene-O-C₃₋₆ carbocycle, -C₁₋₂ alkylene-O-C₁₋₄ alkyl, -C₁₋₂ alkylene-O-C₁₋₂ alkylene-O-C₁₋₄ alkyl, or -C₁₋₂ alkylene-O-C₁₋₃ alkylene-O-C₃₋₆ carbocycle, wherein the alkyl, alkylene, or carbocycle is optionally substituted with 1 to 4 R^{k}; or
alternatively, two R^{b} in B2, together with the atoms or ring skeletons to which they are attached, form C₃₋₆ cycloalkyl or 4- to 7-membered heterocycloalkyl, wherein the cycloalkyl or heterocycloalkyl is optionally substituted with 1 to 4 R^{k};
each R^{k} is independently selected from deuterium, halogen, =O, CN, OH, SH, NO₂, COOH, CONH₂, NH₂, SF₅, C₁₋₄ alkyl, OC₁₋₄ alkyl, SC₁₋₄ alkyl, C₂₋₄ alkenyl, C₂₋₄ alkynyl, NHC₁₋₄ alkyl, N(C₁₋₄ alkyl)₂, -O-C₃₋₆ carbocycle, -O-3- to 6-membered heterocycle, -NH-C₃₋₆ carbocycle, -NH-3- to 6-membered heterocycle, - C₁₋₂ alkylene-C₃₋₆ carbocycle, -C₁₋₂ alkylene-3- to 6-membered heterocycle, C₃₋₆ carbocycle, or 3- to 6-membered heterocycle, wherein the alkyl, alkylene, alkenyl, alkynyl, carbocycle, or heterocycle is optionally substituted with 1 to 4 substituents selected from deuterium, halogen, CN, OH, NH₂, C₁₋₆ alkyl, or C₁₋₆ alkoxy; and
the remaining definitions are the same as those in the first or second embodiment of the present invention.

As a fourth embodiment of the present invention, in the compound represented by general formula (I) described above, or the stereoisomer, tautomer, deuterated compound, solvate, prodrug, metabolite, pharmaceutically acceptable salt, or co-crystal thereof, 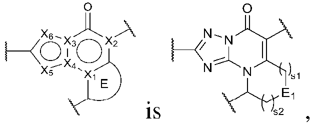
which is directly attached to ring A at the left end, wherein s1 and s2 are each independently selected from 0, 1, or 2;
L is -C(=O)-;
R¹ is selected from H, methyl, ethyl, or cyclopropyl;
R^{a}, R^{b}, R^{c}, R^{d}, and R^{e} are each independently selected from H, deuterium, F, Cl, Br, I, OH, =O, CN, NH₂, SF₅, COOH, CONH₂, NHCH₃, N(CH₃)₂, or one of the following groups optionally substituted with 1 to 4 R^{k}: methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl;
or R^{b} is selected from -methylene-O-cyclopropyl, -ethylene-O-methyl, - methylene-O-methyl, -methylene-O-ethyl, -methylene-O-ethylene-O-methyl, - methylene-O-ethylene-O-ethyl, -methylene-O-ethylene-O-propyl, -methylene-O-ethylene-O-cyclopropyl, -methylene-O-methylene-O-cyclopropyl, or -methylene-O-ethylene-O-cyclobutyl, wherein the methylene, ethylene, methyl, ethyl, propyl, cyclopropyl, or cyclobutyl is optionally substituted with 1 to 4 R^{k}; or
alternatively, two R^{b} in B2, together with the carbon atoms to which they are directly attached, form cyclopropyl, cyclobutyl, cyclopentyl, cyclohexyl, azetidinyl, azacyclopentyl, or azacyclohexyl, wherein the cyclopropyl, cyclobutyl, cyclopentyl, cyclohexyl, azetidinyl, azacyclopentyl, or azacyclohexyl is optionally substituted with 1 to 4 R^{k}; and
alternatively, two R^{e}, together with the carbon atoms to which they are attached, form cyclopropyl, cyclobutyl, cyclopentyl, cyclohexyl, azetidinyl, azacyclopentyl, azacyclohexyl, or oxetanyl, wherein the cyclopropyl, cyclobutyl, cyclopentyl, cyclohexyl, azetidinyl, azacyclopentyl, azacyclohexyl, or oxetanyl is optionally substituted with 1 to 4 R^{k};
each R^{k} is independently selected from deuterium, F, Cl, Br, I, =O, CN, OH, SH, NO₂, COOH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl, wherein the methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl is optionally substituted with 1 to 4 substituents selected from deuterium, F, Cl, Br, I, CN, OH, NH₂, C₁₋₄ alkyl, or C₁₋₄ alkoxy; and
the remaining definitions are the same as those in the first, second, or third embodiment of the present invention.

As a fifth embodiment of the present invention, in the compound represented by general formula (I) described above, or the stereoisomer, tautomer, deuterated compound, solvate, prodrug, metabolite, pharmaceutically acceptable salt, or co-crystal thereof,
ring A is selected from one of the following groups optionally substituted with 1 to 4 R^{a}: cyclopropyl, cyclobutyl, cyclopentyl, cyclohexyl, cyclopropenyl, cyclobutenyl, cyclopentenyl, cyclohexenyl, oxetanyl, azetidinyl, pyrrolidinyl, piperidinyl, piperazinyl, morpholinyl, tetrahydrofuryl, tetrahydropyranyl, dihydropyrrolyl, dihydropyridyl, dihydrofuryl, dihydropyranyl, phenyl, pyrrolyl, thienyl, furyl, pyrazolyl, imidazolyl, thiazolyl, isothiazolyl, oxazolyl, isoxazolyl, pyridyl, pyrimidinyl, pyrazinyl, or pyridazinyl;
ring C is selected from one of the following groups optionally substituted with 1 to 4 R^{c}: phenyl, pyrrolyl, thienyl, furyl, pyrazolyl, imidazolyl, thiazolyl, isothiazolyl, oxazolyl, isoxazolyl, pyridyl, pyrimidinyl, pyrazinyl, pyridazinyl, naphthyl, benzopyrrolyl, benzothienyl, benzofuryl, benzopyrazolyl, benzimidazolyl, benzothiazolyl, benzisothiazolyl, benzoxazolyl, benzisoxazolyl, benzopyridyl, benzopyrimidinyl, benzopyrazinyl, benzopyridazinyl, pyridopyrrolyl, pyridothienyl, pyridofuryl, pyridopyrazolyl, pyridoimidazolyl, pyridothiazolyl, pyridoisothiazolyl, pyridooxazolyl, pyridoisoxazolyl, pyridopyridyl, pyridopyrimidinyl, pyridopyrazinyl, pyridopyridazinyl, 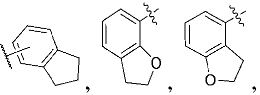 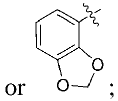
ring D is selected from one of the following groups optionally substituted with 1 to 4 R^{d}: phenyl, pyrrolyl, thienyl, furyl, pyrazolyl, imidazolyl, thiazolyl, isothiazolyl, oxazolyl, isoxazolyl, pyridyl, pyrimidinyl, pyrazinyl, pyridazinyl, triazinyl, benzopyrrolyl, benzothienyl, benzofuryl, benzopyrazolyl, benzimidazolyl, benzothiazolyl, pyridopyrrolyl, pyridothienyl, pyridofuryl, pyridopyrazolyl, pyridoimidazolyl, or pyridothiazolyl;
B2 is selected from 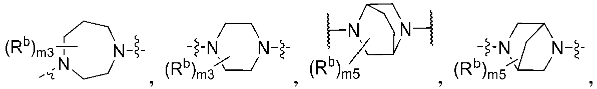 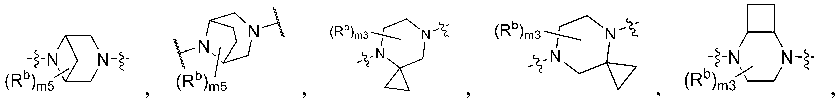 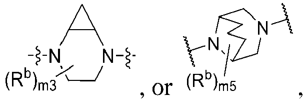 wherein
m5 is selected from 0, 1, 2, 3, 4, 5, or 6;
B1 is selected from 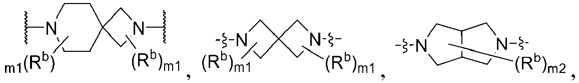 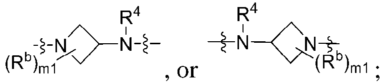
or B1 is selected from 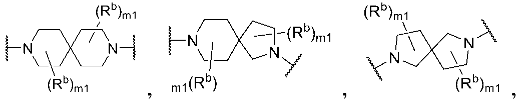 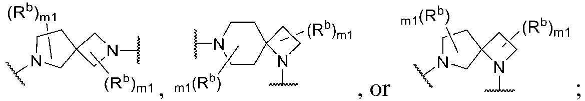
each R^{k} is independently selected from deuterium, F, Cl, Br, I, =O, CN, OH, SH, NO₂, COOH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl, wherein the methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl is optionally substituted with 1 to 4 substituents selected from deuterium, F, Cl, Br, I, CN, OH, NH₂, methyl, ethyl, methoxy, or ethoxy; and
the remaining definitions are the same as those in the first, second, third, or fourth embodiment of the present invention.

As a sixth embodiment of the present invention, in the compound represented by general formula (I) described above, or the stereoisomer, tautomer, deuterated compound, solvate, prodrug, metabolite, pharmaceutically acceptable salt, or co-crystal thereof, 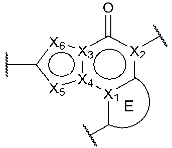
is selected from 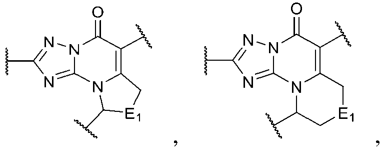 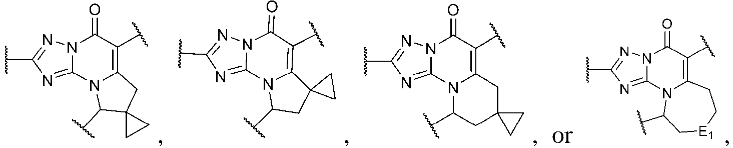 which is directly attached to ring A at the left end, wherein
each E₁ is independently selected from NH, CH₂, O, S(=O)₂, or S;
or each E₁ is independently C(R^{e})₂;
ring A is selected from one of the following groups optionally substituted with 1 to 3 R^{a}: 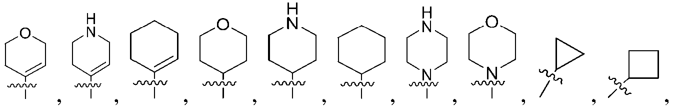 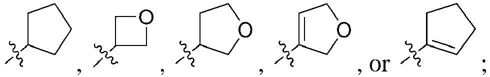
ring C is selected from one of the following groups optionally substituted with 1 to 4 R^{c}: 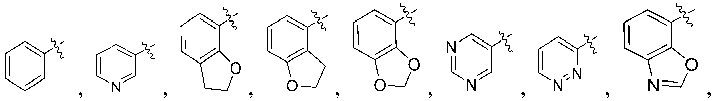 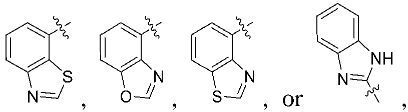 or ring C is selected from one of the following groups optionally substituted with 1 to 3 R^{c}: 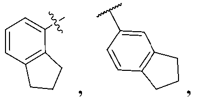 or 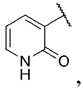 preferably from 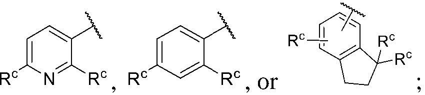
ring D is selected from one of the following groups optionally substituted with 1 to 3 R^{d}: 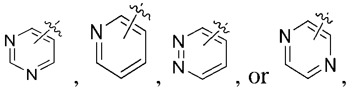 preferably from 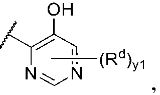 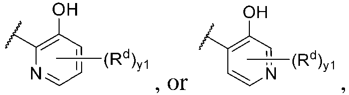 wherein y1 is selected from 0, 1, or 2;
B is selected from 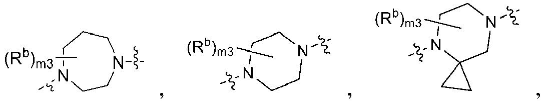 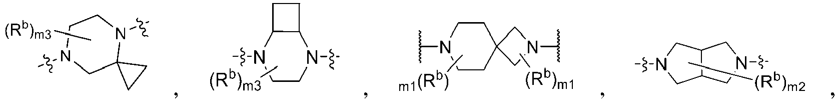 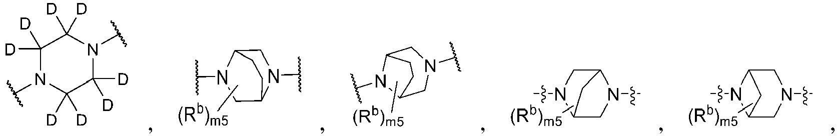 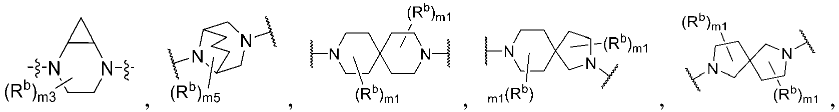 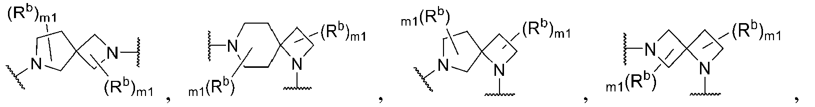 or 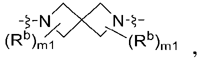 preferably from 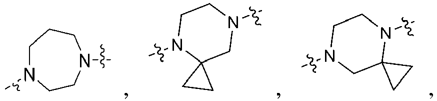 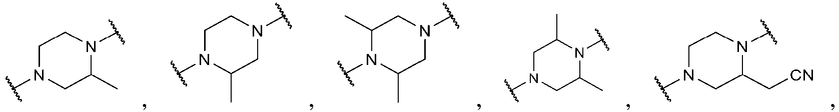 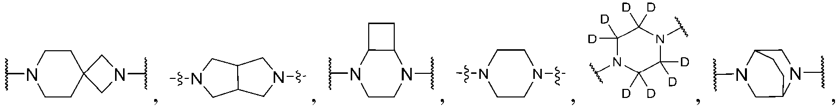 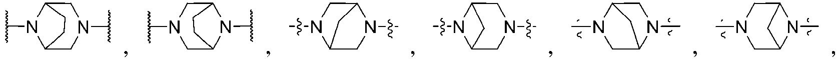 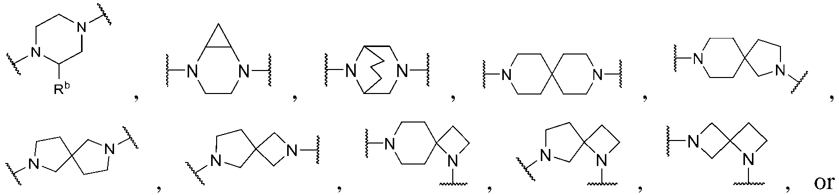 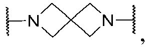 wherein
m3 is selected from 0, 1, 2, 3, or 4;
m5 is selected from 0, 1, 2, 3, or 4; and m1 and m2 are each independently selected from 0, 1, 2, 3, or 4;
R^{a}, R^{b}, R^{c}, and R^{d} are each independently selected from H, deuterium, F, Cl, Br, I, OH, =O, CN, NH₂, SF₅, COOH, CONH₂, NHCH₃, N(CH₃)₂, CD₃, CHF₂, CF₃, CH₂F, OCHF₂, OCF₃, SCF₃, OCH₂F, CH₂CN, CHF₂CH₃, CHF₂CF₃, CH₂OH, methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, morpholinyl, 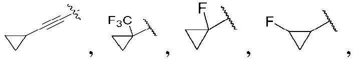 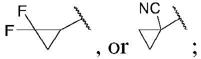
or R^{b} is selected from -CH₂-O-cyclopropyl, -CH₂-OCH₃, -CH₂-O-CH₂CH₂OCH₃, -CH₂OCH₂CH₂OCH₂CH₂CH₂OCH₃, or -CH₂-O-CH₂CH₂O-cyclopropyl;
each R^{e} is independently selected from H, deuterium, F, Cl, Br, I, OH, CN, NH₂, SF₅, COOH, CONH₂, NHCH₃, N(CH₃)₂, CD₃, CHF₂, CF₃, CH₂F, OCHF₂, OCF₃, SCF₃, OCH₂F, CH₂CN, methyl, ethyl, propyl, isopropyl, methoxy, ethoxy, methylthio, vinyl, ethynyl, propynyl, propargyl, cyclopropyl, cyclobutyl, azetidinyl, or oxetanyl; and
the remaining definitions are the same as those in the first, second, third, fourth, or fifth embodiment of the present invention.

As a seventh embodiment of the present invention, in the compound represented by general formula (Ib) described above, or the stereoisomer, tautomer, deuterated compound, solvate, prodrug, metabolite, pharmaceutically acceptable salt, or co-crystal thereof,
ring A is selected from one of the following groups optionally substituted with 1 to 4 R^{a}: cyclopropyl, cyclobutyl, cyclopentyl, cyclohexyl, cyclopropenyl, cyclobutenyl, cyclopentenyl, cyclohexenyl, oxetanyl, azetidinyl, pyrrolidinyl, piperidinyl, piperazinyl, morpholinyl, tetrahydrofuryl, tetrahydropyranyl, dihydropyrrolyl, dihydropyridyl, dihydrofuryl, dihydropyranyl, phenyl, pyrrolyl, thienyl, furyl, pyrazolyl, imidazolyl, thiazolyl, isothiazolyl, oxazolyl, isoxazolyl, pyridyl, pyrimidinyl, pyrazinyl, or pyridazinyl;
ring C is selected from one of the following groups optionally substituted with 1 to 4 R^{c}: phenyl, pyrrolyl, thienyl, furyl, pyrazolyl, imidazolyl, thiazolyl, isothiazolyl, oxazolyl, isoxazolyl, pyridyl, pyrimidinyl, pyrazinyl, pyridazinyl, naphthyl, benzopyrrolyl, benzothienyl, benzofuryl, benzopyrazolyl, benzimidazolyl, benzothiazolyl, benzisothiazolyl, benzoxazolyl, benzisoxazolyl, benzopyridyl, benzopyrimidinyl, benzopyrazinyl, benzopyridazinyl, pyridopyrrolyl, pyridothienyl, pyridofuryl, pyridopyrazolyl, pyridoimidazolyl, pyridothiazolyl, pyridoisothiazolyl, pyridooxazolyl, pyridoisoxazolyl, pyridopyridyl, pyridopyrimidinyl, pyridopyrazinyl, pyridopyridazinyl, 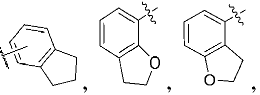 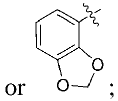
ring D is selected from one of the following groups optionally substituted with 1 to 4 R^{d}: phenyl, pyrrolyl, thienyl, furyl, pyrazolyl, imidazolyl, thiazolyl, isothiazolyl, oxazolyl, isoxazolyl, pyridyl, pyrimidinyl, pyrazinyl, pyridazinyl, triazinyl, benzopyrrolyl, benzothienyl, benzofuryl, benzopyrazolyl, benzimidazolyl, benzothiazolyl, pyridopyrrolyl, pyridothienyl, pyridofuryl, pyridopyrazolyl, pyridoimidazolyl, or pyridothiazolyl;
B is selected from 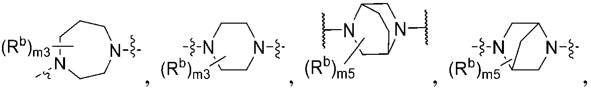 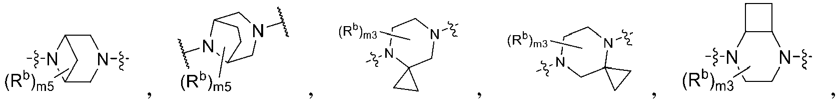 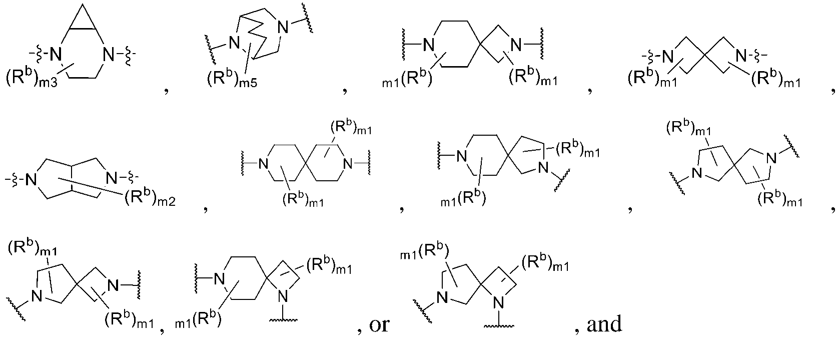
preferably, B is selected from 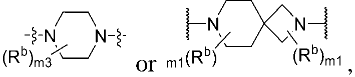 wherein
m5 is selected from 0, 1, 2, 3, 4, 5, or 6;
each m3 is independently selected from 0, 1, 2, 3, 4, 5, 6, 7, or 8, preferably from 0, 1, or 2; and
m1 and m2 are each independently selected from 0, 1, 2, 3, or 4, preferably from 0 or 1;
R^{a}, R^{b}, R^{c}, and R^{d} are each independently selected from H, deuterium, F, Cl, Br, I, OH, =O, CN, NH₂, SF₅, COOH, CONH₂, NHCH₃, N(CH₃)₂, or one of the following groups optionally substituted with 1 to 4 R^{k}: methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl; and
preferably, each R^{b} is independently selected from H, deuterium, F, Cl, Br, I, OH, CN, NH₂, SF₅, NHCH₃, N(CH₃)₂, CD₃, CHF₂, CF₃, CH₂F, CHF₂CF₃, CHF₂CH₃, OCHF₂, OCF₃, SCF₃, OCH₂F, CH₂CN, CH₂OH, -CH₂-OCH₃, methyl, ethyl, propyl, isopropyl, methoxy, or ethoxy; and
each R^{k} is independently selected from deuterium, F, Cl, Br, I, =O, CN, OH, SH, NO₂, COOH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl, wherein the methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl is optionally substituted with 1 to 4 substituents selected from deuterium, F, Cl, Br, I, CN, OH, NH₂, methyl, ethyl, methoxy, or ethoxy.

As an eighth embodiment of the present invention, in the compound represented by general formula (Ib) described above, or the stereoisomer, tautomer, deuterated compound, solvate, prodrug, metabolite, pharmaceutically acceptable salt, or co-crystal thereof,
ring A is selected from one of the following groups optionally substituted with 1 to 3 R^{a}: 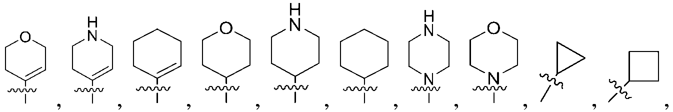 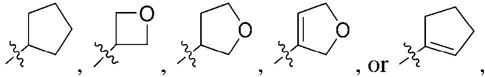 and preferably is 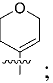
ring C is selected from one of the following groups optionally substituted with 1 to 4 R^{c}: 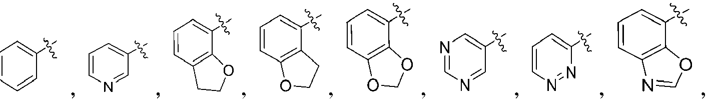 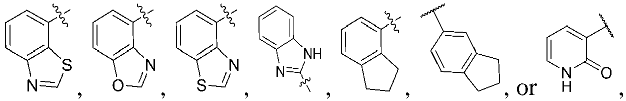 and preferably is the following group optionally substituted with 1 to 2 R^{c}: 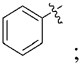
ring D is selected from one of the following groups optionally substituted N with 1 to 3 R^{d}: 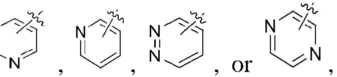 preferably from 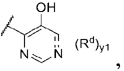 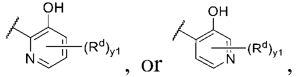 wherein y1 is selected from 0, 1, or 2; and further preferably is 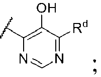
B is selected from 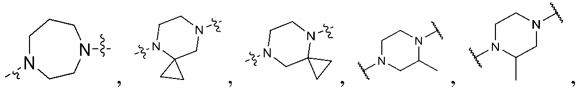 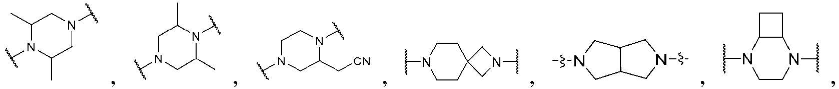 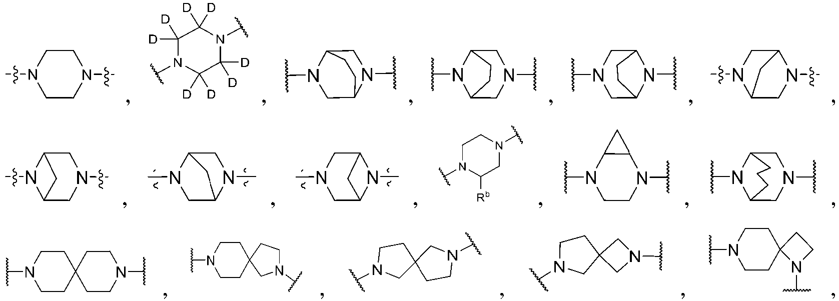 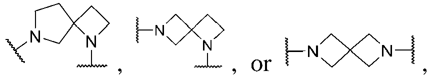 preferably from 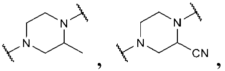 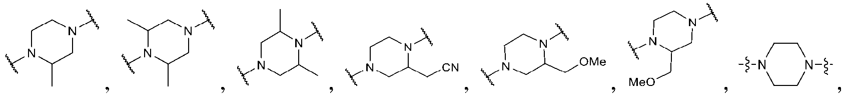 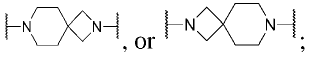 and
R^{a}, R^{c}, and R^{d} are each independently selected from H, deuterium, F, Cl, Br, I, OH, =O, CN, NH₂, SF₅, COOH, CONH₂, NHCH₃, N(CH₃)₂, CD₃, CHF₂, CF₃, CH₂F, CHF₂CF₃, CHF₂CH₃, OCHF₂, OCF₃, SCF₃, OCH₂F, CH₂CN, CH₂OH, - CH₂-OCH₃, methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, morpholinyl, 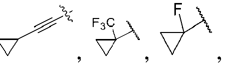 and 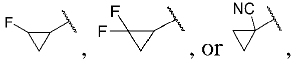
preferably, R^{a}, R^{c}, and R^{d} are each independently selected from H, deuterium, F, Cl, Br, I, OH, CN, NH₂, SF₅, NHCH₃, N(CH₃)₂, CD₃, CHF₂, CF₃, CH₂F, CHF₂CF₃, CHF₂CH₃, OCHF₂, OCF₃, SCF₃, OCH₂F, CH₂CN, CH₂OH, -CH₂-OCH₃, methyl, ethyl, propyl, isopropyl, methoxy, or ethoxy.

The present invention relates to a compound as shown below, or a stereoisomer, tautomer, deuterated compound, solvate, prodrug, metabolite, pharmaceutically acceptable salt, or co-crystal thereof, wherein the compound is selected from one of the structures shown in Table E-1.

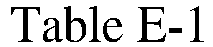

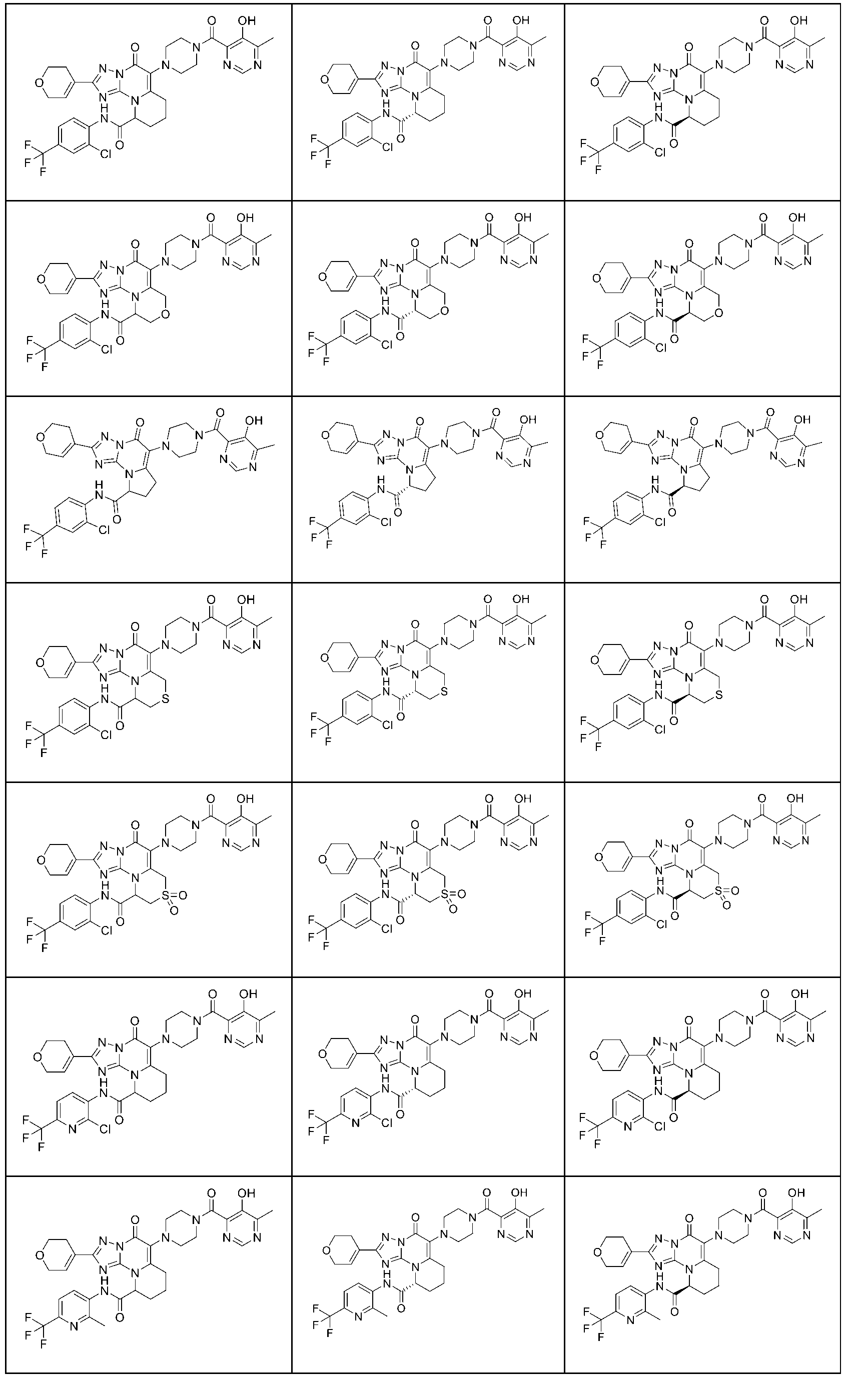

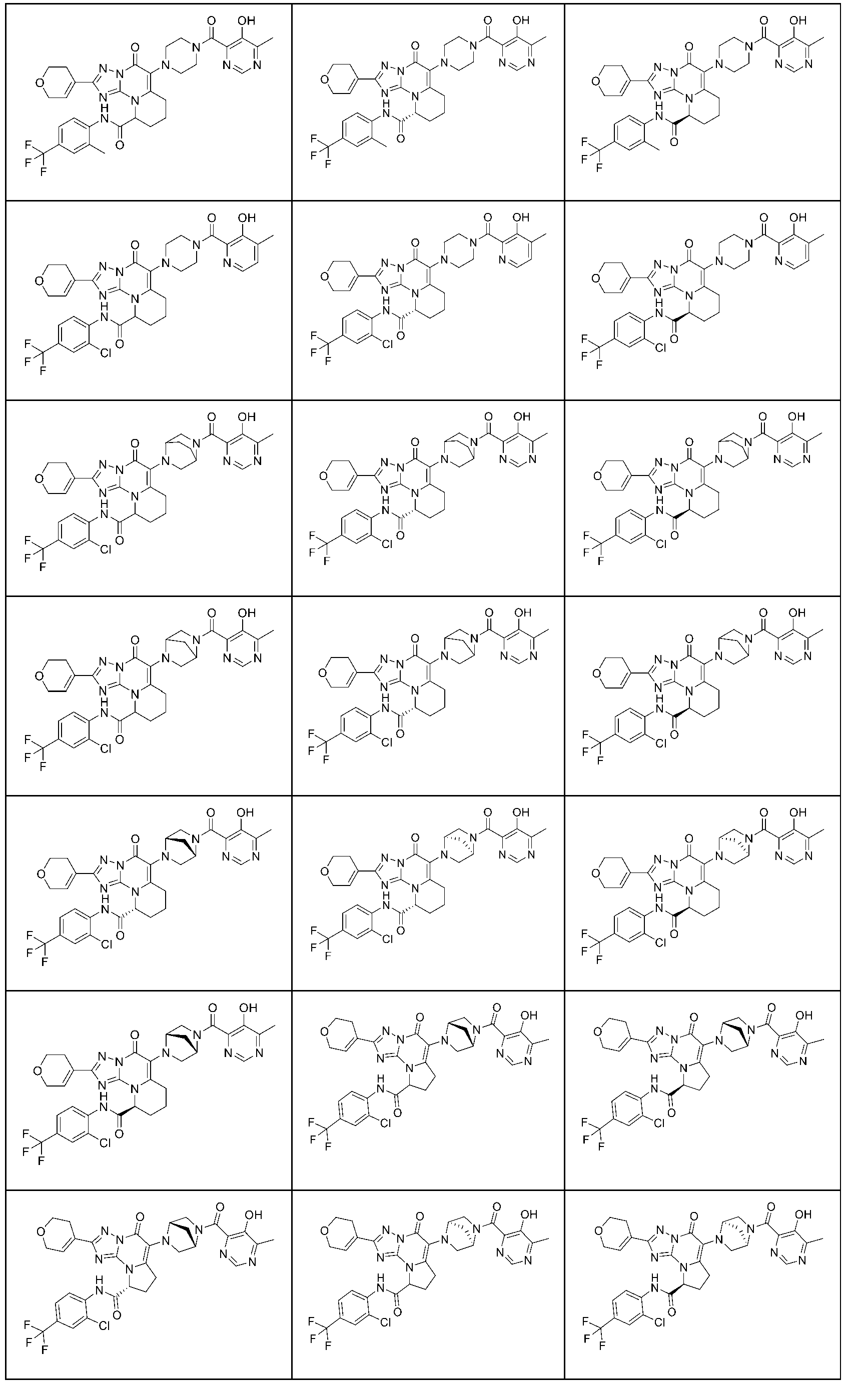

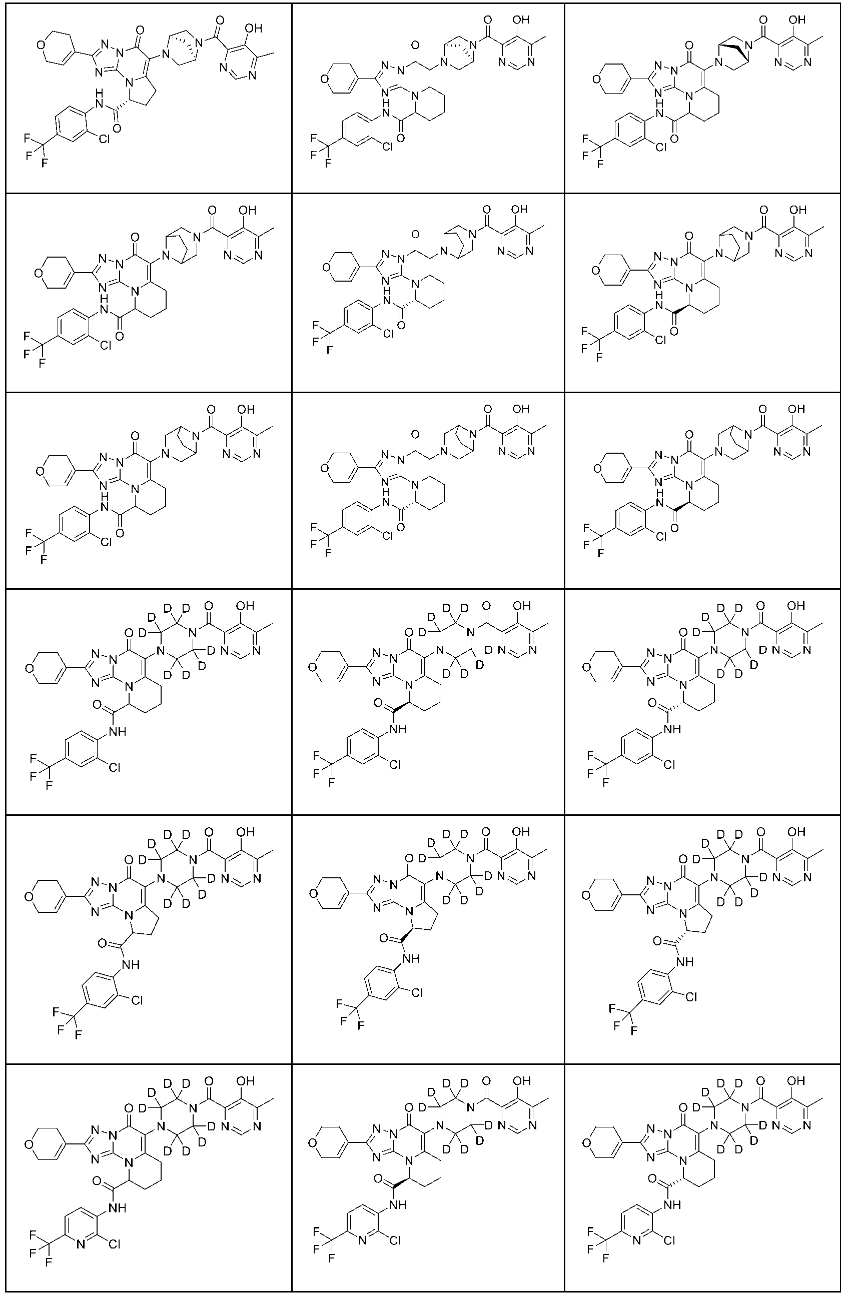

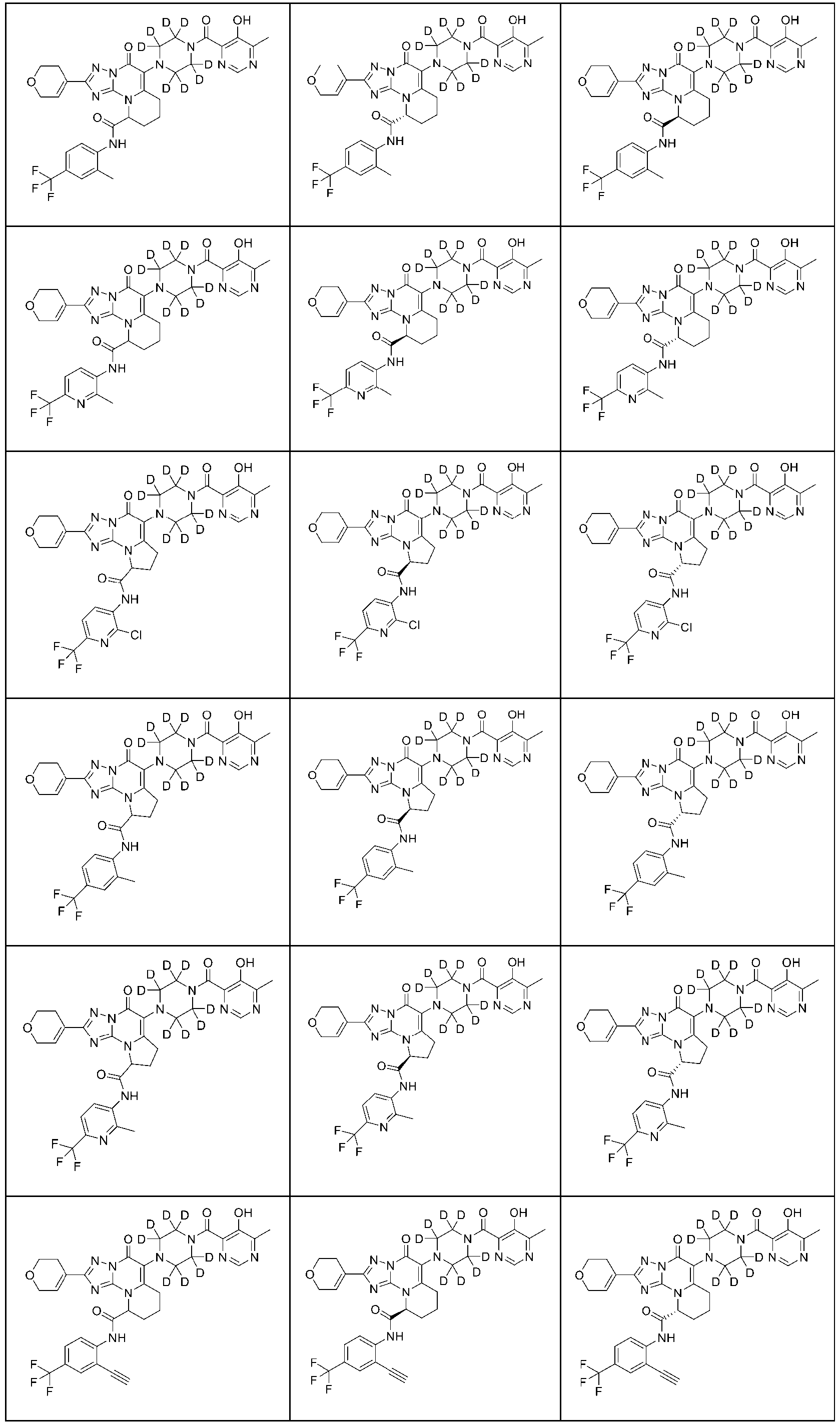

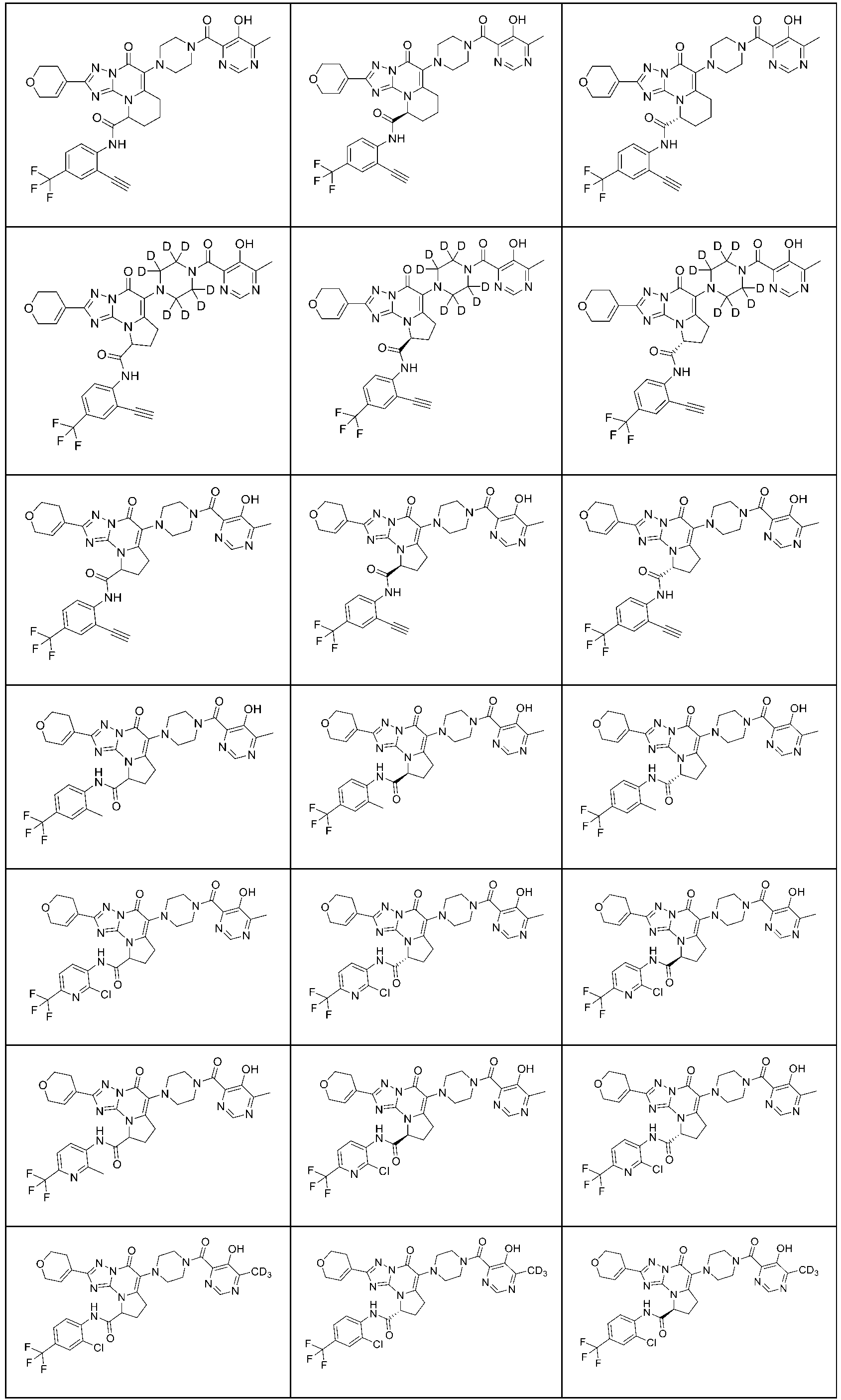

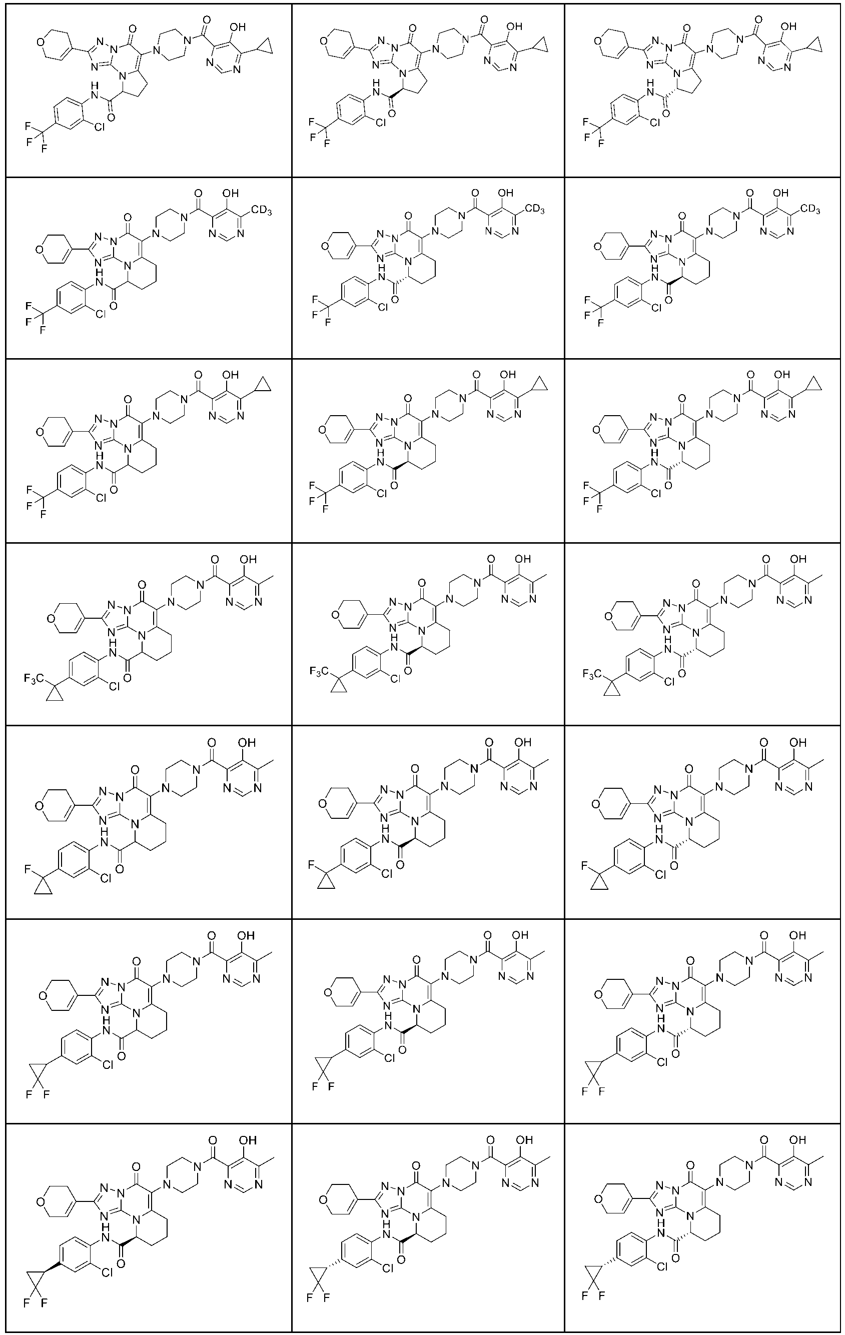

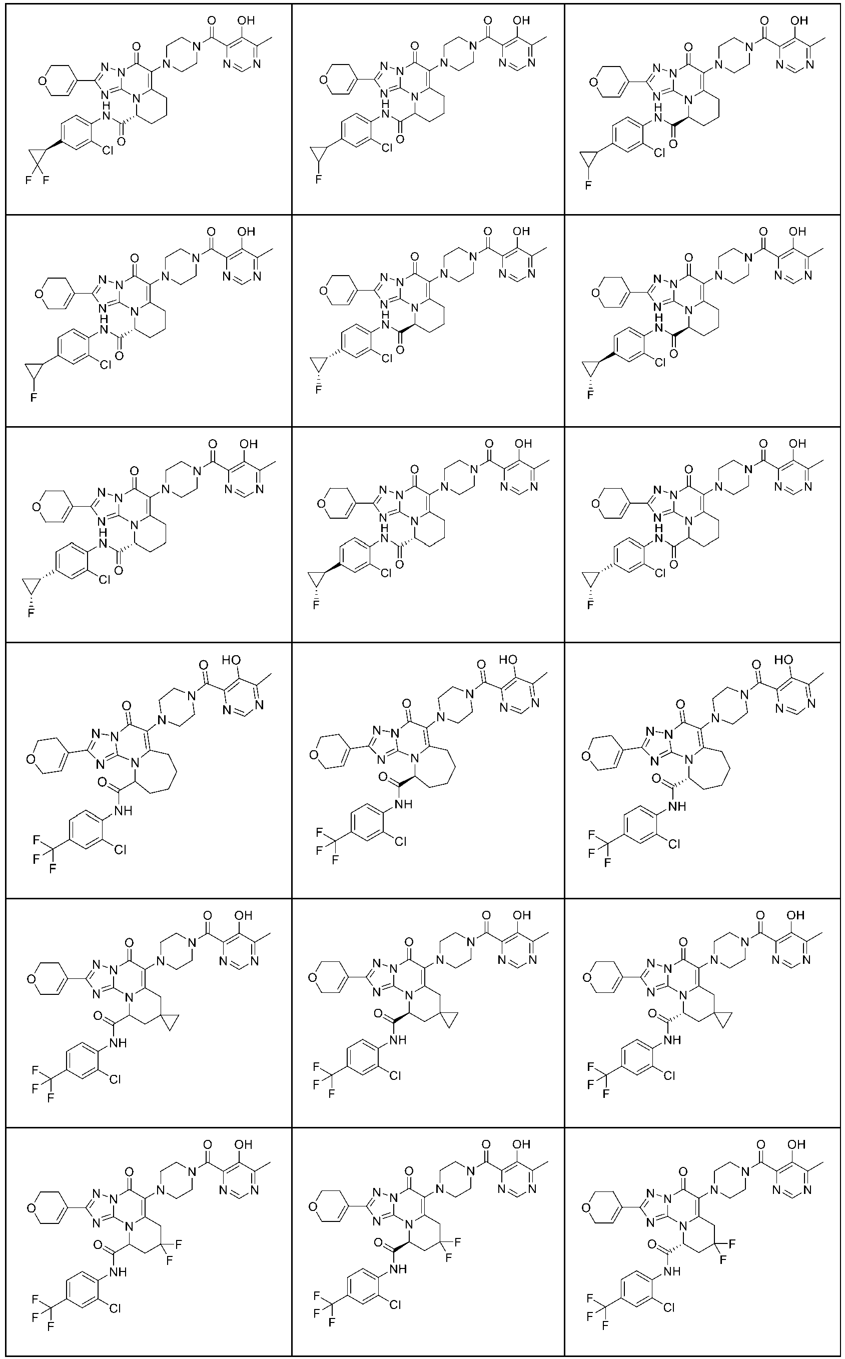

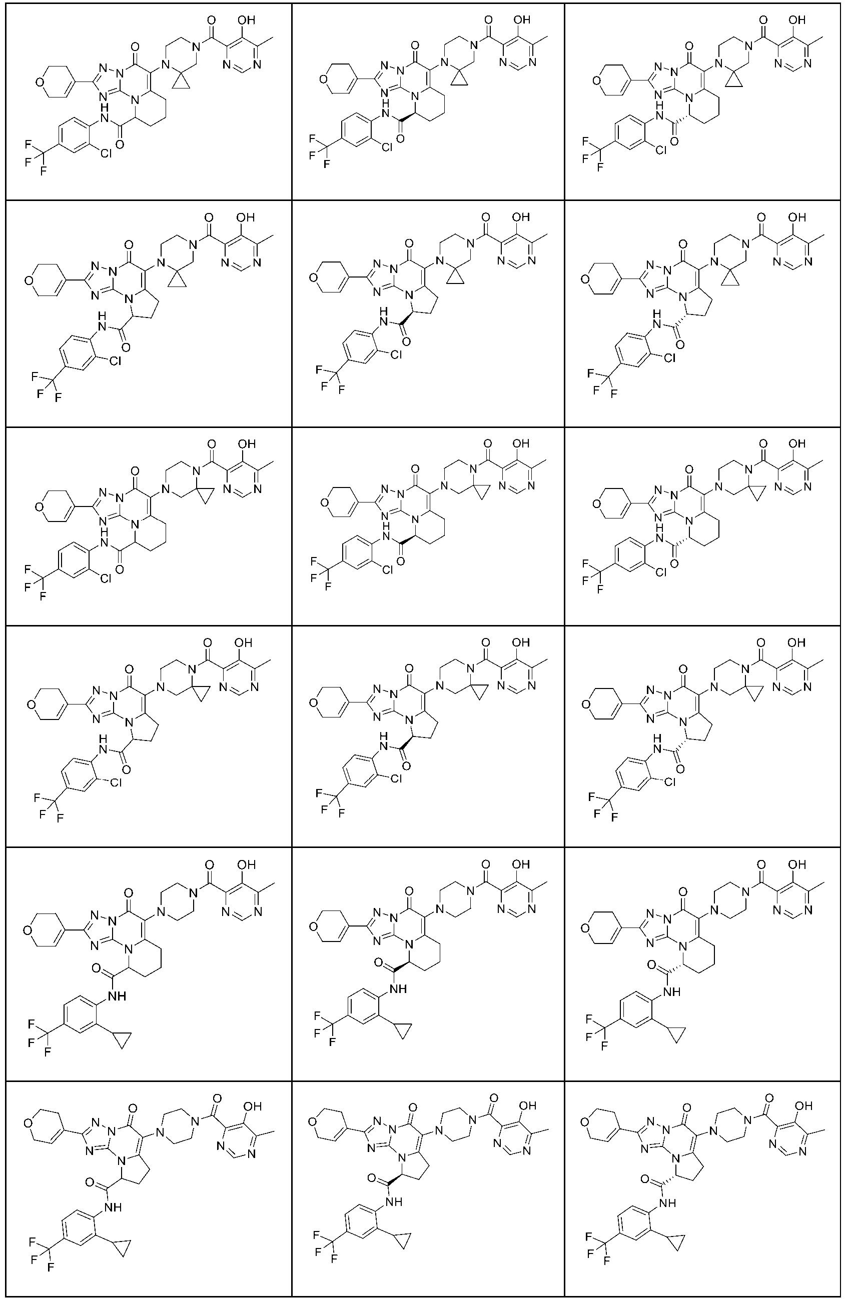

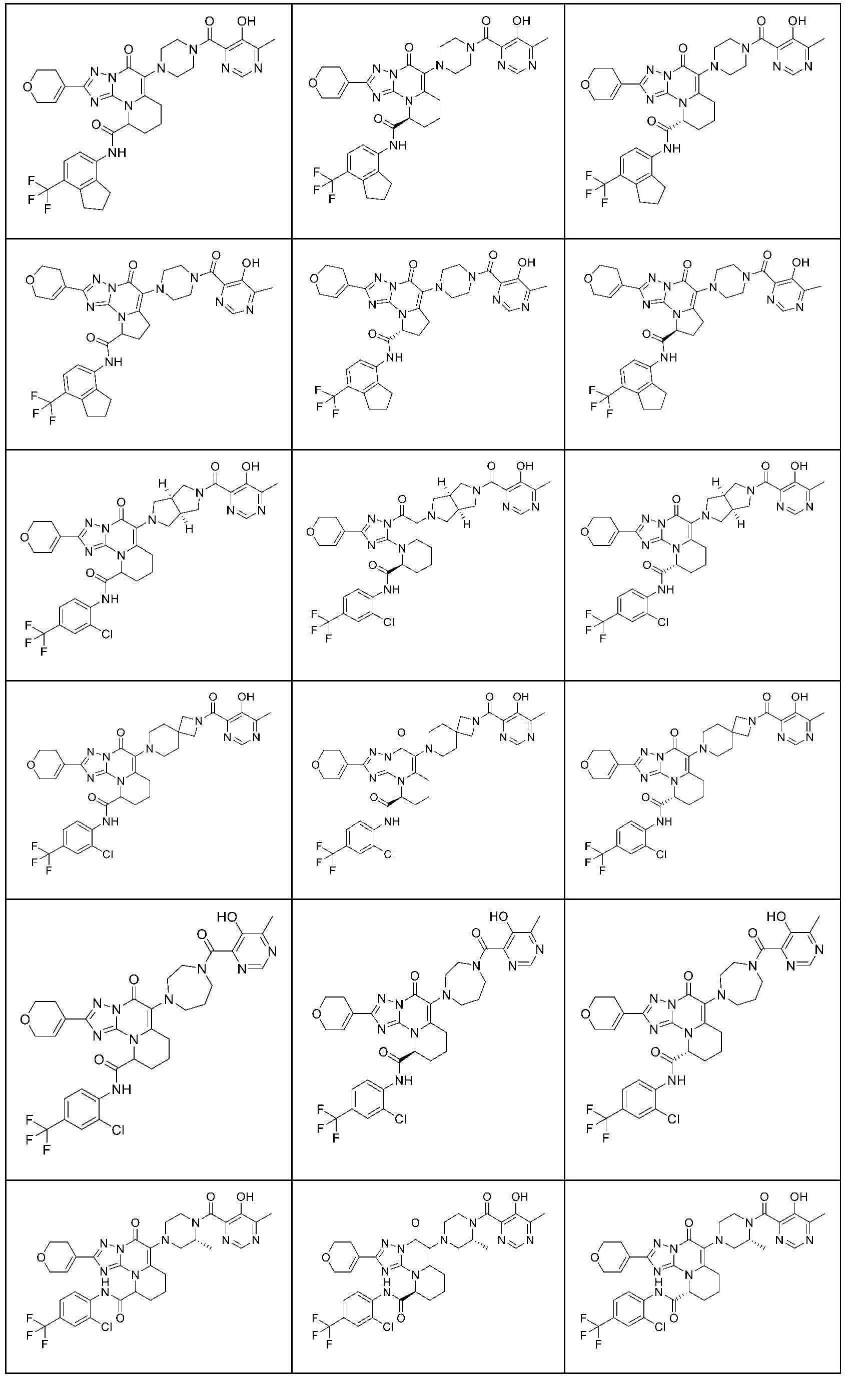

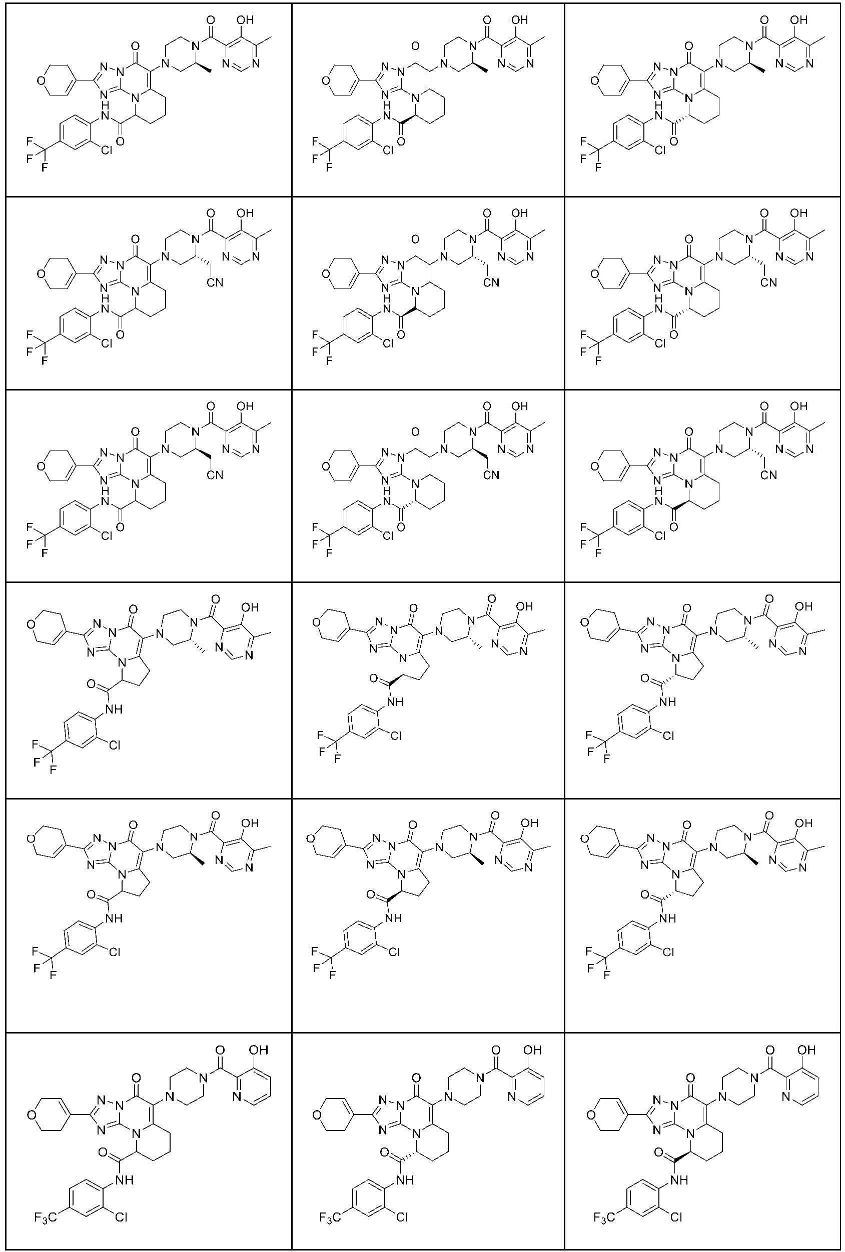

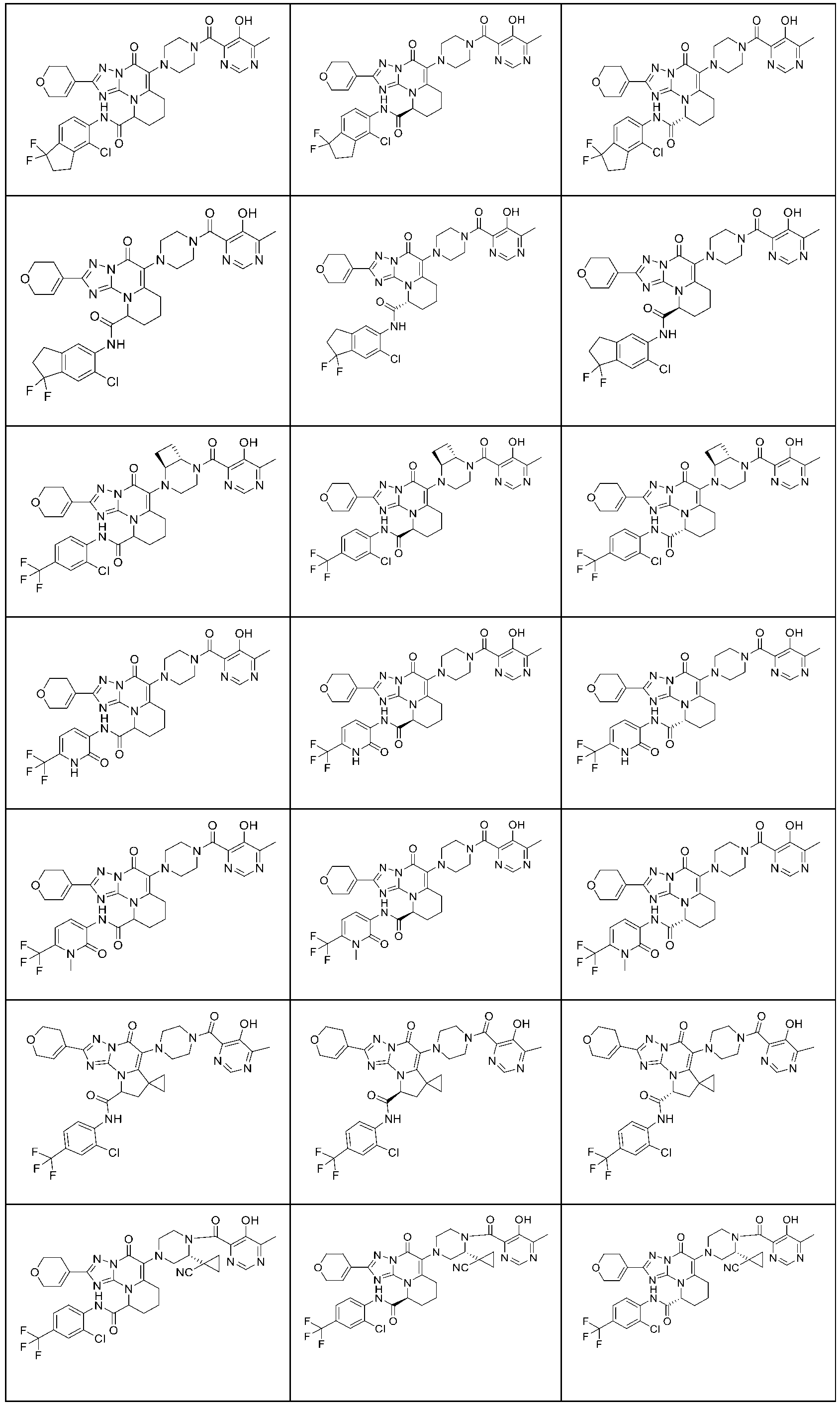

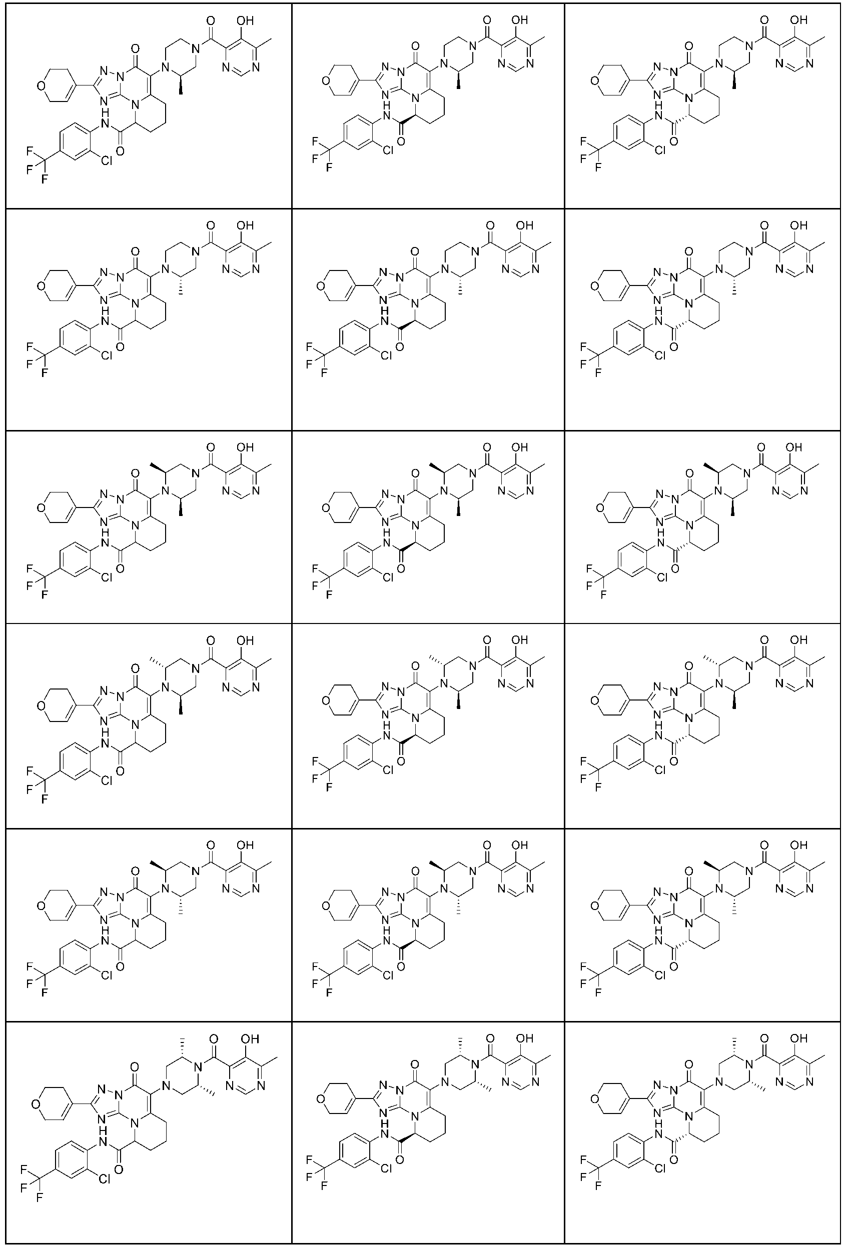

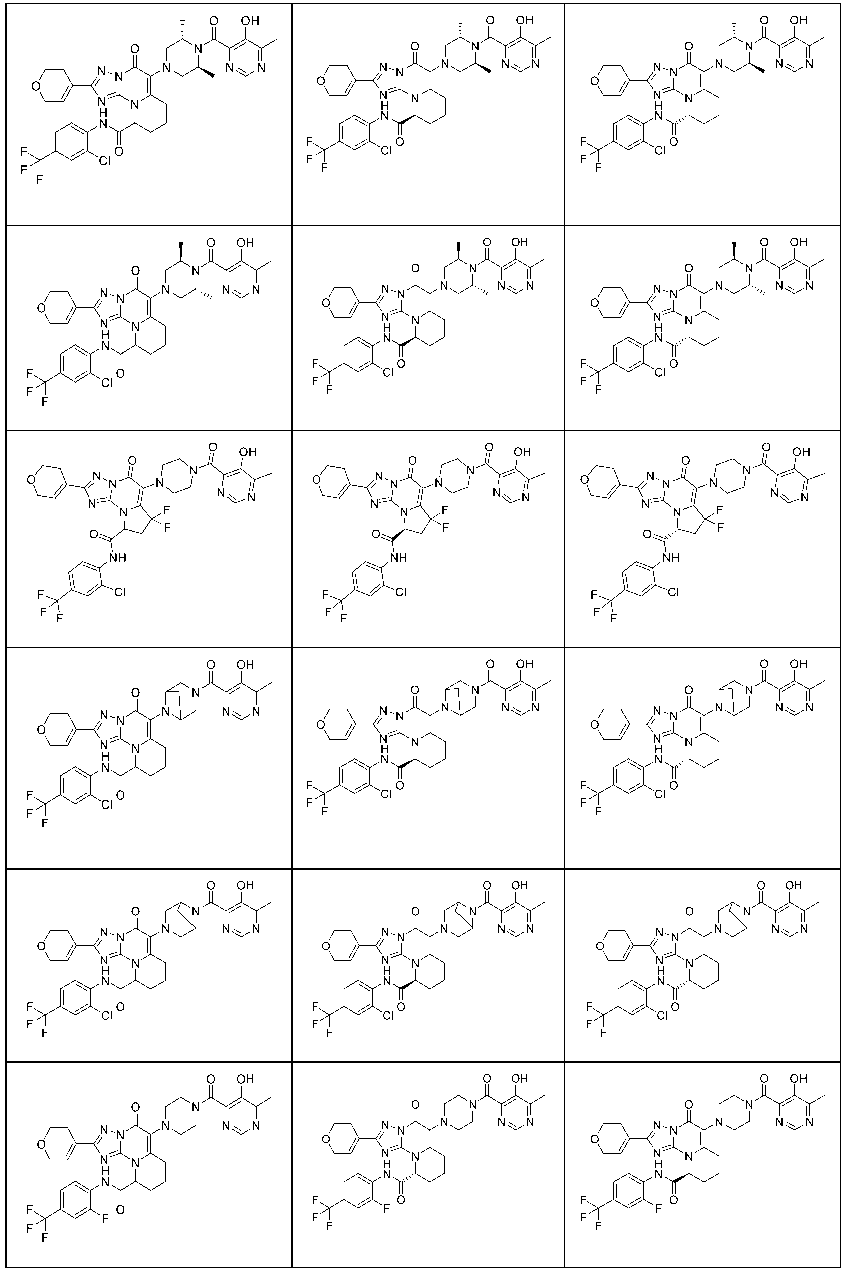

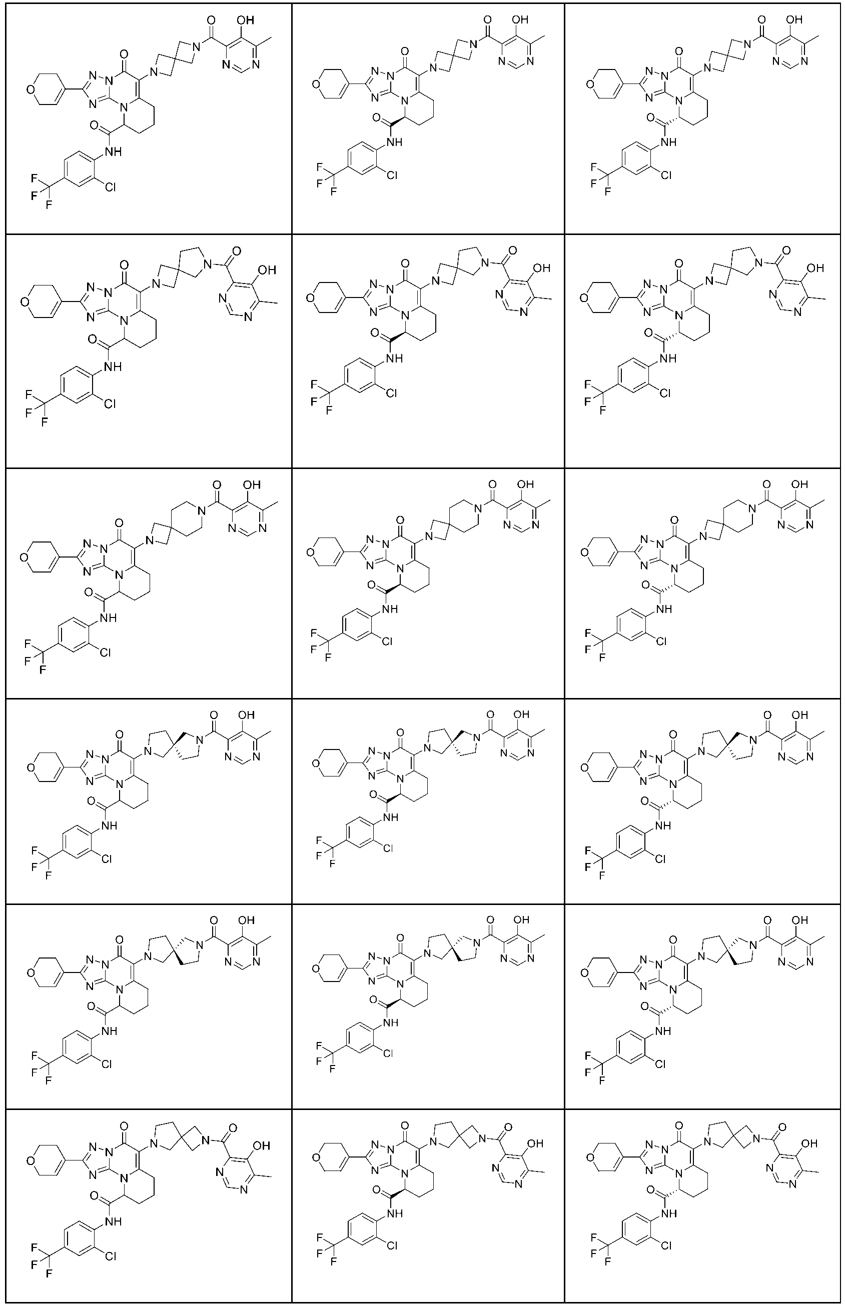

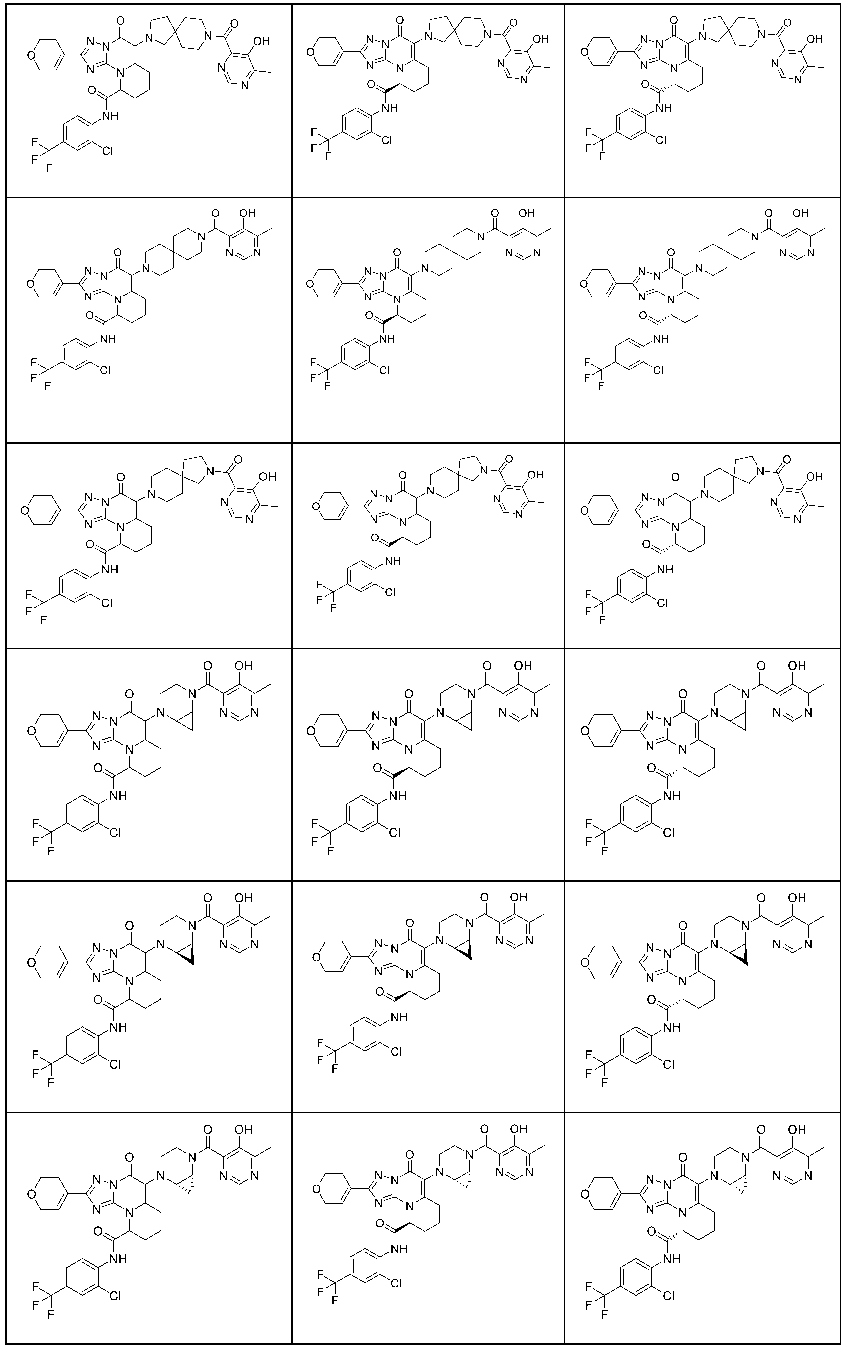

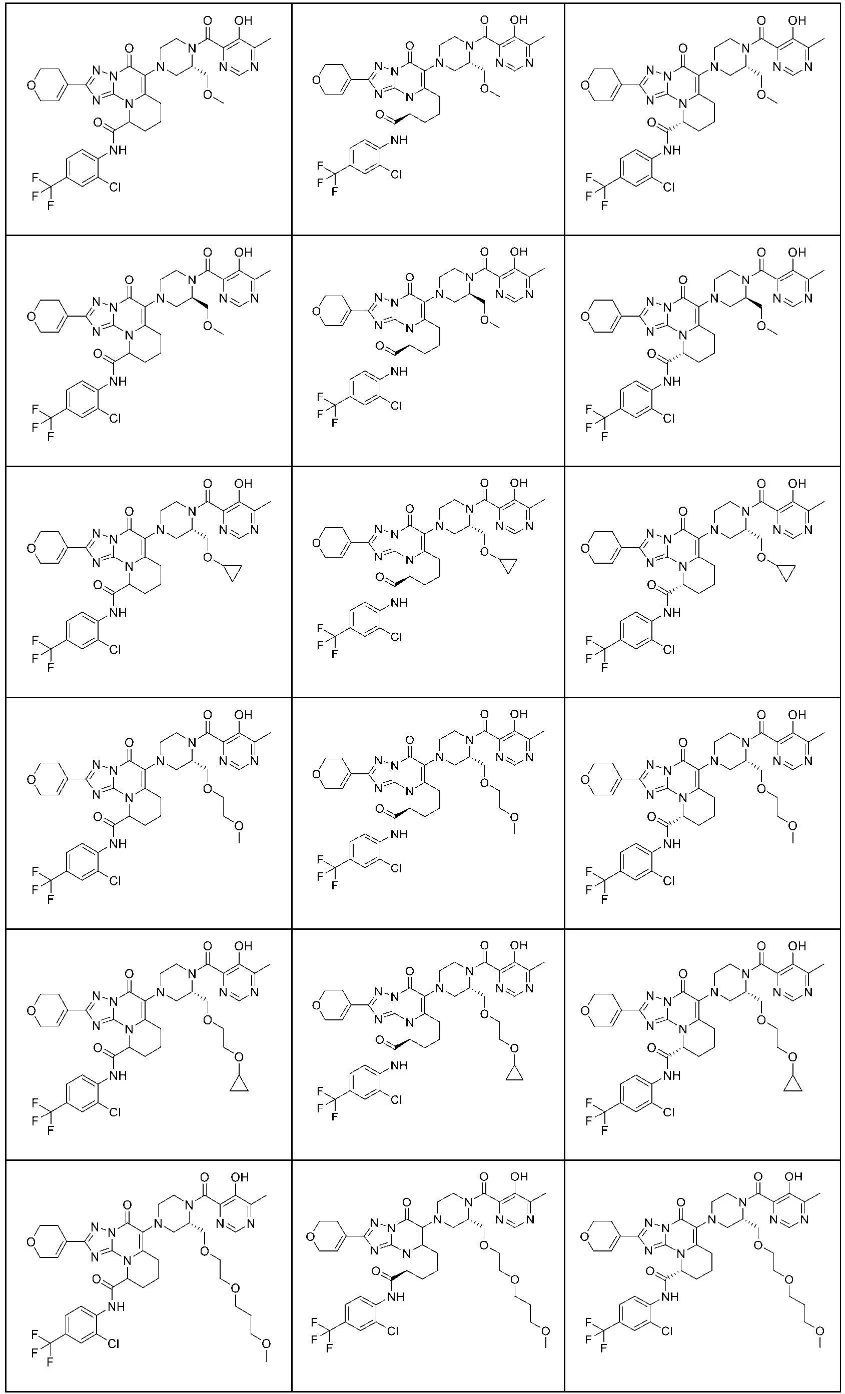

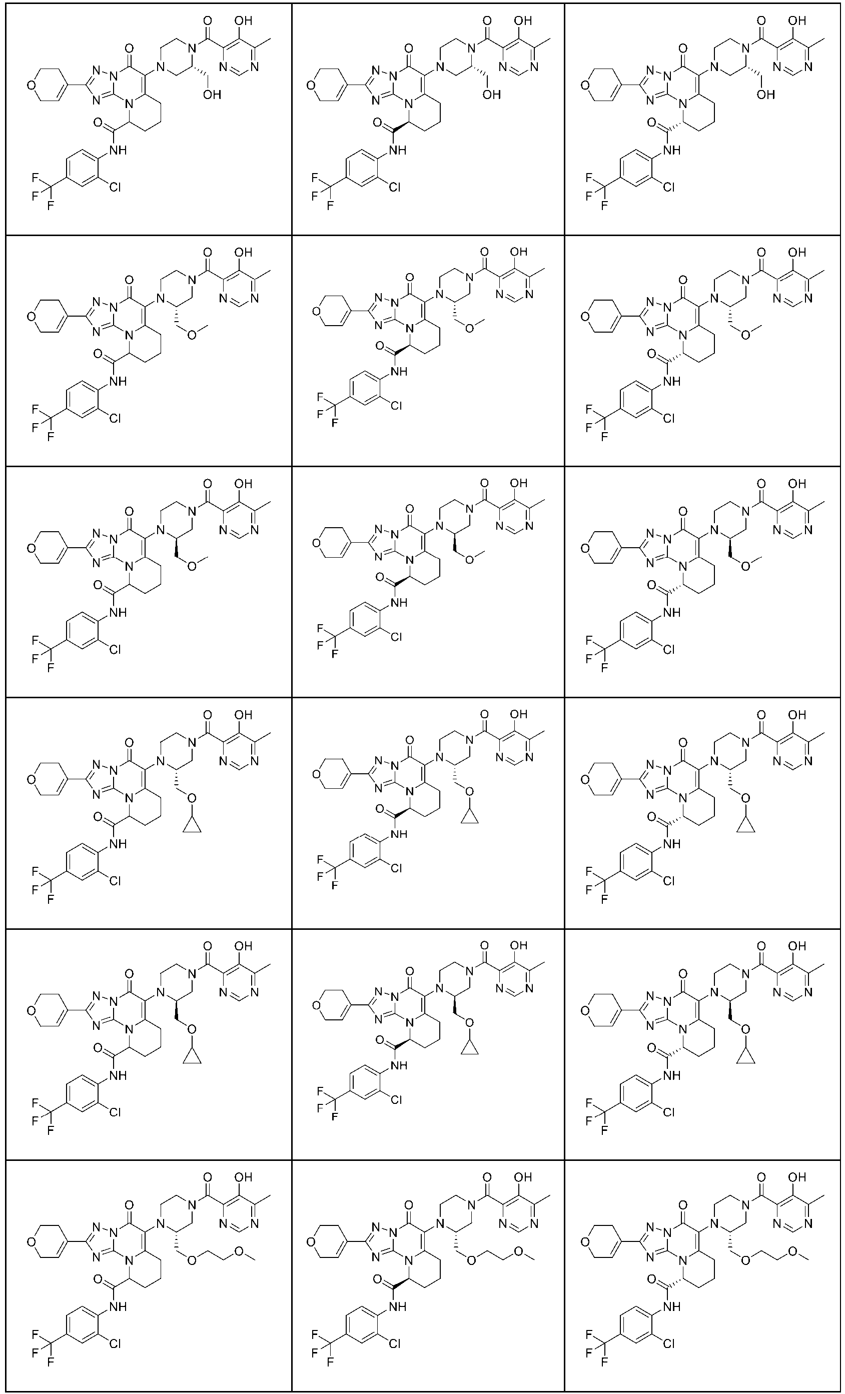

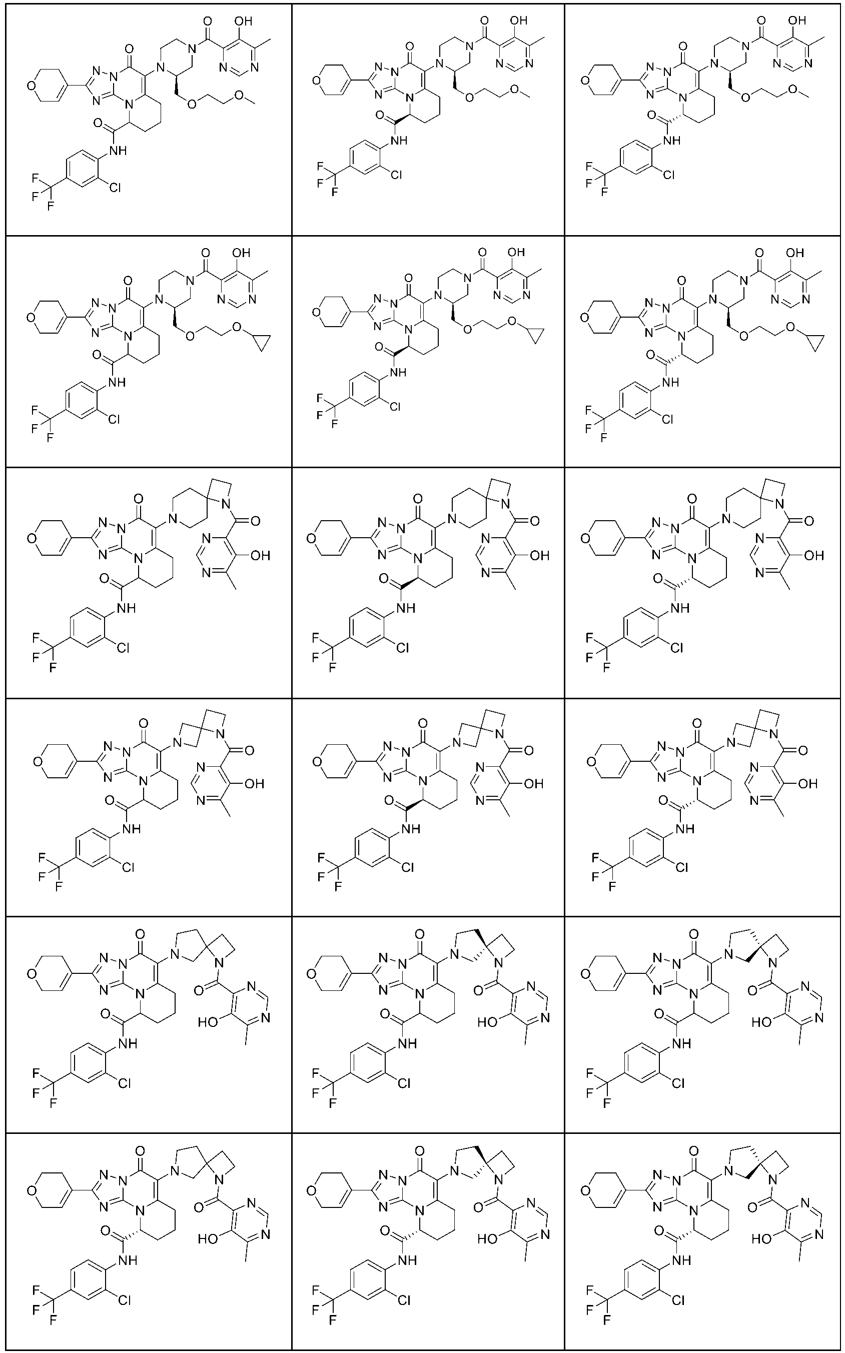

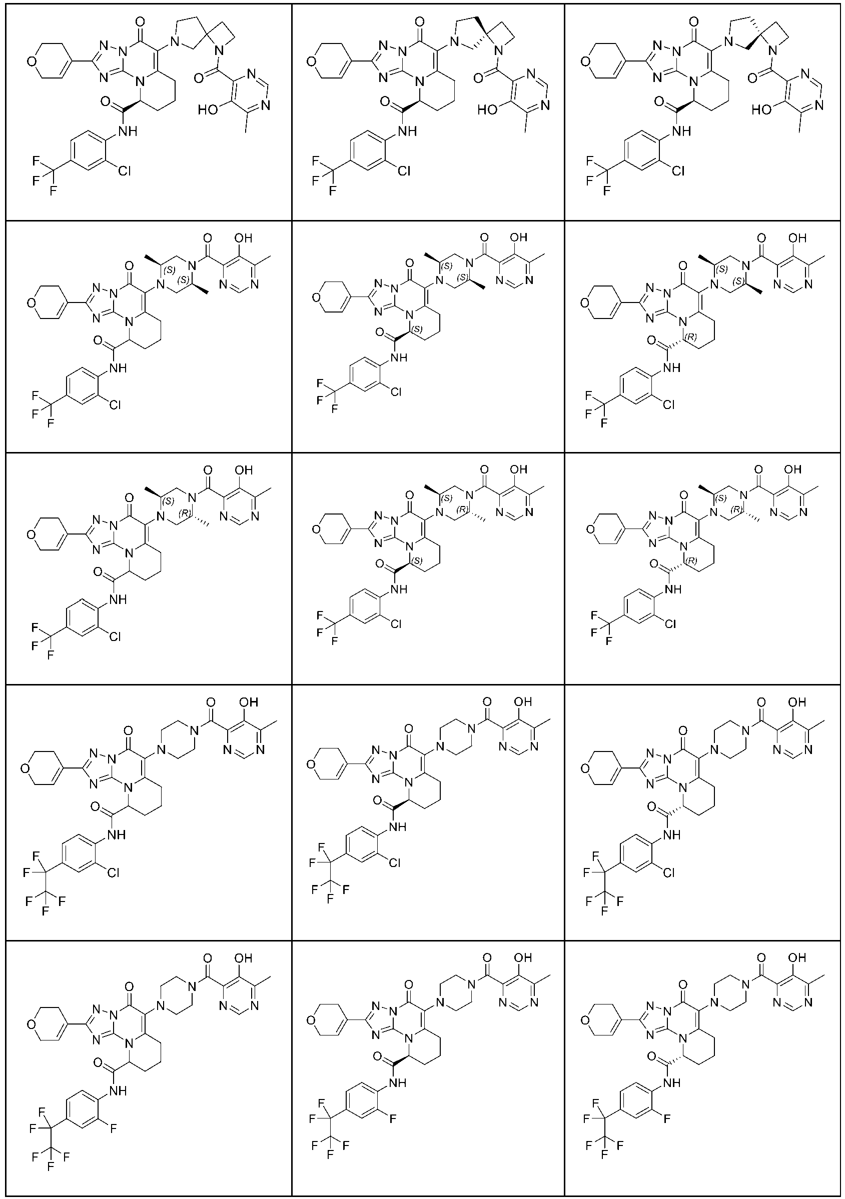

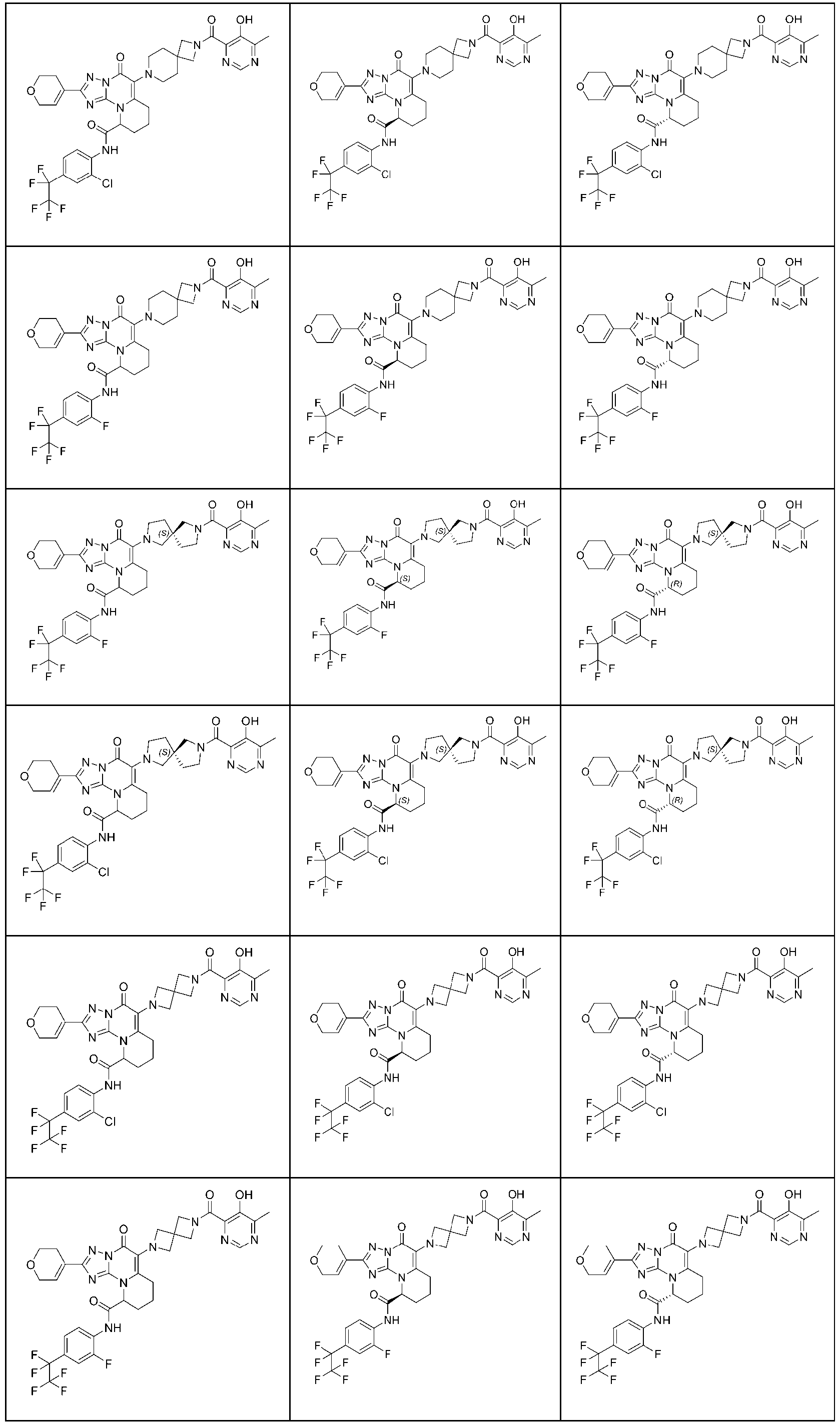

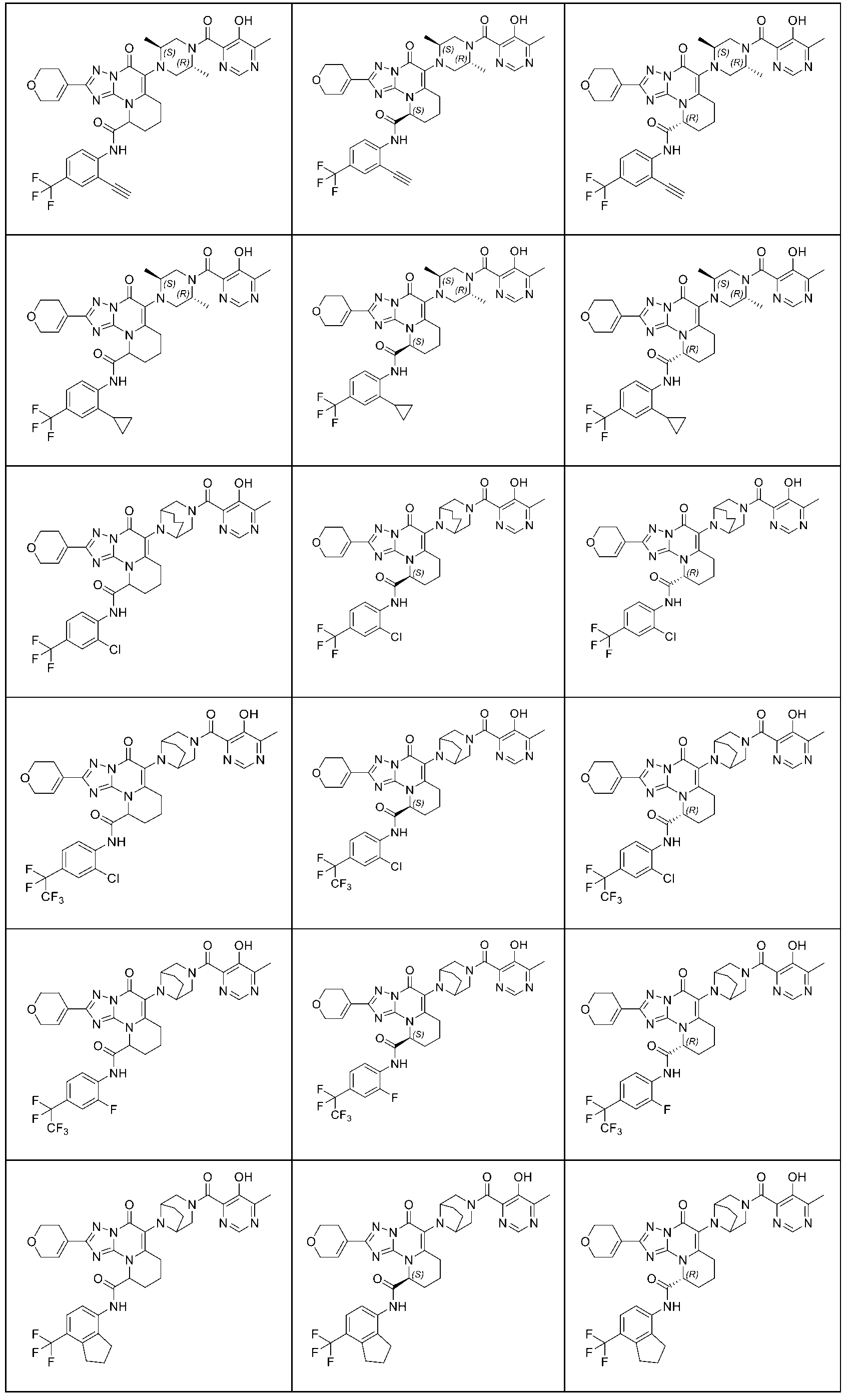

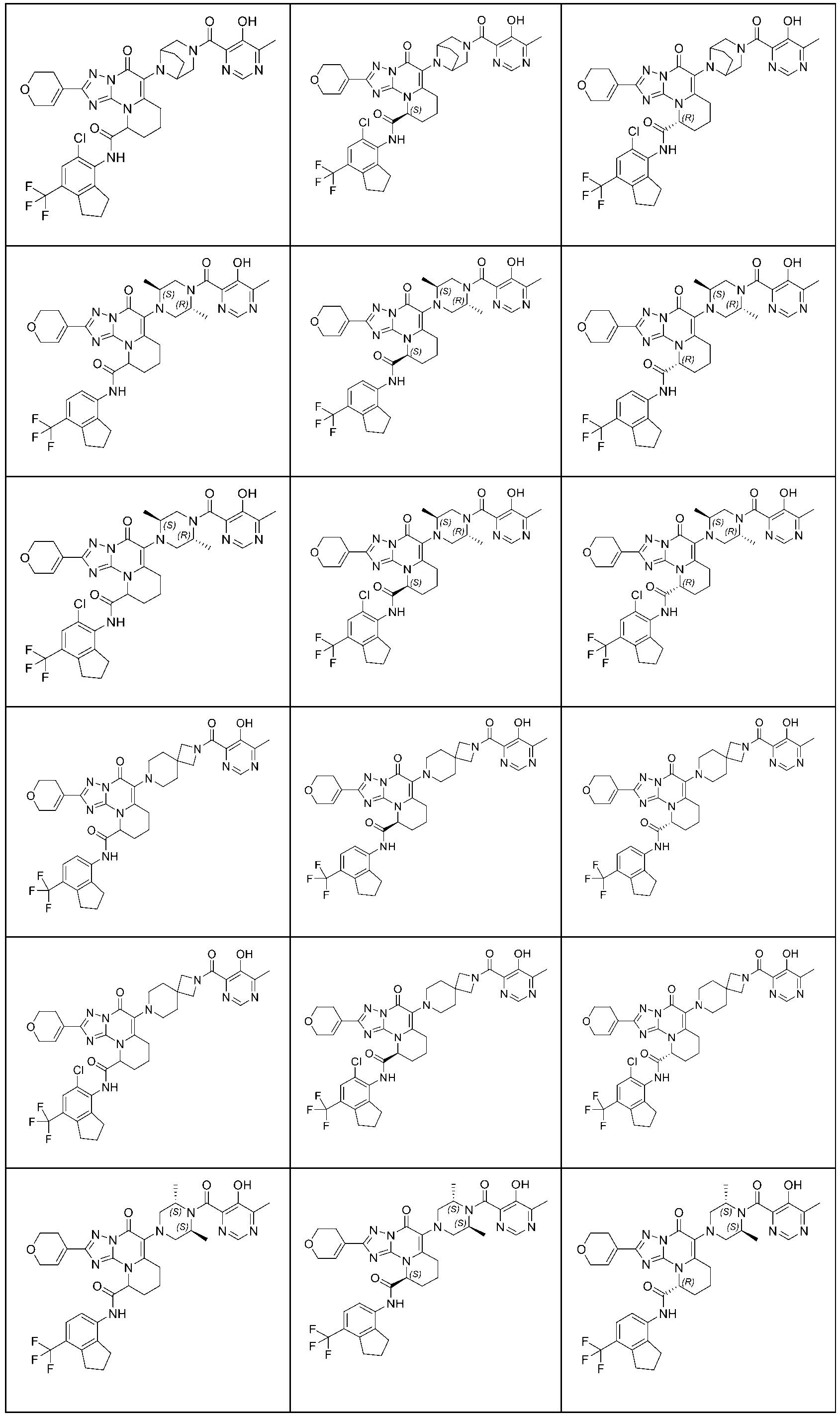

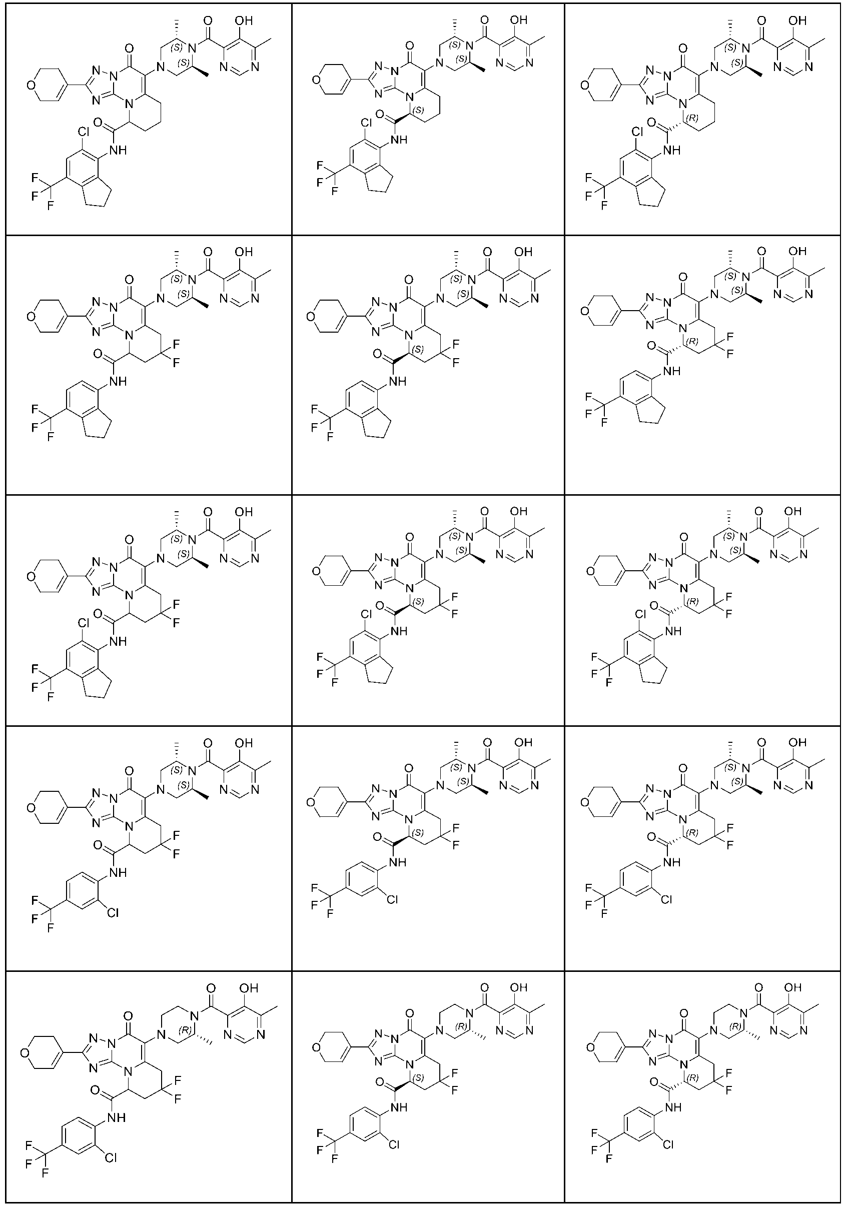

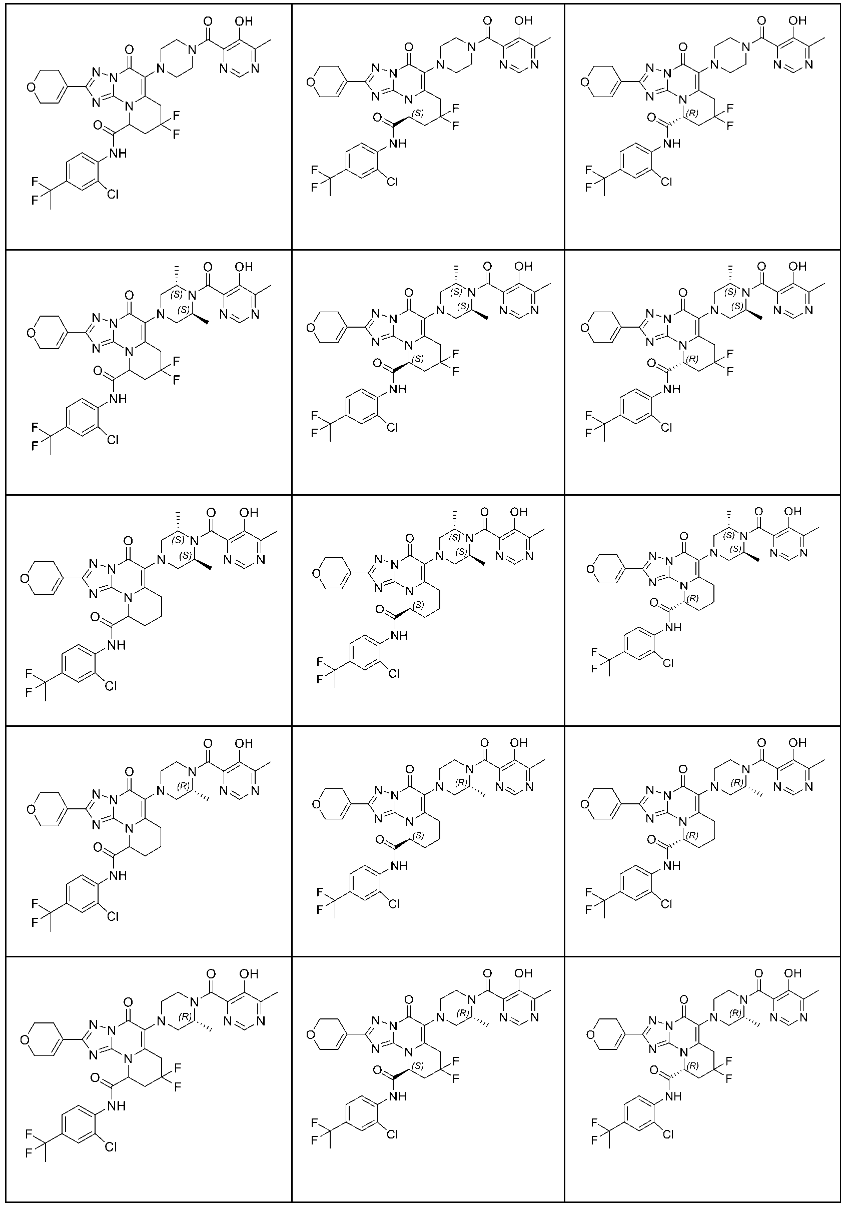

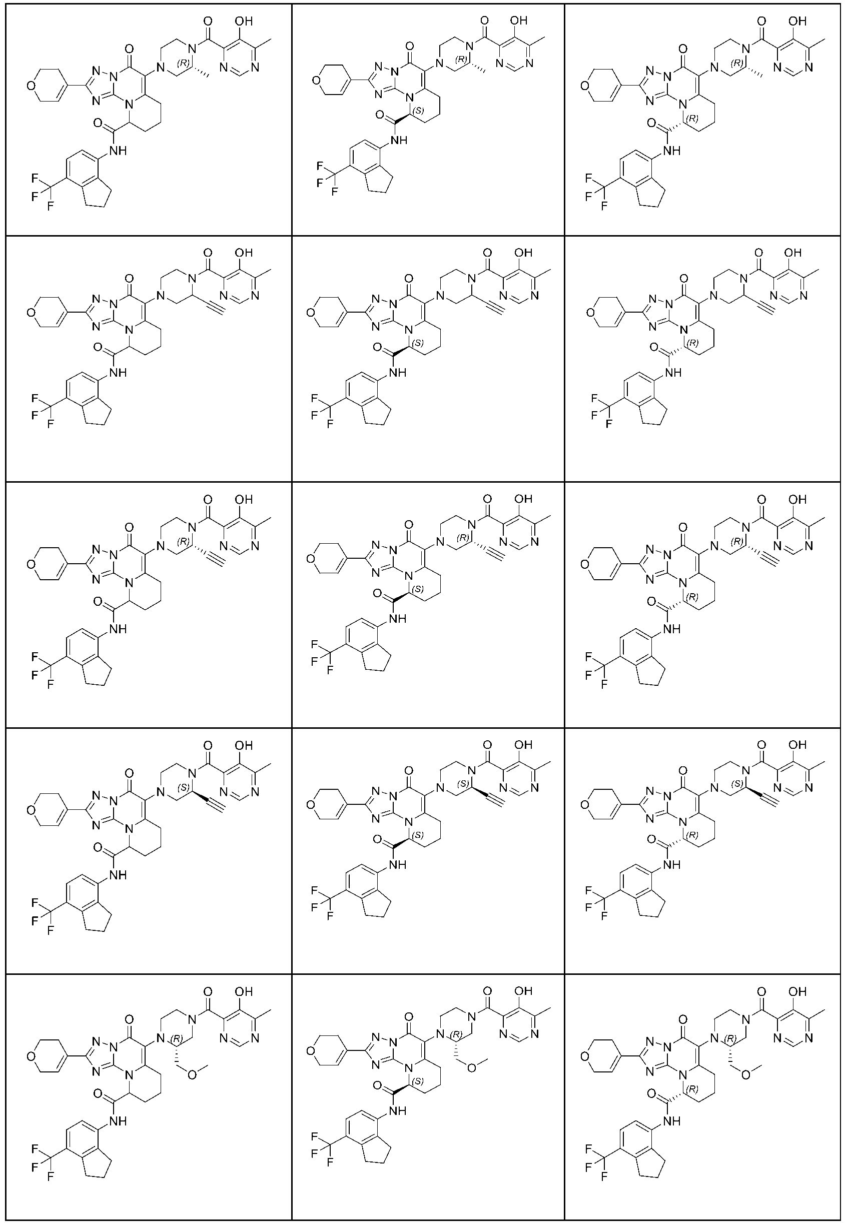

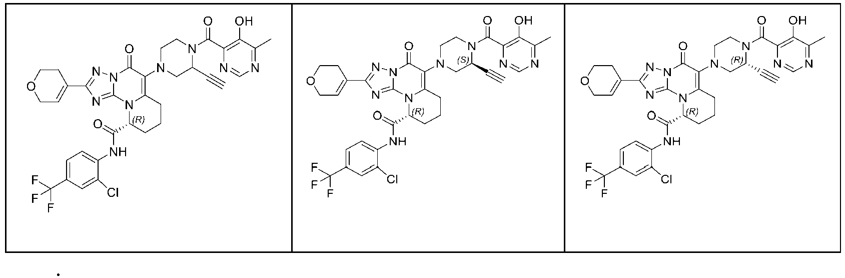

The present invention relates to a pharmaceutical composition comprising any of the compounds described above, or the stereoisomers, tautomers, deuterated compounds, solvates, prodrugs, metabolites, pharmaceutically acceptable salts, or co-crystals thereof, and a pharmaceutically acceptable carrier.

The present invention relates to a pharmaceutical composition comprising a therapeutically effective amount of the compound, or the stereoisomer, deuterated compound, solvate, prodrug, metabolite, pharmaceutically acceptable salt, or co-crystal thereof described above in the present invention, and a pharmaceutically acceptable carrier.

In some embodiments, the pharmaceutical composition of the present invention can be in a unit preparation form (the amount of the active ingredient in the unit preparation is also referred to as the "preparation specification").

The term "effective amount" or "therapeutically effective amount" according to the present application refers to a sufficient amount of the compound disclosed in the present application that is administered to ameliorate, to some extent, one or more symptoms of a disease or condition (e.g., a disease associated with WRN abnormality, such as cancer) being treated. In some embodiments, the outcome is the reduction and/or remission of signs, symptoms, or causes of the disease, or any other desired change in the biological system. For example, an "effective amount" for therapeutic use is an amount of the compound disclosed in the present application that is required to provide a clinically significant reduction of the symptoms of the disease. Examples of the therapeutically effective amount include, but are not limited to 1-1500 mg, 1-1200 mg, 1-1000 mg, 1-900 mg, 1-800 mg, 1-700 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 80-1000 mg, or 80-800 mg.

In some embodiments, the pharmaceutical composition comprises the compound, or the stereoisomer, tautomer, deuterated compound, solvate, prodrug, metabolite, pharmaceutically acceptable salt, or co-crystal thereof of the present invention in an amount including, but not limited to 1-1000 mg, 20-800 mg, 40-800 mg, 40-400 mg, 25-200 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 300 mg, 320 mg, 400 mg, 480 mg, 500 mg, 600 mg, 640 mg, or 840 mg.

Provided is a method for treating a disease in a mammal, comprising administering to the subject a therapeutically effective amount of the compound, or the stereoisomer, tautomer, deuterated compound, solvate, prodrug, metabolite, pharmaceutically acceptable salt, or co-crystal thereof of the present invention, wherein the therapeutically effective amount is preferably 1-1500 mg, and the disease is preferably a disease associated with the activity or expression level of WRN (e.g., a tumor, preferably MSI-H solid tumor or MMRD solid tumor).

Provided is a method for treating a disease in a mammal, comprising administering to the subject a medicament, i.e., the compound, or the stereoisomer, tautomer, deuterated compound, solvate, prodrug, metabolite, pharmaceutically acceptable salt, or co-crystal thereof of the present invention in a daily dose of 1-1000 mg/day, wherein the daily dose can be a single dose or divided doses. In some embodiments, the daily dose includes, but is not limited to 10-1500 mg/day, 10-1000 mg/day, 10-800 mg/day, 25-800 mg/day, 50-800 mg/day, 100-800 mg/day, 200-800 mg/day, 25-400 mg/day, 50-400 mg/day, 100-400 mg/day, or 200-400 mg/day. In some embodiments, the daily dose includes, but is not limited to 10 mg/day, 20 mg/day, 25 mg/day, 50 mg/day, 80 mg/day, 100 mg/day, 125 mg/day, 150 mg/day, 160 mg/day, 200 mg/day, 300 mg/day, 320 mg/day, 400 mg/day, 480 mg/day, 600 mg/day, 640 mg/day, 800 mg/day, or 1000 mg/day.

The present invention relates to a kit, wherein the kit may comprise a composition in the form of a single dose or multiple doses and comprises the compound, or the stereoisomer, tautomer, deuterated compound, solvate, prodrug, metabolite, pharmaceutically acceptable salt, or co-crystal thereof of the present invention, and the amount of the compound, or the stereoisomer, tautomer, deuterated compound, solvate, prodrug, metabolite, pharmaceutically acceptable salt, or co-crystal thereof of the present invention is identical to the amount of same in the pharmaceutical composition described above.

The present invention relates to the use of any of the compounds described above, or the stereoisomers, tautomers, deuterated compounds, solvates, prodrugs, metabolites, pharmaceutically acceptable salts, or co-crystals thereof in the preparation of a medicament for treating a disease associated with the activity or expression level of WRN, wherein preferably, the disease is selected from tumors, further preferably from MSI-H solid tumors or MMRD solid tumors.

The present invention relates to the use of the pharmaceutical composition described above in the preparation of a medicament for treating a disease associated with the activity or expression level of WRN, wherein preferably, the disease is selected from tumors, preferably from MSI-H solid tumors or MMRD solid tumors.

The amount of the compound, or the stereoisomer, tautomer, deuterated compound, solvate, prodrug, metabolite, pharmaceutically acceptable salt, or co-crystal thereof of the present invention is calculated in the form of free base in each case.

Unless otherwise stated, the terms used in the description and claims have the following meanings.

The carbon, hydrogen, oxygen, sulfur, nitrogen, F, Cl, Br and I involved in the groups and compounds of the present invention all comprise their isotopes, and the carbon, hydrogen, oxygen, sulfur or nitrogen involved in the groups and compounds of the present invention is optionally further replaced with one or more of their corresponding isotopes, wherein the isotopes of carbon comprise ¹²C, ¹³C and ¹⁴C, the isotopes of hydrogen comprise protium (H), deuterium (D, also known as heavy hydrogen) and tritium (T, also known as superheavy hydrogen), the isotopes of oxygen comprise ¹⁶O, ¹⁷O and ¹⁸O, the isotopes of sulfur comprise ³²S, ³³S, ³⁴S and ³⁶S, the isotopes of nitrogen comprise ¹⁴N and ¹⁵N, the isotopes of fluorine comprise ¹⁷F and ¹⁹F, the isotopes of chlorine comprise ³⁵Cl and ³⁷Cl, and the isotopes of bromine comprise ⁷⁹Br and ⁸¹Br.

"CN" refers to cyano.

"Halogen" refers to F, Cl, Br or I.

"Halogen-substituted" refers to substitution with F, Cl, Br, or I, including but not limited to substitution with 1 to 10 substituents selected from F, Cl, Br, or I, or substitution with 1 to 6 substituents selected from F, Cl, Br, or I, or substitution with 1 to 4 substituents selected from F, Cl, Br, or I. "Halogen-substituted" is referred to simply as "halo".

"Alkyl" refers to a substituted or unsubstituted linear or branched saturated aliphatic hydrocarbon group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl and various branched isomers thereof. The alkyl can be monovalent, divalent, trivalent or tetravalent.

"Alkylene" refers to a substituted or unsubstituted linear or branched divalent saturated hydrocarbon group, including -(CH₂)ᵥ- (v is an integer from 1 to 10), and examples of alkylene include, but are not limited to, methylene, ethylene, propylene, butylene, etc.

"Cycloalkyl" refers to a substituted or unsubstituted saturated carbocyclic hydrocarbon group, typically having 3 to 12 carbon atoms, and the cycloalkyl can be monocyclic, fused, bridged or spirocyclic. Non-limiting examples include cyclopropyl, cyclobutyl, cyclopentyl, cyclohexyl, cycloheptyl, cyclobutyl fused cyclobutyl, cyclobutyl spiro cyclobutyl, adamantane, etc. The cycloalkyl can be monovalent, divalent, trivalent or tetravalent.

"Heterocycloalkyl" refers to a substituted or unsubstituted saturated heteroatom-containing cyclic hydrocarbon group, including but not limited to 3 to 12 atoms, 3 to 8 atoms, or 1 to 3 heteroatoms selected from N, O, S, or Se. The C, N and S in the ring of the heterocycloalkyl can be oxidized to various oxidation states. The heterocycloalkyl can be monocyclic, fused, bridged or spirocyclic. The heterocycloalkyl can be connected to a heteroatom or a carbon atom, and non-limiting examples include oxiranyl, aziridinyl, oxetanyl, azetidinyl, tetrahydrofuryl, tetrahydro-2H-pyranyl, dioxolanyl, dioxanyl, pyrrolidyl, piperidyl, imidazolidinyl, oxazolidinyl, oxazinanyl, morpholinyl, hexahydropyrimidyl, piperazinyl, 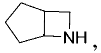 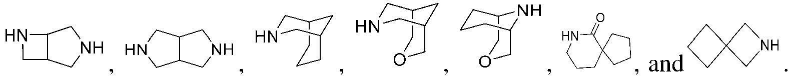 The heterocycloalkyl can be monovalent, divalent, trivalent or tetravalent.

"Alkenyl" refers to a substituted or unsubstituted linear or branched unsaturated hydrocarbon group, having at least 1, usually 1, 2 or 3 carbon-carbon double bonds, with a main chain including but not limited to 2 to 10, 2 to 6, or 2 to 4 carbon atoms. Examples of alkenyl include, but are not limited to, ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc. The alkenyl can be monovalent, divalent, trivalent or tetravalent.

"Alkynyl" refers to a substituted or unsubstituted linear or branched unsaturated hydrocarbon group, having at least 1, usually 1, 2 or 3 carbon-carbon triple bonds, with a main chain including 2 to 10 carbon atoms, including but not limited to a main chain including 2 to 6 carbon atoms, or a main chain including 2 to 4 carbon atoms. Examples of alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5-hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, etc. The alkynyl can be monovalent, divalent, trivalent or tetravalent.

"Alkoxy" refers to substituted or unsubstituted -O-alkyl. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocyclic ring" refers to a substituted or unsubstituted aromatic ring or non-aromatic ring, wherein the aromatic ring or non-aromatic ring can be a 3- to 8-membered monocyclic ring, 4- to 12-membered bicyclic ring, 10-to 15-membered tricyclic ring, or 12- to 18-membered tetracyclic ring system. The carbocyclyl can be connected to an aromatic ring or non-aromatic ring, and the ring is optionally monocyclic, fused, bridged or spirocyclic. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring, 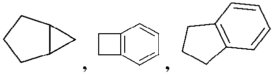 or 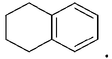 The "carbocyclyl" or "carbocyclic ring" can be monovalent, divalent, trivalent or tetravalent.

"Heterocyclyl" or "heterocyclic ring" refers to a substituted or unsubstituted aromatic ring or non-aromatic ring, wherein the aromatic ring or non-aromatic ring can be a 3- to 8-membered monocyclic ring, 4- to 12-membered bicyclic ring, 10-to 15-membered tricyclic ring, or 12- to 18-membered tetracyclic ring system, and contains 1 or more (including but not limited to 2, 3, 4 or 5) heteroatoms selected from N, O, S or Se, and C, N, S, or Se (which may be selectively substituted) in the ring of the heterocyclyl can be oxidized to various oxidation states. Heterocyclyl can be connected to a heteroatom or a carbon atom; heterocyclyl can be connected to an aromatic ring or a non-aromatic ring; and heterocyclyl is optionally monocyclic, bridged, fused, or spiro. Non-limiting examples include oxiranyl, aziridinyl, oxetanyl, azetidinyl, 1,3-dioxolanyl, 1,4-dioxolanyl, 1,3-dioxanyl, azepanyl, pyridyl, furyl, thienyl, pyranyl, N-alkylpyrrolyl, pyrimidyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzoimidazolyl, benzothiazolyl, benzoxazolyl, benzopyridyl, benzopyrimidyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl, 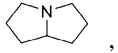 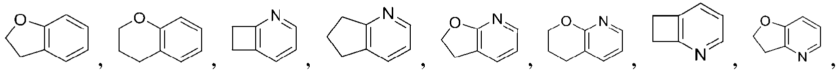 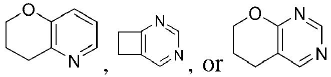 . The "heterocyclyl" or "heterocyclic ring" can be monovalent, divalent, trivalent or tetravalent.

"Spiro ring" or "spiro ring group" refers to a polycyclic group that shares one atom (called a spiro atom) between substituted or unsubstituted monocyclic rings. The number of ring atoms in the spiro ring system includes but is not limited to 5 to 20, 6 to 14, 6 to 12, or 6 to 10, wherein one or more rings may contain 0 or more (including but not limited to 1, 2, 3 or 4) double bonds, and may optionally contain 0 to 5 heteroatoms selected from N, O or S(=O)ₙ (n is 0, 1, or 2). 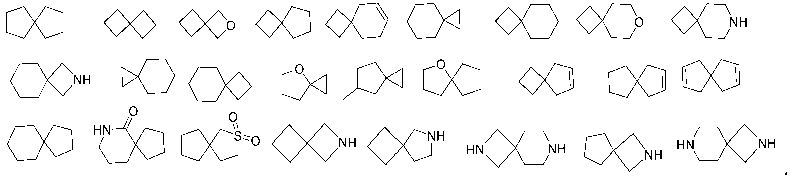 The "spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, S(=O)ₙ or O, wherein n is 0, 1 or 2). The number of ring atoms in the fused ring system includes, but is not limited to 5 to 20, 5 to 14, 5 to 12, or 5 to 10. Non-limiting examples include: 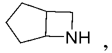 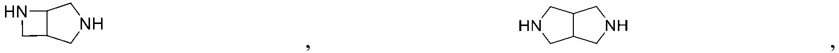 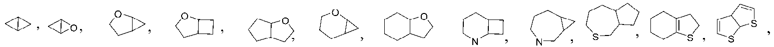 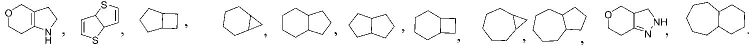 The "fused ring" or "fused ring group" can be monovalent, divalent, trivalent or tetravalent.

"Bridged ring" or "bridged ring group" means a substituted or unsubstituted polycyclic group containing any two atoms that are not directly connected, and may contain 0 or more double bonds. Any ring in the bridged ring system may contain 0 to 5 groups selected from heteroatoms or groups containing heteroatoms (including but not limited to N, S(=O)n or O, wherein n is 0, 1 or 2). The number of ring atoms includes, but is not limited to 5 to 20, 5 to 14, 5 to 12, or 5 to 10. Non-limiting examples include 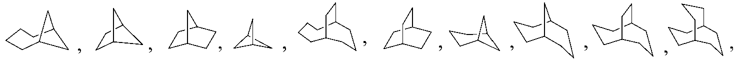 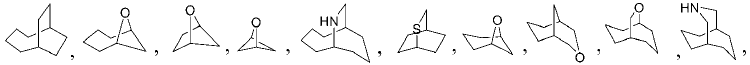 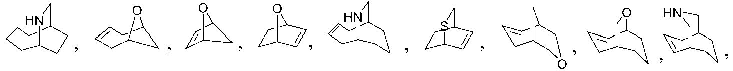 cubane, or adamantane. The "bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms.

"Carbo-fused ring", "fused ring carbocyclyl", "fused carbocyclyl" or "carbo-fused ring group" refers to a "fused ring" with a ring system consisting only of carbon atoms.

"Carbo-bridged ring", "bridged ring carbocyclyl", "bridged carbocyclyl" or "carbo-bridged ring group" refers to a "bridged ring" with a ring system consisting only of carbon atoms.

"Mono-heterocyclic ring", "monocyclic heterocyclyl" or "mono-heterocyclyl" refers to "heterocyclyl" or "heterocyclic ring" with a monocyclic system.

"Fused-heterocyclic ring", "fused-heterocyclyl", "fused ring heterocyclyl" or "fused-heterocyclic ring group" refers to a "fused ring" containing a heteroatom.

"Spiro-heterocyclic ring", "spiro-heterocyclyl", "spiro ring heterocyclyl" or "spiro-heterocyclic ring group" refers to a "spiro ring" containing a heteroatom.

"Bridged-heterocyclic ring", "bridged-heterocyclyl", "bridged ring heterocyclyl" or "bridged-heterocyclic ring group" refers to a "bridged ring" containing a heteroatom.

"Aryl" or "aromatic ring" refers to a substituted or unsubstituted aromatic hydrocarbon group with a monocyclic ring or a fused ring, wherein the number of ring atoms in the aromatic ring includes, but is not limited to 6 to 18, 6 to 12, or 6 to 10 carbon atoms. The aryl ring can be fused to a saturated or unsaturated carbocyclic ring, wherein the ring connected to the parent structure is an aryl ring. Non-limiting examples include a benzene ring, a naphthalene ring, and 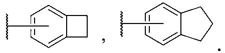 The "aryl" or "aromatic ring" can be monovalent, divalent, trivalent or tetravalent. When divalent, trivalent or tetravalent, the point of connection is on the aryl ring.

"Heteroaryl" or "heteroaromatic ring" refers to a substituted or unsubstituted aromatic hydrocarbon group containing 1 to 5 heteroatoms or groups containing heteroatoms (including but not limited to N, O, S(=O)n, or Se(=O)n, wherein n is 0, 1 or 2), wherein the number of ring atoms in the heteroaromatic ring includes, but is not limited to, 5 to 15, 5 to 10, or 5 to 6. The atoms C, N, and S in the ring are optionally oxidized (i.e., C(=O), NO, S(=O)n, and Se(=O)n, wherein n is 1 or 2). Non-limiting examples of heteroaryl include, but are not limited to pyridyl, furyl, thienyl, selenophenyl, pyridyl, pyranyl, N-alkylpyrrolyl, pyrimidyl, pyrazinyl, pyridazinyl, imidazolyl, benzopyrazolyl, benzimidazolyl, benzopyridyl, pyrrolopyridyl, pyridonyl, etc. The heteroaryl ring can be fused to a saturated or unsaturated carbocyclic ring or heterocyclic ring, wherein the ring connected to the parent structure is an aryl ring. Non-limiting examples include 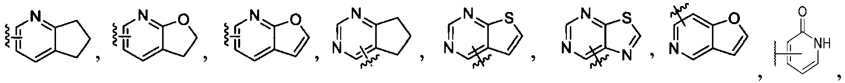 and 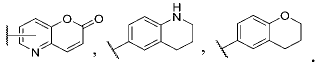 The definition of the heteroaryl herein is consistent with this definition. The heteroaryl can be monovalent, divalent, trivalent or tetravalent. When divalent, trivalent or tetravalent, the points of connection are on the aromatic ring.

"Substitution" or "substituted" refers to substitution with 1 or more (including but not limited to 2, 3, 4 or 5) substituents including but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, a bridged ring group, a spiro ring group, a fused ring group, hydroxyalkyl, =O, carbonyl, aldehyde, carboxylic acid, carboxylate, -(CH₂)ₘ-C(=O)-R^{a}, -O-(CH₂)ₘ-C(=O)-R^{a}, -(CH₂)ₘ-C(=O)-NR^{b}R^{c}, -(CH₂)ₘS(=O)ₙR^{a}, -(CH₂)ₘ-alkenyl-R^{a}, OR^{d}, -(CH₂)ₘ-alkynyl-R^{a} (wherein m and n are 0, 1 or 2), arylthio, thiocarbonyl, silyl, -NR^{b}R^{c}, etc., wherein R^{b} and R^{c} are independently selected from H, hydroxyl, amino, carbonyl, alkyl, alkoxy, cycloalkyl, heterocyclyl, aryl, heteroaryl, sulfonyl, or trifluoromethylsulfonyl; alternatively, R^{b} and R^{c} may form five- or six-membered cycloalkyl or heterocyclyl; R^{a} and R^{d} are each independently selected from aryl, heteroaryl, alkyl, alkoxy, cycloalkyl, heterocyclyl, carbonyl, an ester group, a bridged ring group, a spiro ring group or a fused ring group.

"Substituted with 1 to X substituents selected from ..." refers to a substitution with 1, 2, 3 ... X substituents selected from ..., wherein X is selected from any integer between 1 and 10. For example, "substituted with 1 to 4 R^{k}" refers to a substitution with 1, 2, 3 or 4 R^{k}. For example, "substituted with 1 to 5 substituents selected from ..." refers to a substitution with 1, 2, 3, 4 or 5 substituents selected from ... For example, "bridged-heterocyclic ring is optionally substituted with 1 to 4 substituents selected from H or F" means that the bridged-heterocyclic ring is optionally substituted with 1, 2, 3 or 4 substituents selected from H or F.

An X- to Y-membered ring (X and Y are integers, 3 ≤ X < Y, and X < Y ≤ 20 (i.e., any integer from 4 to 20)) includes X-, X+1-, X+2-, X+3-, X+4-,...or Y-membered rings. Rings include a heterocyclic ring, a carbocyclic ring, an aromatic ring, aryl, heteroaryl, cycloalkyl, a mono-heterocyclic ring, a fused-heterocyclic ring, a spiro-heterocyclic ring, or a bridged-heterocyclic ring. For example, a "4- to 7-membered mono-heterocyclic ring" refers to a 4-, 5-, 6- or 7-membered mono-heterocyclic ring, and a "5- to 10-membered fused-heterocyclic ring" refers to a 5-, 6-, 7-, 8-, 9- or 10-membered fused-heterocyclic ring.

A C_{x-y} carbocyclic ring (including aryl, cycloalkyl, a mono-carbocyclic ring, a spiro-carbocyclic ring, a fused-carbocyclic ring, or a bridged-carbocyclic ring) includes Cₓ-, Cₓ₊₁-, Cₓ₊₂-, Cₓ₊₃-, Cₓ₊₄-,...or C_{y}-membered rings (x is an integer, 3 ≤ x < y, and y is any integer from 4 to 20). For example, "C₃₋₆ cycloalkyl" refers to C₃, C₄, C₅ or C₆ cycloalkyl.

When a group has one or more connectable sites, any one or more sites of the group can be connected to other groups via chemical bonds. When the connection mode of the chemical bond is indeterminate and there are hydrogen atoms at the connectable sites, the number of H atoms at the site will decrease correspondingly with the number of connected chemical bonds when the chemical bond is connected, forming a group with the corresponding valence. For example, 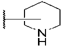 indicates that any connectable site on the piperidyl group can be connected to other groups via one chemical bond, including at least 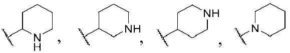 these 4 connection modes. Even if an H atom is shown on the N atom, 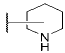 also includes 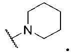 For example, 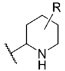 indicates that the R group on the piperidyl group can be located on C or N, including at least

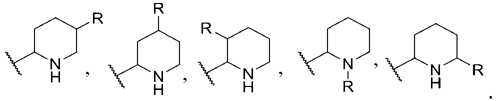

When the connecting groups listed do not specify their connection direction, their connection directions include both the left-to-right and right-to-left reading orders. For example, for A-L-B, wherein L is -M-W-, it includes both A-M-W-B and A-W-M-B.

The term "optional" or "optionally" means that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutically acceptable salt" or "pharmaceutically acceptable salt thereof" refers to a salt of the compound of the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Pharmaceutical composition" refers to a mixture of one or more compounds of the present invention, or stereoisomers, tautomers, deuterated compounds, solvates, prodrugs, metabolites, pharmaceutically acceptable salts or co-crystals thereof and other chemical components, wherein "other chemical components" refer to pharmaceutically acceptable carriers, excipients and/or one or more other therapeutic agents.

The term "preparation strength" refers to the weight of the active ingredient contained in each vial, tablet or other unit preparation.

"Carrier" refers to a material that does not cause significant irritation to an organism and does not eliminate the biological activity and characteristics of a compound administered.

"Prodrug" refers to a compound that can be converted into the compound of the present invention with the biological activity by metabolism *in vivo.* The prodrug of the present invention is prepared by modifying an amino or carboxyl group in the compound of the present invention, and the modification can be removed by conventional operations or removed in vivo to obtain a parent compound. When the prodrug of the present invention is administered to a mammalian individual, the prodrug is split to form a free amino or carboxyl group.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

"Animal" is meant to include mammals, such as humans, companion animals, zoo animals, and domestic animals, preferably humans, horses, or dogs.

"Stereoisomer" refers to an isomer produced as a result of different spatial arrangement of atoms in molecules, including cis-trans isomers, enantiomers, diastereomers and conformational isomers.

"Tautomer" refers to a functional group isomer produced by the rapid movement of an atom in two positions in a molecule, such as keto-enol isomerization and amide-imino alcohol isomerization.

"IC₅₀" refers to the concentration of a medicament or inhibitor required to inhibit half of a given biological process (or a component of the process such as an enzyme, a receptor and a cell).

### Detailed Description of Embodiments

The technical solutions of the present invention will be described in detail by the following examples, but the scope of protection of the present invention includes but is not limited thereto.

The structures of the compounds are determined by nuclear magnetic resonance (NMR) or (and) mass spectrometry (MS). The NMR shift (δ) is given in the unit of 10⁻⁶ (ppm). NMR is measured with NMR instruments (Bruker Avance III 400 and Bruker Avance 300), and the solvent for determination is deuterated dimethyl sulfoxide (DMSO-d*₆*), deuterated chloroform (CDCl₃), and deuterated methanol (CD₃OD), and the internal standard is tetramethylsilane (TMS);

MS is determined with Agilent 6120B (ESI) and Agilent 6120B (APCI); and

HPLC is determined with Agilent 1260DAD high pressure liquid chromatograph (Zorbax SB-C18 100 × 4.6 mm, 3.5 µM).

Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm-0.5 mm;
and for the column chromatography, Yantai Huanghai silica gel of 200-300 mesh silica gel is generally used as a carrier.

To achieve the objectives of the present invention, the compounds used in the reactions described herein are prepared according to organic synthesis techniques known to those skilled in the art, and starting from commercially available chemicals and/or compounds described in chemical documents. The "commercially available chemicals" are obtained from standard commercial sources, including Shanghai Aladdin Bio-Chem Technology Co., Ltd., Shanghai Macklin Biochemical Co., Ltd., Sigma-Aldrich, Alfa Aesar (China) Chemical Co., Ltd., Tokyo Chemical Industry (Shanghai) Co., Ltd., Energy Chemical Co., Ltd., Shanghai Titan Scientific Co., Ltd., Kelong Chemical Co., Ltd., J&K Scientific and the like.

THF: tetrahydrofuran; DMF: N,N-dimethylformamide; DIPEA: N,N-diisopropylethylamine; Boc: tert-butoxycarbonyl.

### Example 1: Preparation of Compound 1-A and Compound 1-B

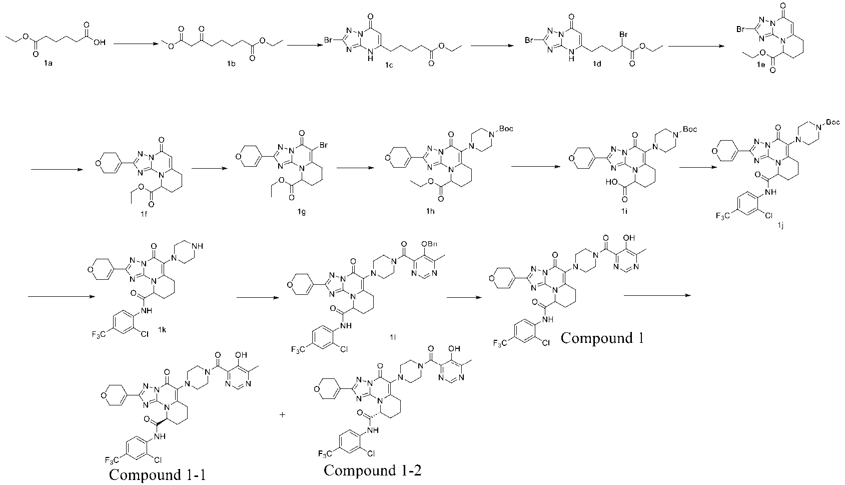

### Step 1: Preparation of 1b

Carbonyldiimidazole (15.36 g, 94.73 mmol) was added to **1a** (15.00 g, 86.13 mmol) in anhydrous tetrahydrofuran (150 mL), and the mixture was reacted at room temperature for 1 h. Then, monomethyl malonate potassium salt (16.14 g, 103.36 mmol) and anhydrous magnesium chloride (12.30 g, 129.19 mmol) were added, and the mixture was reacted at room temperature for 16 h. At 0°C, the pH value was adjusted to 4-5 with an aqueous hydrochloric acid solution (1.0 M), water (200 mL) was added, and the mixture was extracted with ethyl acetate (100 mL x 2). The organic phases were combined, then washed with saturated brine (200 mL), dried over anhydrous sodium sulfate, and filtered. The filtrate was concentrated under reduced pressure to afford the crude product, which was purified by column chromatography (petroleum ether : ethyl acetate (v : v) = 84 : 16) to give **1b** (14.85 g, yield: 75%).
LCMS m/z = 231.3[M+H]⁺

### Step 2: Preparation of 1c

Phosphoric acid (4.16 g, 42.43 mmol) was added to **1b** (9.77 g, 42.43 mmol) and 5-bromo-1H-1,2,4-triazol-3-amine (6.92 g, 42.43 mmol) in absolute ethanol (20 mL), and the mixture was reacted at 90°C for 48 h. The residue was afforded by concentration under reduced pressure. A saturated aqueous sodium bicarbonate solution was added to the residue to adjust the pH to 4-5, water (100 mL) was added, and the mixture was extracted with dichloromethane (50 mL x 2). The organic phases were combined, then washed with saturated brine (100 mL), dried over anhydrous sodium sulfate, and filtered. The filtrate was concentrated under reduced pressure to afford the crude product, which was purified by column chromatography (dichloromethane : tetrahydrofuran (v : v) = 87 : 13) to give **1c** (8.24 g, yield: 57%).
LCMS m/z = 343.1[M+H]⁺

### Step 3: Preparation of 1d

At -78°C under nitrogen protection, lithium bis(trimethylsilyl)amide (46.6 mL, 1 M in THF) was slowly added dropwise to **1c** (4.00 g, 11.66 mmol) in anhydrous tetrahydrofuran (120 mL), and the mixture was reacted at -78°C for 1 h. Trimethylchlorosilane (5.19 g, 47.78 mmol) was added at-78°C, and the mixture was reacted at -78°C for 30 min. N-Bromosuccinimide (2.08 g, 11.66 mmol) in tetrahydrofuran (20 mL) was slowly added dropwise at -78°C, and the mixture was reacted at -78°C for 16 h. At 0°C, the pH was adjusted to 4-5 with hydrochloric acid (1 N), saturated brine (150 mL) was added, and the mixture was extracted with ethyl acetate (80 mL x 2). The organic phases were combined, then washed with saturated brine (100 mL), dried over anhydrous sodium sulfate, and filtered. The filtrate was concentrated under reduced pressure to afford 1d (5.18 g, crude).
LCMS m/z = 423.3[M+H]⁺

### Step 4: Preparation of 1e

N,N-Diisopropylethylenediamine (4.52 g, 34.98 mmol) was added to 1d (5.18 g, crude) in N,N-dimethylformamide (50 mL), and the mixture was reacted at room temperature for 16 h. At 0°C, water (100 mL) was added, and the mixture was extracted with ethyl acetate (30 mL x 2). The organic phases were combined, then washed with a saturated aqueous sodium chloride solution (100 mL), dried over anhydrous sodium sulfate, and then filtered. The filtrate was concentrated under reduced pressure to afford the crude product, which was purified by column chromatography (petroleum ether : ethyl acetate (v : v) = 60 : 40) to give 1e (773 mg, two-step yield: 19%).
LCMS m/z =341.2[M+H]⁺

### Step 5: Preparation of 1f

[1,1'-Bis(diphenylphosphino)ferrocene]palladium(II)dichloride dichloromethane complex (221 mg, 0.27 mmol) was added to **1e** (1.85 g, 5.42 mmol), 3,6-dihydro-2H-pyran-4-boronic acid pinacol ester (1.48 g, 7.05 mmol), and potassium phosphate (3.45 g, 16.26 mmol) in 1,4-dioxane (20 mL) and water (4 mL), and the mixture was reacted at 80°C for 4 h under nitrogen protection. The mixture was cooled to room temperature, water (30 mL) was added, and the mixture was extracted with ethyl acetate (30 mL x 2). The organic phases were combined, then washed with saturated brine, dried over anhydrous sodium sulfate, and filtered. The filtrate was concentrated under reduced pressure to afford the crude product, which was purified by column chromatography (petroleum ether : ethyl acetate (v : v) = 30 : 70) to give **1f** (1.59 g, yield: 85%).
LCMS m/z = 345.3[M+H]⁺

### Step 6: Preparation of 1g

N-Bromosuccinimide (1.07 g, 6.01 mmol) was added to 1f (1.73 g, 5.01 mmol) in N,N-dimethylformamide (15 mL), and the mixture was reacted at room temperature for 2 h. Water (40 mL) was added, and the mixture was extracted with ethyl acetate (20 mL x 2). The organic phases were combined, then washed with saturated brine, dried over anhydrous sodium sulfate, and filtered. The filtrate was concentrated under reduced pressure to afford the crude product, which was purified by column chromatography (dichloromethane: (dichloromethane/methanol = 10/1) (v : v) = 93 : 7) to give **1g** (1.82 g, yield: 86%).
LCMS m/z = 423.0[M+H]⁺

### Step 7: Preparation of 1h

N-Boc-Piperazine (3.57 g, 19.15 mmol) was added to **1g** (1.62 g, 3.83 mmol) in dimethyl sulfoxide (20 mL), and the mixture was reacted at 120°C for 4 h. The mixture was cooled to room temperature, water (50 mL) was added, and the mixture was extracted with ethyl acetate (30 mL x 2). The organic phases were combined, then washed with saturated brine, dried over anhydrous sodium sulfate, and filtered. The filtrate was concentrated under reduced pressure to afford the crude product, which was purified by column chromatography (dichloromethane : (dichloromethane/methanol = 10/1) (v : v) = 80 : 20) to give **1h** (1.60 g, yield: 79%).

### LCMS m/z = 473.3[M-55]⁺

### Step 8: Preparation of 1i

Lithium hydroxide monohydrate (159 mg, 3.80 mmol) was added to **1h** (500 mmol, 0.95 mmol) in tetrahydrofuran (4 mL), water (1 mL), and methanol (2 mL), and the mixture was reacted at room temperature for 1 h. At 0°C, the pH was adjusted to 5-6 with hydrochloric acid (1 N), saturated brine (20 mL) was added, and the mixture was extracted with ethyl acetate (20 mL x 2). The organic phases were combined, then washed with saturated brine (30 mL), dried over anhydrous sodium sulfate, and filtered. The filtrate was concentrated under reduced pressure to afford **1i** (480 mg, crude).
LCMS m/z = 445.2[M-55]⁺

### Step 9: Preparation of 1j

At 0°C, oxalyl chloride (0.06 mL, 0.71 mmol) was slowly added to **1i** (240 mg, 0.48 mmol) in anhydrous dichloromethane (5 mL), N,N-dimethylformamide (0.02 mL) was added, and the mixture was reacted at room temperature for 1 h under nitrogen protection. Triethylamine (143 mg, 1.41 mmol) and 2-chloro-4-(trifluoromethyl)aniline (119 mg, 0.61 mmol) in dichloromethane (2 mL) was added, and the mixture was reacted at room temperature for 1 h. Silica gel was added, and the mixture was concentrated under reduced pressure to afford the residue, which was purified by column chromatography (dichloromethane : (dichloromethane/methanol = 10/1) (v : v) = 84 : 16) to give **1j** (197 mg, yield: 61%).
LCMS m/z = 622.2[M-55]⁺

### Step 10: Preparation of 1k

A solution of hydrogen chloride in 1,4-dioxane (4 N, 2.3 mL) was added dropwise to **1j** (209 mg, 0.31 mmol) in dichloromethane (3 mL), and the mixture was reacted at room temperature for 1 h. The hydrochloride salt of **1k** (190 mg, crude) was afforded after concentration under reduced pressure.
LCMS m/z = 578.2[M+H]⁺

### Step 11: Preparation of 1l

N,N-Diisopropylethylamine (80 mg, 0.62 mmol) was added to the hydrochloride salt of **1k** (190 mg, crude), 5-(benzyloxy)-6-methylpyrimidine-4-carboxylic acid (83 mg, 0.34 mmol), and 2-(7-azabenzotriazol-1-yl)-N,N,N',N'-tetramethyluronium hexafluorophosphate (141 mg, 0.37 mmol) in N,N-dimethylformamide (5 mL), and the mixture was reacted at room temperature for 1 h. Water (20 mL) was added, and the mixture was extracted with ethyl acetate (20 mL x 2). The organic phases were combined, then washed with saturated brine, dried over anhydrous sodium sulfate, and filtered. The filtrate was concentrated under reduced pressure to afford the crude product, which was purified by column chromatography (dichloromethane : (dichloromethane/methanol = 10/1) (v : v) = 70 : 30) to give **1l** (244 mg, two-step yield: 98%).
LCMS m/z = 804.2[M+H]⁺

### Step 12: Preparation of Compound 1

Triflic acid (0.08 mL) was added to **1l** (244 mg, 0.30 mmol) in trifluoroacetic acid (5 mL), and the mixture was reacted at 50°C for 1 h. The residue was afforded by concentration under reduced pressure. The residue was dissolved in 10 mL of dichloromethane, the pH was adjusted to 8-9 with a saturated aqueous sodium bicarbonate solution at 0°C, and the mixture was extracted with dichloromethane 20 mL x 2). The organic phases were combined, then washed with saturated brine, dried over anhydrous sodium sulfate, and filtered. The filtrate was concentrated under reduced pressure to afford the crude product, which was purified by Pre-HPLC (the instrument and preparative column were as follows: Waters 2767 preparative liquid chromatograph was employed, and the preparative column model was XBridge@Prep C18 with an inner diameter x length of 19 mm x 250 mm). Preparation method: The crude product was dissolved in DMF and filtered with a 0.45 µm filter membrane to prepare into a sample solution. Mobile phase system: Acetonitrile/water (with 0.05% NH₃.H₂O). Gradient elution method: An acetonitrile gradient from 30% to 80% (flow rate: 12 mL/min; elution time: 15 min). Compound 1 (150 mg, yield: 70%) was afforded after lyophilization.
LCMS m/z = 714.1[M+H]⁺

### Step 13: Preparation of Compound 1-A and Compound 1-B

**Compound 1** was purified by SFC on AD column (the instrument and preparative column were as follows: Waters 150 Prep-SFC was employed, and the preparative column model was Chiral IK column). Preparation method: **Compound 1** was dissolved in acetonitrile and filtered with a 0.45 µm filter membrane to prepare into a sample solution. Mobile phase system: A for CO₂ and B for 0.1% NH3•H2O in ethanol and acetonitrile. Gradient elution method: Isocratic elution with 70% mobile phase B (flow rate: 100 mL/min; elution time: 4.1 min). Compound **1-A** and Compound **1-B** were afforded after lyophilization.

Analytical method 1 (instrument and preparative column: high-performance liquid chromatograph-normal-phase chromatograph, and analytical column model: Chiral IK column; mobile phase system: A for CO2; B for 0.05% DEA in ethanol and acetonitrile; column temperature: 35°C; flow rate: 3.0 mL/min). Retention time T = 0.606 min for Compound **1-A.** Retention time T = 1.397 min for Compound **1-B.**

Analytical method 2 (instrument: SHIMADZU LC-20AT; preparative column model: CHIRALCEL OD-HOD-H, 4.6 x 250 mm, 5 µm; mobile phase system: n-hexane : ethanol = 60 : 40; wavelength: 210 nm; column temperature: 35°C; flow rate: 1.0 ml/min). Retention time T = 8.803 min for Compound **1-A,** and retention time T = 16.026 min for Compound **1-B** (one of Compound 1-A and Compound 1-B is Compound 1-1, and the other is Compound 1-2).

According to Example 30, **Compound 1-B** was confirmed to be **Compound 1-2.**

### Compound 1-A:

LCMS m/z = 714.1[M+H]⁺;
¹H NMR (400 MHz, CDCl₃) δ 11.80 (s, 1H), 8.59 (s, 1H), 8.51-8.37 (m, 2H), 7.66 (s, 1H), 7.54 (d, 1H), 6.95-6.80 (m, 1H), 5.76-5.53 (m, 1H), 5.52-5.43(m, 1H), 4.89-4.63 (m, 1H), 4.43-4.21 (m, 2H), 3.97-3.68 (m, 4H), 3.59-3.31 (m, 2H),3.18-2.97 (m, 2H), 2.92-2.64 (m, 4H),2.62-2.50 (m, 4H),2.40-2.11 (m, 2H) ,2.05-1.85 (m, 1H).

### Compound 1-B:

LCMS m/z = 714.1[M+H]⁺;
¹H NMR (400 MHz, CDCl₃) δ 11.90 (s, 1H), 8.60 (s, 1H), 8.53-8.35 (m, 2H), 7.66 (s, 1H), 7.54 (d, 1H), 6.95-6.80 (m, 1H), 5.72-5.54 (m, 1H), 5.53-5.43(m, 1H), 4.94-4.62 (m, 1H), 4.44-4.19 (m, 2H), 4.00-3.66 (m, 4H), 3.60-3.28 (m, 2H),3.21-2.97 (m, 2H), 2.94-2.45 (m, 8H), 2.37-2.12 (m, 2H) ,2.03-1.89 (m, 1H).

### Example 2: Preparation of Compound 2-A and Compound 2-B

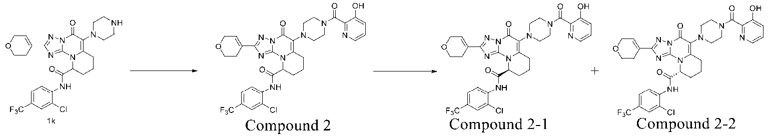

### Step 1: Preparation of Compound 2

In an ice bath, N,N-diisopropylethylamine (78 mg, 0.60 mmol), 3-hydroxy-2-pyridinecarboxylic acid (28 mg, 0.20 mmol), 1-(3-dimethylaminopropyl)-3-ethylcarbodiimide hydrochloride (38 mg, 0.20 mmol), and 1-hydroxybenzotriazole (27 mg, 0.20 mmol) in N,N-dimethylformamide (1 mL) was added to the hydrochloride salt of **1k** (63 mg, 0.10 mmol) in N,N-dimethylformamide (2 mL), and the mixture was reacted at room temperature for 2 h. 3-Hydroxy-2-pyridinecarboxylic acid (12 mg, 0.86 mmol), 2-(7-azabenzotriazol-1-yl)-N,N,N',N'-tetramethyluronium hexafluorophosphate (37 mg, 0.97 mmol), and N,N-diisopropylethylamine (21 mg, 1.63 mmol) in N,N-dimethylformamide (1 mL) was additionally added, and the mixture was reacted for 1 h. Water (20 mL) was added, and the mixture was extracted with ethyl acetate (20 mL x 2). The organic phases were combined, then washed with saturated brine, dried over anhydrous sodium sulfate, and filtered. The filtrate was concentrated under reduced pressure to afford the crude product. The crude product was dissolved in ethanol (10 mL), hydrazine hydrate (1.2 mL, 80% aq) was added, and the mixture was reacted at room temperature for 16 h. The mixture was concentrated under reduced pressure, water (20 mL) was added, the pH was adjusted to 5-6 with hydrochloric acid (1 M), and the mixture was extracted with ethyl acetate (20 mL x 2). The organic phases were combined, then washed with saturated brine, dried over anhydrous sodium sulfate, and filtered. The filtrate was concentrated under reduced pressure to afford the crude product. The crude product was purified by column chromatography (dichloromethane : (dichloromethane/methanol = 10/1) (v : v) = 70 : 30) to give **Compound 2** (58 mg, yield: 79%).
LCMS m/z = 699.2[M+H]⁺

### Step 2: Preparation of Compound 2-A and Compound 2-B

**Compound 2** was purified by SFC on OX column (the instrument and preparative column were as follows: Waters 150 Prep-SFC was employed, and the preparative column model was Chiral OX column). Preparation method: **Compound 2** was dissolved in acetonitrile and methanol, and filtered with a 0.45 µm filter membrane to prepare into a sample solution. Mobile phase system: A for CO₂ and B for 0.1% NH3•H2O in methanol and acetonitrile. Gradient elution method: Isocratic elution with 45% mobile phase B (flow rate: 110 mL/min; elution time: 7.4 min). Compound **2-A** and Compound **2-B** were afforded after lyophilization.

Analytical method (instrument and preparative column: high-performance liquid chromatograph-normal-phase chromatograph, and analytical column model: Chiral OX column; mobile phase system: A for CO2; B for 0.05% DEA in methanol and acetonitrile; column temperature: 35°C; flow rate: 3.0 mL/min). Retention time T = 0.860 min for Compound **2-A.** Retention time T = 1.343 min for Compound **2-B** (one of Compound 2-A and Compound 2-B is Compound 2-1, and the other is Compound 2-2).

### Compound 2-A: LCMS m/z = 699.2[M+H]⁺;

¹H NMR (400 MHz, CDCl₃) δ 11.79 (s, 1H), 8.51-8.36 (m, 2H), 8.14 (s, 1H), 7.66 (s, 1H), 7.53 (d, 1H), 7.44-7.32 (m, 1H), 6.94-6.79 (m, 1H), 5.61-5.43 (m, 1H), 5.38-4.50 (m, 2H), 4.49-4.19 (m, 2H), 3.97-3.63 (m, 4H), 3.61-3.30 (m, 2H),3.27-2.97 (m, 2H), 2.92-2.62 (m, 4H),2.61-2.47 (m, 1H),2.39-2.14 (m, 2H) ,2.01-1.90 (m, 1H).

### Compound 2-B: LCMS m/z = 699.2[M+H]⁺;

¹H NMR (400 MHz, CDCl₃) δ 11.47 (s, 1H), 8.56-8.32 (m, 2H), 8.20 (s, 1H), 7.80 (s, 1H), 7.66 (s, 1H), 7.59-7.43 (m, 2H), 6.95-6.84 (m, 1H), 5.61-5.43 (m, 1H), 4.92-4.51 (m, 2H), 4.42-4.22 (m, 2H), 3.97-3.81 (m, 2H), 3.80-3.30 (m, 4H),3.29-2.96 (m, 2H), 2.92-2.62 (m, 4H),2.61-2.47 (m, 1H),2.42-2.14 (m, 2H) ,2.01-1.90 (m, 1H).

### Example 3: Preparation of Compound 3-A and Compound 3-B

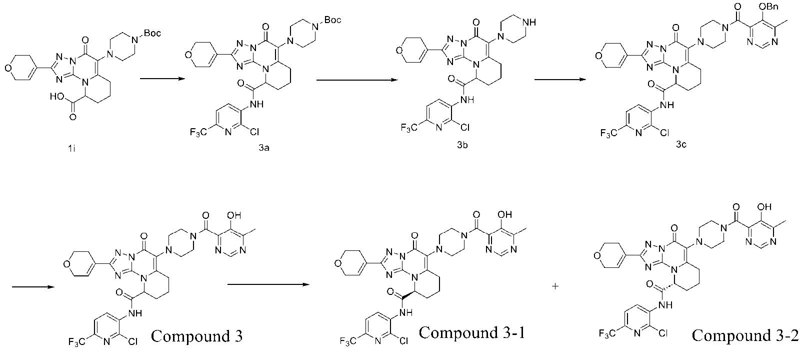

**Compound 3** was afforded with **1i** and 2-chloro-6-(trifluoromethyl)pyridin-3-amine as the substrates by referring to the synthetic method in Example 1. **Compound 3** was purified by SFC on OX column (the instrument and preparative column were as follows: Waters 150 Prep-SFC was employed, and the preparative column model was Chiral OX column). Preparation method: **Compound 3** was dissolved in acetonitrile and methanol, and filtered with a 0.45 µm filter membrane to prepare into a sample solution. Mobile phase system: A for CO₂ and B for 0.1% NH3•H2O in methanol and acetonitrile. Gradient elution method: Isocratic elution with 45% mobile phase B (flow rate: 110 mL/min; elution time: 9.0 min). Compound **3-A** and Compound **3-B** were afforded after lyophilization.

Analytical method (instrument and preparative column: high-performance liquid chromatograph-normal-phase chromatograph, and analytical column model: Chiral OX column; mobile phase system: A for CO2; B for 0.05% DEA in methanol and acetonitrile; column temperature: 35°C; flow rate: 3.0 mL/min). Retention time T = 1.009 min for Compound **3-A.** Retention time T = 1.537 min for Compound **3-B** (one of Compound 3-A and Compound 3-B is Compound 3-1, and the other is Compound 3-2).

### Compound 3-A: LCMS m/z = 715.2[M+H]⁺;

¹H NMR (400 MHz, CDCl₃) δ 11.90 (s, 1H), 8.83-8.76 (m, 1H),8.68-8.56 (m, 2H), 7.64 (d, 1H), 6.91-6.81 (m, 1H), 5.72-5.56 (m, 1H), 5.55-5.47 (m, 1H),4.91-4.63 (m, 1H), 4.42-4.22 (m, 2H), 3.97-3.63 (m, 4H), 3.61-3.31 (m, 2H),3.20-2.97 (m, 2H), 2.96-2.62 (m, 4H),2.61-2.47 (m, 4H),2.40-2.11 (m, 2H) ,2.04-1.91 (m, 1H).

### Compound 3-B: LCMS m/z = 715.1[M+H]⁺;

¹H NMR (400 MHz, CDCl₃) δ 11.88 (s, 1H), 8.83-8.76 (m, 1H),8.68-8.56 (m, 2H), 7.64 (d, 1H), 6.91-6.81 (m, 1H), 5.72-5.56 (m, 1H), 5.55-5.47 (m, 1H),4.91-4.63 (m, 1H), 4.42-4.22 (m, 2H), 3.97-3.63 (m, 4H), 3.61-3.31 (m, 2H),3.20-2.97 (m, 2H), 2.96-2.62 (m, 4H),2.61-2.47 (m, 4H),2.40-2.11 (m, 2H) ,2.04-1.91 (m, 1H).

### Example 4: Preparation of Compound 4-A and Compound 4-B

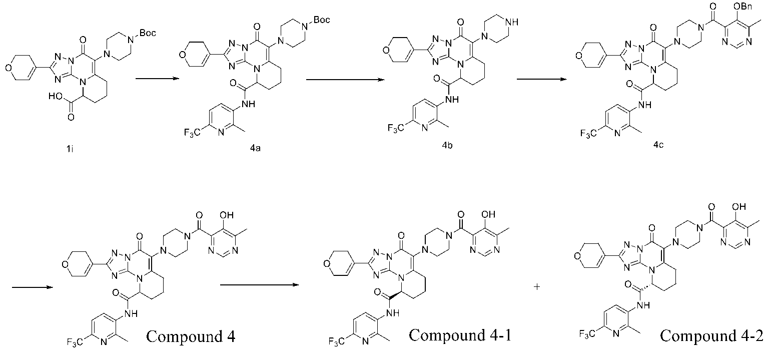

**Compound** 4 was afforded with **1i** and 2-methyl-6-(trifluoromethyl)pyridin-3-amine as the substrates by referring to the synthetic method in Example 1. **Compound** 4 was purified by SFC on IK column (the instrument and preparative column were as follows: Waters 150 Prep-SFC was employed, and the preparative column model was Chiral IK column). Preparation method: **Compound 4** was dissolved in acetonitrile and methanol, and filtered with a 0.45 µm filter membrane to prepare into a sample solution. Mobile phase system: A for CO₂ and B for 0.1% NH3•H2O in ethanol and acetonitrile. Gradient elution method: Isocratic elution with 60% mobile phase B (flow rate: 90 mL/min; elution time: 3.2 min). Compound **4-A** and Compound **4-B** were afforded after lyophilization.

Analytical method (instrument and preparative column: high-performance liquid chromatograph-normal-phase chromatograph, and analytical column model: Chiral IK column; mobile phase system: A for CO2; B for 0.05% DEA in methanol and acetonitrile; column temperature: 35°C; flow rate: 3.0 mL/min). Retention time T = 0.709 min for Compound **4-A.** Retention time T = 1.899 min for Compound **4-B** (one of Compound 4-A and Compound 4-B is Compound 4-1, and the other is Compound 4-2).

### Compound 4-A: LCMS m/z = 695.4[M+H]⁺;

¹H NMR (400 MHz, DMSO-*d*₆) δ 10.45-10.29 (m, 1H), 10.28-10.10 (m, 1H), 8.57 (s, 1H),8.10-7.98 (m, 1H), 7.78-7.66 (m, 1H), 6.86-6.74 (m, 1H), 5.62-5.43 (m, 1H), 4.60-4.43 (m, 1H),4.34-4.16 (m, 2H), 3.89-3.72 (m, 2H), 3.59-3.46 (m, 2H), 3.45-3.33 (m, 2H), 3.30-3.20 (m, 3H), 3.07-2.76 (m, 3H), 2.70-2.58 (m, 1H),2.54 (s, 3H),2.44 (s, 3H) ,2.41-2.27 (m, 2H) ,1.97-1.84 (m, 1H), 1.82-1.66 (m, 1H).

### Compound 4-B: LCMS m/z = 695.4[M+H]⁺;

¹H NMR (400 MHz, DMSO-*d*₆) δ 10.45-10.29 (m, 1H), 10.28-10.10 (m, 1H), 8.58 (s, 1H),8.10-7.98 (m, 1H), 7.78-7.66 (m, 1H), 6.86-6.74 (m, 1H), 5.62-5.43 (m, 1H), 4.60-4.43 (m, 1H),4.34-4.16 (m, 2H), 3.89-3.72 (m, 2H), 3.59-3.46 (m, 2H), 3.45-3.33 (m, 2H), 3.30-3.20 (m, 3H),3.07-2.76 (m, 3H), 2.70-2.58 (m, 1H),2.54 (s, 3H),2.45 (s, 3H) ,2.41-2.27 (m, 2H) ,1.97-1.84 (m, 1H), 1.82-1.66 (m, 1H).

### Example 5: Preparation of Compound 5-A and Compound 5-B

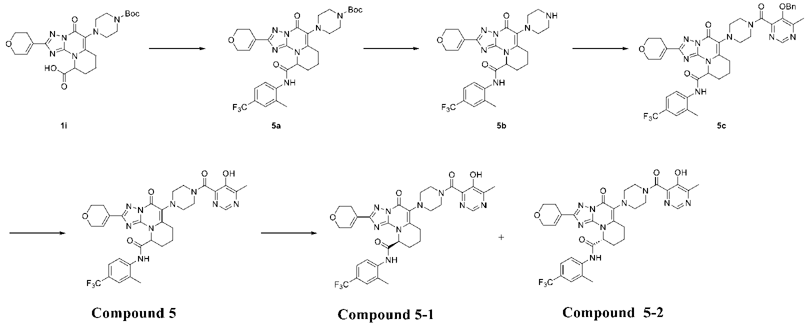

**Compound 5** was afforded with **1i** and **2-methyl-4-(trifluoromethyl)aniline** as the substrates by referring to the synthetic method in Example 1. **Compound 5** was purified by SFC on AD column (the instrument and preparative column were as follows: Waters 150 Prep-SFC was employed, and the preparative column model was Chiral IK column). Preparation method: **Compound** 5 was dissolved in acetonitrile and ethanol, and filtered with a 0.45 µm filter membrane to prepare into a sample solution. Mobile phase system: A for CO₂ and B for 0.1% NH3•H2O in ethanol and acetonitrile. Gradient elution method: Isocratic elution with 55% mobile phase B (flow rate: 100 mL/min; elution time: 5.5 min). Compound **5-A** and Compound **5-B** were afforded after lyophilization.

Analytical method (instrument and preparative column: high-performance liquid chromatograph-normal-phase chromatograph, and analytical column model: Chiral IK column; mobile phase system: A for CO2; B for 0.05% DEA in ethanol and acetonitrile; column temperature: 35°C; flow rate: 3.0 mL/min). Retention time T = 0.505 min for Compound **5-A.** Retention time T = 0.894 min for Compound **5-B** (one of Compound 5-A and Compound 5-B is Compound 5-1, and the other is Compound 5-2).

### Compound 5-A: LCMS m/z = 694.2[M+H]⁺;

¹H NMR (400 MHz, DMSO-*d*₆) δ 10.30-10.15 (m, 1H), 10.15-10.09 (m, 1H), 8.57 (s, 1H),7.65-7.60 (m, 1H), 7.59-7.50 (m, 2H), 6.84-6.78 (m, 1H), 5.58-5.43 (m, 1H), 4.57-4.44 (m, 1H),4.34-4.21 (m, 2H), 3.88-3.75 (m, 2H), 3.60-3.46 (m, 2H), 3.45-3.33 (m, 3H), 3.30-3.20 (m, 2H),3.07-2.76 (m, 3H), 2.70-2.60 (m, 1H), 2.45 (s, 3H) ,2.41-2.29 (m, 5H) ,1.96-1.85 (m, 1H), 1.82-1.67 (m, 1H).

### Compound 5-B: LCMS m/z = 694.2[M+H]⁺;

¹H NMR (400 MHz, DMSO-*d*₆) δ 10.35-9.98 (m, 2H), 8.58 (s, 1H),7.65-7.60 (m, 1H), 7.59-7.50 (m, 2H), 6.84-6.78 (m, 1H), 5.58-5.43 (m, 1H), 4.57-4.44 (m, 1H),4.34-4.21 (m, 2H), 3.88-3.74 (m, 2H), 3.60-3.46 (m, 2H), 3.45-3.33 (m, 3H), 3.30-3.20 (m, 2H),3.07-2.76 (m, 3H), 2.70-2.60 (m, 1H), 2.45 (s, 3H) ,2.41-2.29 (m, 5H) ,1.96-1.85 (m, 1H) , 1.82-1.67 (m, 1H).

### Example 6: Preparation of Compound 6-A and Compound 6-B

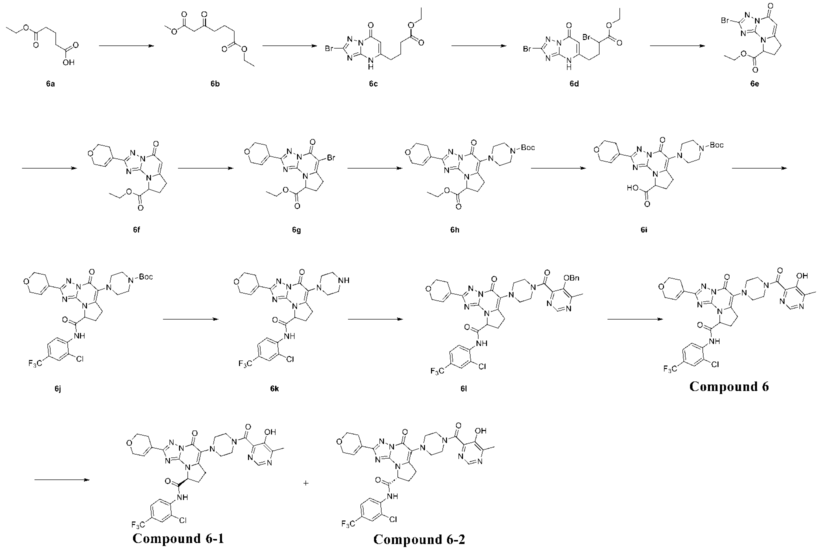

**Compound 6** was afforded with **6a** as the substrate by referring to the synthetic method in Example 1. **Compound 6** was purified by SFC on AD column (the instrument and preparative column were as follows: Waters 150 Prep-SFC was employed, and the preparative column model was Chiral IK column). Preparation method: **Compound 6** was dissolved in acetonitrile and ethanol, and filtered with a 0.45 µm filter membrane to prepare into a sample solution. Mobile phase system: A for CO₂ and B for 0.1% NH3•H2O in ethanol and acetonitrile. Gradient elution method: Isocratic elution with 55% mobile phase B (flow rate: 100 mL/min; elution time: 5.5 min). Compound **6-A** and Compound **6-B** were afforded after lyophilization.

Analytical method (instrument and preparative column: high-performance liquid chromatograph-normal-phase chromatograph, and analytical column model: Chiral IK column; mobile phase system: A for CO2; B for 0.05% DEA in ethanol and acetonitrile; column temperature: 35°C; flow rate: 3.0 mL/min). Retention time T = 0.578 min for Compound **6-A.** Retention time T = 1.129 min for Compound **6-B** (one of Compound 6-A and Compound 6-B is Compound 6-1, and the other is Compound 6-2).

### Compound 6-A:

LCMS m/z = 700.2[M+H]⁺;
¹H NMR (400 MHz, CF3COOD) δ 9.11 (s, 1H), 8.14 (d, 1H), 7.93 (s, 1H), 7.76 (d, 1H), 7.22 (s, 1H), 6.09 (dd, 1H), 5.26-5.00 (m, 2H), 4.75-4.70 (m, 2H), 4.69-4.59 (m, 2H), 4.58-4.41 (m, 4H), 4.39-4.26(m, 2H), 4.17-3.97 (m, 2H), 3.35-3.19 (m, 1H), 3.18-3.05 (m, 4H), 2.96-2.86 (m, 2H).

### Compound 6-B:

LCMS m/z = 700.1[M+H]⁺;
¹H NMR (400 MHz, CF3COOD) δ 9.08 (s, 1H), 8.11 (d, 1H), 7.90 (s, 1H), 7.74 (d, 1H), 7.20 (s, 1H), 6.07 (dd, 1H), 5.28-4.95 (m, 2H), 4.73- 4.67 (m, 2H), 4.66-4.57 (m, 2H), 4.56-4.39 (m, 4H), 4.36-4.23 (m, 2H), 4.14-3.95 (m, 2H), 3.31-3.17 (m, 1H), 3.16-3.01 (m, 4H), 2.95-2.82 (m, 2H).

### Example 7: Preparation of Compound 7-A and Compound 7-B

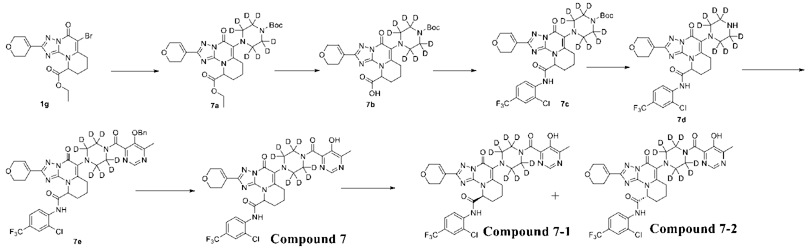

**Compound** 7 was afforded with **1g** and ***tert-butyl* 2,2,3,3,5,5,6,6-d8-piperazine-1-carboxylate** as the substrates by referring to the synthetic method in Example 1.
LCMS m/z = 722.2 [M+1]⁺

### Preparation of Compound 7-A and Compound 7-B

**Compound 7 was** purified by SFC on IK column (the instrument and preparative column were as follows: Waters 150 Prep-SFC was employed, and the preparative column model was Chiral IK column). Preparation method: **Compound 7** was dissolved in acetonitrile and dichloromethane, and filtered with a 0.45 µm filter membrane to prepare into a sample solution. Mobile phase system: A for CO₂ and B for 0.1% NH3•H2O in ethanol and acetonitrile. Gradient elution method: Isocratic elution with 60% mobile phase B (flow rate: 100 mL/min; elution time: 6.2 min). Compound **7-A** and Compound **7-B** were afforded after lyophilization.

Analytical method (instrument and preparative column: high-performance liquid chromatograph-normal-phase chromatograph, and analytical column model: Chiral IK column; mobile phase system: A for CO2; B for 0.05% DEA in ethanol and acetonitrile; column temperature: 35°C; flow rate: 3.0 mL/min). Retention time T = 0.600 min for Compound **7-A.** Retention time T = 1.351 min for Compound **7-B** (one of Compound 7-A and Compound 7-B is Compound 7-1, and the other is Compound 7-2).

### Compound 7-A:

LCMS m/z = 722.2[M+H]+;
¹H NMR (400 MHz, CDCl₃) δ 11.89 (s, 1H), 8.60 (s, 1H), 8.48 (s, 1H), 8.41 (d, 1H), 7.66 (d, 1H), 7.53 (d, 1H), 6.89-6.82 (m, 1H), 5.58-5.46 (m, 1H), 4.42-4.20 (m, 2H), 4.00-3.80 (m, 2H), 3.43-3.29 (m, 1H), 3.16-3.00 (m, 1H), 2.72-2.63 (m, 2H), 2.62-2.49 (m, 4H), 2.36-2.24 (m, 1H), 2.24-2.10(m, 1H), 1.99-1.91 (m, 1H).

### Compound 7-B:

LCMS m/z = 722.1 [M+H]+;
¹H NMR (400 MHz, CDCl₃) δ 11.90 (s, 1H), 8.60 (s, 1H), 8.48 (s, 1H), 8.41 (d, 1H), 7.66 (d, 1H), 7.53 (d, 1H), 6.90-6.82 (m, 1H), 5.56-5.46 (m, 1H), 4.41-4.22(m, 2H), 3.96-3.79 (m, 2H), 3.46-3.31 (m, 1H), 3.18-3.02 (m, 1H), 2.73-2.62 (m, 2H), 2.62-2.50 (m, 4H), 2.36-2.24 (m, 1H), 2.24-2.10(m, 1H), 1.99-1.89(m, 1H).

### Example 8: Preparation of Compound 8-A and Compound 8-B

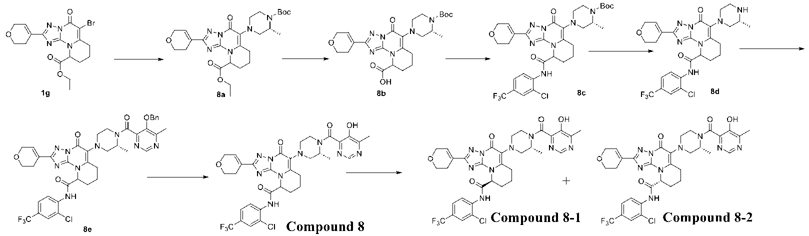

**Compound 8** was afforded with **1g** and **(R)-1-N-Boc-2-methylpiperazine as** the substrates by referring to the synthetic method **in Example 1.**
LCMS m/z = 728.2 [M+1]⁺

### Preparation of Compound 8-A and Compound 8-B

**Compound 8** was purified by SFC on Chiral IK column (the instrument and preparative column were as follows: Waters 150 Prep-SFC was employed, and the preparative column model was Chiral IK column). Preparation method: **Compound 8** was dissolved in acetonitrile and dichloromethane, and filtered with a 0.45 µm filter membrane to prepare into a sample solution. Mobile phase system: A for CO₂ and B for 0.1% NH3•H2O in methanol and acetonitrile. Gradient elution method: Isocratic elution with 60% mobile phase B (flow rate: 100 mL/min; elution time: 6.2 min). Compound **8-A** and Compound **8-B** were afforded after lyophilization.

Analytical method (instrument and preparative column: high-performance liquid chromatograph-normal-phase chromatograph, and analytical column model: Chiral IK column; mobile phase system: A for CO₂; B for 0.05% DEA in ethanol and acetonitrile; column temperature: 35°C; flow rate: 3.0 mL/min). Retention time T = 0.534 min for Compound **8-A.** Retention time T = 0.984 min for Compound **8-B** (one of Compound 8-A and Compound 8-B is Compound 8-1, and the other is Compound 8-2).

### Compound 8-A:

LCMS m/z = 728.1[M+H]+;
¹H NMR (400 MHz, CF3COOD) δ 9.62-9.42(m, 1H), 8.02-7.87 (m, 1H), 7.81 (s, 1H), 7.68-7.57(m, 1H), 7.24 -7.08 (m, 1H), 6.05-5.89 (m, 1H), 5.19-4.72 (m, 1H), 4.71-4.54(m, 2H), 4.32-4.07 (m, 4H), 4.06-3.87 (m, 1H), 3.84 -3.52 (m, 2H), 3.51-3.29 (m, 1H), 3.24-3.13(m, 1H), 3.12-2.88 (m, 4H), 2.85-2.58(m, 4H), 2.36-2.08 (m, 2H), 1.92-1.54 (m, 3H).

### Compound 8-B:

LCMS m/z = 728.2[M+H]+;
¹H NMR (400 MHz, CF3COOD) δ 9.12-8.97(m, 1H), 8.03-7.92 (m, 1H), 7.85 (s, 1H), 7.74-7.62(m, 1H), 7.26-7.10 (m, 1H), 6.03-5.92 (m, 1H), 5.35-5.03 (m, 1H), 4.94-4.57(m, 3H), 4.33-4.04(m, 5H), 3.88-3.74 (m, 1H), 3.50-3.20 (m, 2H), 3.17-2.91 (m, 4H), 2.89-2.60 (m, 4H), 2.35-2.18 (m, 2H), 1.95-1.59 (m, 3H).

### Example 9: Preparation of Compound 9

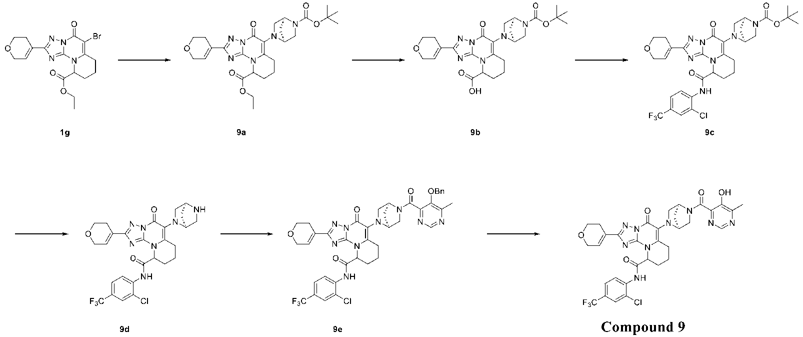

**Compound 9** was afforded with **1g** and ***tert-butyl* (1S,4S)-2,5-diazabicyclo[2.2.1]heptane-2-carboxylate** as the substrates by referring to the synthetic method in Example 1.
LCMS m/z = 726.1 [M+H]+;
¹H NMR (400 MHz, CF3COOD) δ 9.61-8.86(m, 1H), 8.08-7.94 (m, 1H), 7.92-7.85 (m, 1H), 7.76-7.65(m, 1H), 7.25-7.11 (m, 1H), 6.13-5.93 (m, 1H), 5.66-5.08 (m, 2H), 5.07-4.82(m, 1H), 4.81-4.35(m, 4H), 4.34-3.99 (m, 3H), 3.89-3.29(m, 2H), 3.28-2.95 (m, 4H), 2.92-2.56 (m, 5H), 2.48-2.23 (m, 2H).

### Example 10: Preparation of Compound 10

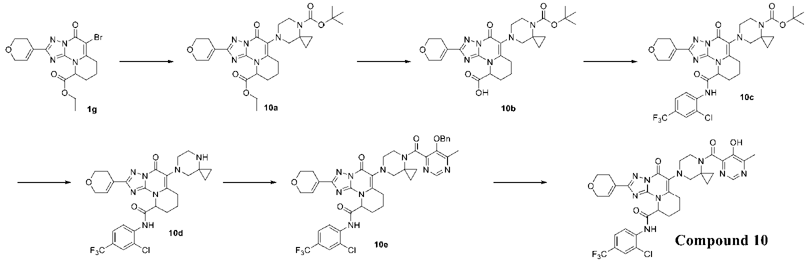

**Compound 10** was afforded with 1g and *tert-butyl 4,7-***diazaspiro[2.5]octane-4-carboxylate** as the substrates by referring to the synthetic method in Example 1.
LCMS m/z = 740.1[M+H]+;
¹H NMR (400 MHz, CF3COOD) δ 9.65-9.48(m, 1H), 7.98 (d, 1H), 7.90 (s, 1H), 7.72(d, 1H), 7.25 (s, 1H), 6.19-5.96 (m, 1H), 5.27-3.35 (m, 12H), 3.15-2.65(m, 7H), 2.55-2.24(m, 2H), 1.98-1.18 (m, 4H).

### Example 11: Preparation of Compound 11

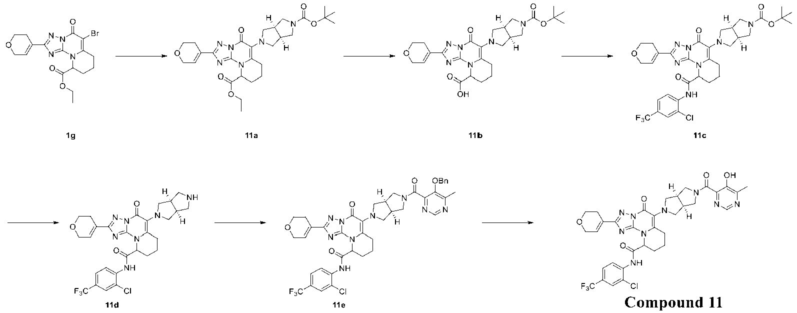

**Compound** 11 was afforded with 1g and *tert-Butyl cis-***hexahydropyrrolo[3,4-c]pyrrole-2-carboxylate as** the substrates by referring to the synthetic method in Example 1.
LCMS m/z = 740.1[M+H]+;
¹H NMR (400 MHz, CF3COOD) δ 9.66-9.04(m, 1H), 8.06-7.98 (m, 1H), 7.90 (s, 1H),7.76-7.68(m, 1H), 7.25-7.17 (m, 1H), 6.03-5.94 (m, 1H), 4.80-4.47 (m, 7H), 4.44-4.18(m, 5H), 4.03-3.77(m, 2H), 3.70-3.56 (m, 1H), 3.55-3.41(m, 1H), 3.04 (s, 3H), 2.92-2.65 (m, 4H), 2.42-2.27 (m, 2H).

### Example 12: Preparation of Compound 12

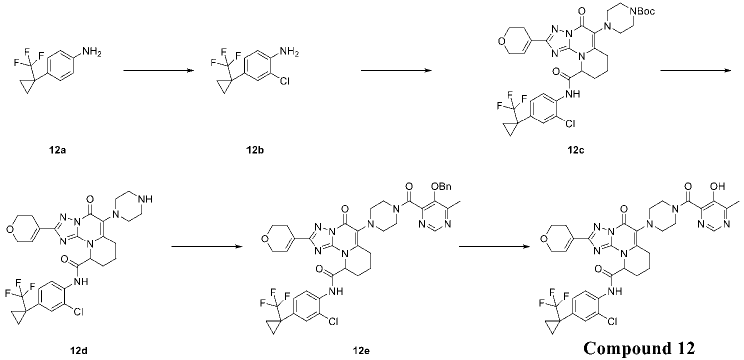

### Step 1: Preparation of 12b

**12a** (500 mg, 2.49 mmol) and N-chlorosuccinimide (403 mg, 3.02 mmol) were added to acetonitrile (10 mL), and the mixture was reacted at 90°C for 2 h. The mixture was cooled to room temperature, water (10 mL) was added, and the mixture was extracted with ethyl acetate (10 mL x 3). The organic phases were combined, then washed with saturated brine (10 mL), dried over anhydrous sodium sulfate, and filtered. The filtrate was subjected to column chromatography (petroleum ether : ethyl acetate (v : v) = 85 : 15) to give **12b** (570 mg, yield: 97%).
LCMS m/z = 236.0 [M+1]⁺

**Compound 12** was afforded with **1g** and **12b** as the substrates by referring to the synthetic method in Example 1.
LCMS m/z = 754.2 [M+1]⁺
¹H NMR (400 MHz, CF3COOD) δ 9.08(s, 1H), 7.82-7.69 (m, 2H), 7.59 (d, 1H),7.33-7.20 (m, 1H), 6.10-5.95 (m, 1H), 5.74-5.51 (m, 1H), 5.28-5.05(m, 1H), 5.01-4.62(m, 4H), 4.60-4.41 (m, 1H), 4.40-3.89(m, 5H), 3.81-3.65 (m, 1H), 3.62-3.43 (m, 1H), 3.06(s, 3H), 2.97-2.68 (m, 4H), 2.49-2.23 (m, 2H) , 1.62-1.47 (m, 2H), 1.20-1.10 (m, 2H).

### Example 13: Preparation of Compound 13

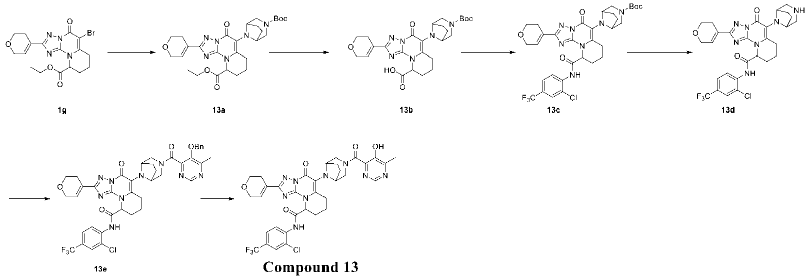

**Compound 13** was afforded with **1g** and ***tert-butyl* 3,8-diazabicyclo[3.2.1]octane-3-carboxylate** as the substrates by referring to the synthetic method in Example 1.
LCMS m/z = 740.2 [M+1]⁺

### Preparation of Compound 13-A and Compound 13-B

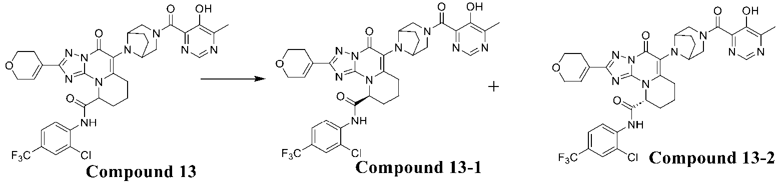

**Compound 13** was purified by SFC on AD column (the instrument and preparative column were as follows: Waters 150 Prep-SFC was employed, and the preparative column model was Chiral IK column). Preparation method: **Compound 13** was dissolved in acetonitrile and dichloromethane, and filtered with a 0.45 µm filter membrane to prepare into a sample solution. Mobile phase system: A for CO₂ and B for 0.1% NH3•H2O in methanol and acetonitrile. Gradient elution method: Isocratic elution with 70% mobile phase B (flow rate: 100 mL/min; elution time: 5.3 min). Compound **13-A** and Compound **13-B** were afforded after lyophilization.

Analytical method (instrument and preparative column: high-performance liquid chromatograph-normal-phase chromatograph, and analytical column model: Chiral IK column; mobile phase system: A for CO2; B for 0.05% DEA in methanol and acetonitrile; column temperature: 35°C; flow rate: 3.0 mL/min). Retention time T = 0.753 min for Compound **13-A.** Retention time T = 1.364 min for Compound **13-B** (one of Compound 13-A and Compound 13-B is Compound 13-1, and the other is Compound 13-2).

### Compound 13-A:

LCMS m/z = 740.2[M+H]⁺;
¹H NMR (400 MHz, CDCl₃) δ 11.61-11.50 (m, 1H), 8.61 (s, 1H), 8.52-8.35 (m, 2H), 7.69-7.64 (m, 1H), 7.57 -7.50 (m, 1H), 6.90-6.84(m, 1H), 5.54-5.45 (m, 1H), 5.11-5.01 (m, 1H), 4.58-4.49 (m, 1H), 4.39-4.22 (m, 2H), 3.96 -3.82 (m, 2H), 3.82-3.70 (m, 2H), 3.69-3.62 (m, 1H), 3.39-3.26 (m, 2H), 3.24-3.13(m, 1H), 2.73-2.63(m, 2H), 2.62 -2.50(m, 4H), 2.48-2.30 (m, 3H), 2.28-2.13(m, 1H), 2.00-1.89 (m, 1H), 1.88-1.76 (m, 2H).

### Compound 13-B:

LCMS m/z = 740.2[M+H]⁺;
¹H NMR (400 MHz, CDCl₃) δ 11.56-11.43 (m, 1H), 8.61 (s, 1H), 8.50-8.39 (m, 2H), 7.69-7.64 (m, 1H), 7.57 -7.50 (m, 1H), 6.90-6.84(m, 1H), 5.54-5.43 (m, 1H), 5.11 -5.01 (m, 1H), 4.58-4.49 (m, 1H), 4.40-4.23 (m, 2H), 3.97-3.81 (m, 2H), 3.81-3.71 (m, 2H), 3.70-3.60 (m, 1H), 3.39-3.26 (m, 2H), 3.26-3.12(m, 1H), 2.74-2.64(m, 2H), 2.61-2.48(m, 4H), 2.48-2.29 (m, 3H), 2.28-2.13(m, 1H), 2.00-1.89 (m, 1H), 1.88-1.76 (m, 2H).

### Example 14: Preparation of Compound 14

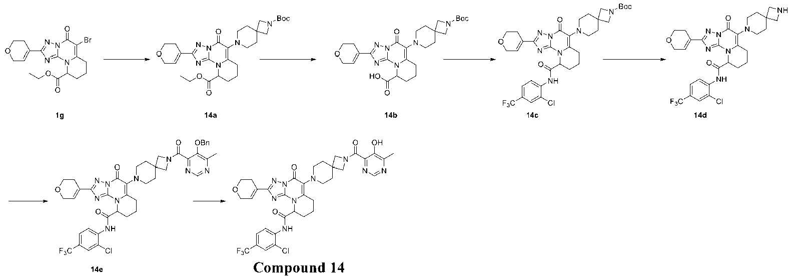

**Compound 14** was afforded with **1g** and **2-tert-butoxycarbonyl-2,7-diazaspiro[3.5]nonane as** the substrates by referring **to** the synthetic method in Example **1.**
LCMS m/z = 754.3 [M+1]⁺

### Preparation of Compound 14-A and Compound 14-B

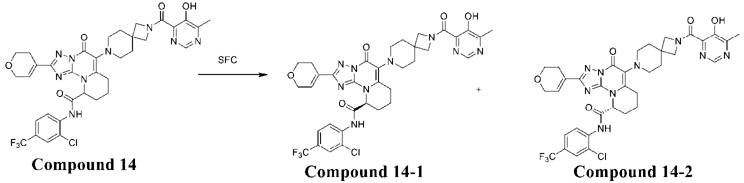

**Compound 14** was purified by SFC on AD column (the instrument and preparative column were as follows: Waters 150 Prep-SFC was employed, and the preparative column model was Chiral AD column). Preparation method: **Compound 14** was dissolved in acetonitrile and filtered with a 0.45 µm filter membrane to prepare into a sample solution. Mobile phase system: A for CO₂ and B for isopropanol. Gradient elution method: Isocratic elution with 55% mobile phase B (flow rate: 100 mL/min; elution time: 4.0 min). Compound **14-A** and Compound **14-B** were afforded after lyophilization.

Analytical method (instrument and preparative column: high-performance liquid chromatograph-normal-phase chromatograph, and analytical column model: Chiral AD column; mobile phase system: A for CO2; B for 0.05% DEA in isopropanol; column temperature: 35°C; flow rate: 3.0 mL/min). Retention time T = 0.403 min for Compound **14-A.** Retention time T = 0.808 min for Compound **14-B** (one of Compound 14-A and Compound 14-B is Compound 14-1, and the other is Compound 14-2).

### Compound 14-A:

LCMS m/z = 754.2[M+H]⁺;
¹H NMR (400 MHz, CDCl₃) δ 12.69-12.46 (m 1H), 8.69-8.55 (m, 1H), 8.47 (s, 1H), 8.44-8.38 (m, 1H), 7.67- 7.63 (m, 1H), 7.56-7.50 (m, 1H), 6.94- 6.79 (m, 1H), 5.57-5.41 (m, 1H), 4.74 -4.65 (m, 1H), 4.57-4.47 (m, 1H), 4.39-4.24 (m, 2H), 4.14 -4.05 (m, 1H), 3.96-3.83 (m, 3H), 3.62-3.46 (m, 2H), 3.39-3.24 (m, 1H), 3.17-2.98 (m, 1H), 2.85-2.72 (m 1H), 2.72-2.60 (m, 3H), 2.60-2.48 (m, 4H), 2.36-2.11 (m, 2H), 2.08-1.76 (m, 5H).

### Compound 14-B:

LCMS m/z = 754.2[M+H]⁺;
¹H NMR (400 MHz, CDCl₃) δ 12.56-12.32 (m 1H), 8.66-8.53 (m, 1H), 8.46 (s, 1H), 8.45-8.40 (m, 1H), 7.67- 7.62 (m, 1H), 7.56-7.50 (m, 1H), 6.90-6.82 (m, 1H), 5.53-5.43(m, 1H), 4.75-4.65 (m, 1H), 4.57-4.46 (m, 1H), 4.42 -4.26 (m, 2H), 4.14-4.04 (m, 1H), 3.98-3.80 (m, 3H), 3.63-3.45 (m, 2H), 3.36-3.25 (m, 1H), 3.14 - 2.98 (m, 1H), 2.83- 2.73(m 1H), 2.73-2.61 (m, 3H), 2.61-2.49 (m, 4H), 2.36-2.09 (m, 2H), 2.05-1.74(m, 5H).

**Example 15: Preparation of Compound 15**

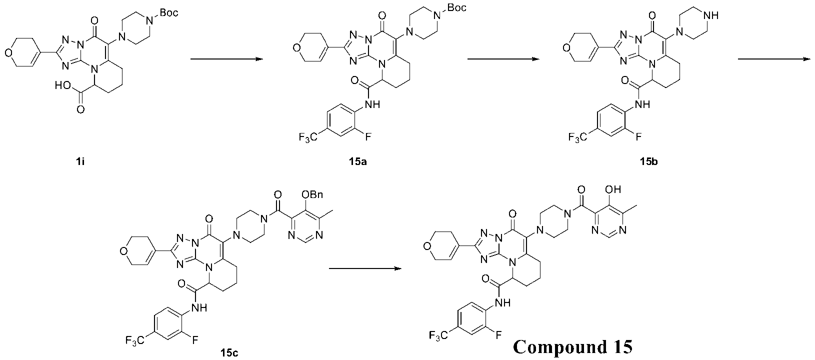

**Compound 15** was afforded with **1i** and **2-fluoro-4-(trifluoromethyl)aniline** as the substrates by referring to the synthetic method in Example 1.
LCMS m/z = 698.2[ M+1]⁺

### Preparation of Compound 15-A and Compound 15-B

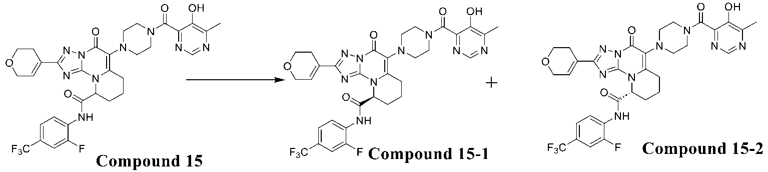

**Compound 15** was purified by SFC on AD column (the instrument and preparative column were as follows: Waters 150 Prep-SFC was employed, and the preparative column model was Chiral IK column). Preparation method: **Compound 15** was dissolved in acetonitrile and dichloromethane, and filtered with a 0.45 µm filter membrane to prepare into a sample solution. Mobile phase system: A for CO₂ and B for 0.1% NH3•H2O in ethanol and acetonitrile. Gradient elution method: Isocratic elution with 60% mobile phase B (flow rate: 100 mL/min; elution time: 4.5 min). Compound **15-A** and Compound **15-B** were afforded after lyophilization.

Analytical method (instrument and preparative column: high-performance liquid chromatograph-normal-phase chromatograph, and analytical column model: Chiral IK column; mobile phase system: A for CO2; B for 0.05% DEA in ethanol and acetonitrile; column temperature: 35°C; flow rate: 3.0 mL/min). Retention time T = 0.478 min for Compound **15-A.** Retention time T = 0.997 min for Compound **15-B** (one of Compound 15-A and Compound 15-B is Compound 15-1, and the other is Compound 15-2).

### Compound 15-A:

¹H NMR (400 MHz, CDCl₃) δ 12.53 (s, 1H), 8.72 - 8.62 (m, 2H), 8.43 - 8.33 (m, 1H), 7.45 - 7.35 (m, 2H), 6.97 - 6.89 (m, 1H), 5.60 - 5.40(m, 2H), 4.87 - 4.72 (m, 1H), 4.39 - 4.30 (m, 2H), 3.98 - 3.86 (m, 2H), 3.83 - 3.68(m, 2H), 3.62 - 3.46 (m, 1H), 3.46 - 3.31 (m, 1H), 3.17 - 3.02(m, 2H), 2.99 - 2.79 (m, 2H), 2.76 (s, 3H), 2.74 - 2.63 (m, 2H), 2.62 - 2.49(m, 1H), 2.34 - 2.19 (m, 2H), 1.99 - 1.86 (m, 1H).

### Compound 15-B:

¹H NMR (400 MHz, CDCl₃) δ 11.91(s, 1H), 8.66 - 8.56 (m, 2H), 8.43 - 8.36 (m, 1H), 7.46 - 7.34 (m, 2H), 6.91 - 6.85 (m, 1H), 5.68 - 5.42(m, 2H), 4.86- 4.66 (m, 1H), 4.39 - 4.28 (m, 2H), 3.98 - 3.83(m, 2H), 3.83 - 3.67(m, 2H), 3.68- 3.41 (m, 1H), 3.41 - 3.31 (m, 1H), 3.18 - 2.98(m, 2H), 2.94 - 2.73 (m, 2H), 2.73 - 2.65 (m, 2H),2.59 (s, 3H) , 2.58 - 2.51(m, 1H), 2.36 - 2.16 (m, 2H), 1.99 - 1.87 (m, 1H).

### Example 16: Preparation of Compound 16

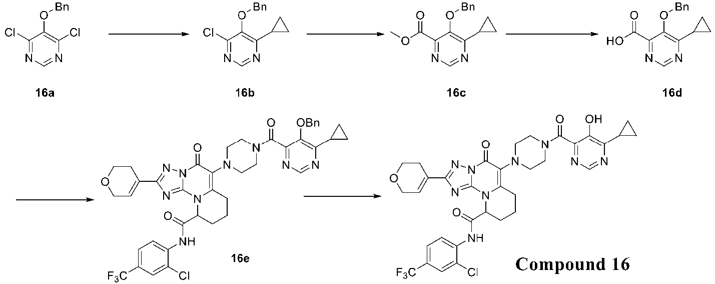

### Step 1: Preparation of 16b

**16a** (4.00 g, 15.68 mmol) was dissolved in tetrahydrofuran (50 mL). The system was purged with nitrogen, and then cyclopropylmagnesium bromide (24 mL, 24.00 mmol, 1 M solution in THF) and [1,1'-bis(diphenylphosphino)ferrocene]palladium(II)dichloride dichloromethane complex (1.28 g, 1.57 mmol) were successively added in an ice-water bath. The mixture was reacted at this temperature for 3 h. The reaction was quenched by adding 1 M hydrochloric acid solution (25 mL), and the mixture was extracted with ethyl acetate (25 mL x 2) and washed with saturated brine (25 mL). The organic phases were dried over anhydrous sodium sulfate, and then concentrated under reduced pressure. The residue was purified by column chromatography (petroleum ether : ethyl acetate (v : v) = 75 : 25) to afford **16b** (2.94 g, yield: 72%).
LCMS m/z = 261.3 [M+H]⁺

### Step 2: Preparation of 16c

**16b** (2.94 g, 11.28 mmol) was dissolved in methanol (50 mL) and N,N-dimethylacetamide (10 mL). [1,1'-Bis(diphenylphosphino)ferrocene]palladium(II)dichloride dichloromethane complex (0.92 g, 1.13 mmol) and triethylamine (3.42 g, 33.80 mmol) were added, and the mixture was heated to 75°C under carbon monoxide atmosphere (0.3-0.5 MPa) and reacted for 16 h. The mixture was cooled to room temperature and concentrated under reduced pressure. The residue was purified by column chromatography (petroleum ether : ethyl acetate (v : v) = 67 : 33) to afford 16c (0.62 g, yield: 19%).
LCMS m/z = 285.2 [M+H]⁺

### Step 3: Preparation of 16d

**16c** (0.62 g, 2.18 mmol) was dissolved in methanol (5 mL) and tetrahydrofuran (5 mL). 2 M aqueous sodium hydroxide solution (5 mL) was added, and the mixture was reacted at room temperature for 10 min. The solvents were removed by concentration under reduced pressure. The pH was adjusted to 2-3 with 2 M hydrochloric acid solution, a solid was precipitated, the solid was filtered, and the filter cake was dried to afford **16d** (0.58 g, yield: 98%).
LCMS m/z = 271.2 [M+H]⁺

### Preparation of Compound 16

**Compound 16** was afforded with **16d** and **1k** as the substrates by referring to the synthetic method in Example 1.
LCMS m/z = 740.2[ M+H]⁺

### Example 17: Preparation of Compound 17

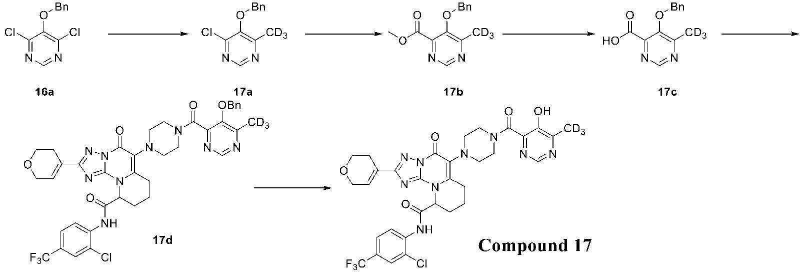

**Compound 17** was afforded with **methyl-D3-magnesium iodide as** the substrate by referring to the synthetic method in **Example 16.**
LCMS m/z = 717.2[ M+H]⁺

### Example 18: Synthesis of Compound 18

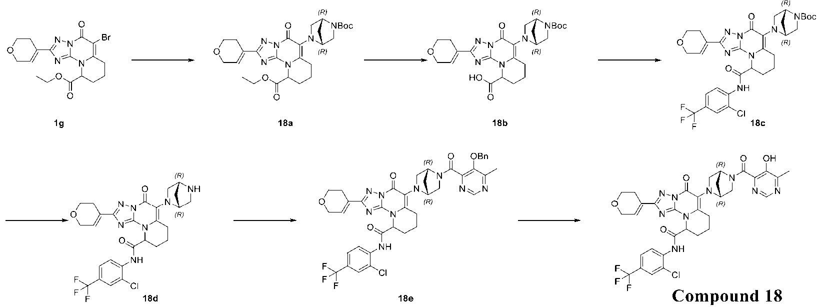

**Compound** 18 was afforded with **1g and *tert-butyl* (1R,4R)-2,5-diazabicyclo[2.2.1]heptane-2-carboxylate** as the substrates by referring to the synthetic method in Example 1.
LCMS m/z = 726.2 [M+1]⁺
¹H NMR (400 MHz, CF3COOD) δ 9.68-8.86(m, 1H), 8.08-7.94 (m, 1H), 7.92-7.85 (m, 1H), 7.76-7.65(m, 1H), 7.25-7.11 (m, 1H), 6.13-5.93 (m, 1H), 5.66-5.08 (m, 2H), 5.07-4.82(m, 1H), 4.81-4.35(m, 4H), 4.34-3.99 (m, 3H), 3.89-3.29(m, 2H), 3.28-2.95 (m, 4H), 2.92-2.56 (m, 5H), 2.48-2.23 (m, 2H).

### Example 19: Synthesis of Compound 19

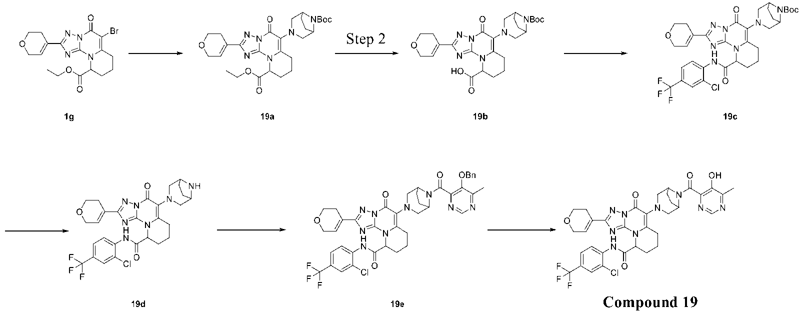

**Compound 19** was afforded with **1g** and ***tert-butyl* 3,8-diazabicyclo[3.2.1]octane-8-carboxylate** as the substrates by referring to the synthetic method in Example 1.
LCMS m/z = 740.2 [M+1]⁺
¹H NMR (400 MHz, CF3COOD) δ 9.09-8.99(m, 1H), 8.12-8.00 (m, 1H), 7.91 (s, 1H), 7.78-7.69(m, 1H), 7.26-7.15 (m, 1H), 6.19-6.07 (m, 1H), 6.06-5.93 (m, 1H), 5.32-5.17(m, 1H), 4.80-4.60(m, 2H), 4.39-4.03 (m, 4H), 3.91-3.73(m, 1H), 3.53-3.31 (m, 1H), 3.20-2.96 (m, 5H), 2.94-2.67 (m, 4H), 2.58-2.14 (m, 6H).

### Example 20: Synthesis of Compound 20-A and Compound 20-B

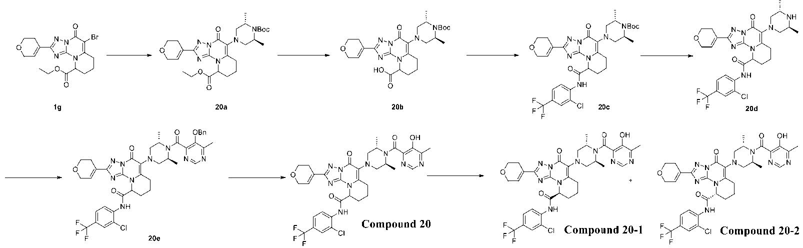

**Compound 20** (crude) was afforded with **1g** and ***tert-butyl* (2S,6S)-2,6-dimethylpiperazine-1-carboxylate** as the substrates by referring to the synthetic method in Example 1. Compound 20 (200 mg) was purified by Pre-HPLC (the instrument and preparative column were as follows: Waters 2767 preparative liquid chromatograph was employed, and the preparative column model was Xbridge Prep C18 (19 mm × 250 mm) 5 um with an inner diameter * length of 19 mm * 150 mm). Preparation method: The crude product was dissolved in DMF and filtered with a 0.45 µm filter membrane to prepare into a sample solution. Mobile phase system: Acetonitrile/water (with 0.05% ammonium bicarbonate). Gradient elution method: An acetonitrile gradient from 10% to 55% (flow rate: 15 mL/min; elution time: 20 min). Compound **20-A** (18 mg, retention time T = 17.85 min) and Compound **20-B** (13 mg, retention time T = 19.15 min) were afforded after lyophilization.

Analytical method (instrument: SHIMADZU LC-20AT; preparative column model: CHIRALPAKAS-H, 4.6 x 150 mm, 5 µm; Mobile phase system: n-hexane : ethanol = 70 : 30; wavelength: 210 nm; column temperature: 35°C; flow rate: 1.0 ml/min). Retention time **T** = 6.573 min for Compound 20-A, and retention time T = 4.488 min for **Compound 20-B** (one of **Compound 20-A** and **Compound 20-B** is Compound 20-1, and the other is Compound 20-2).

According to Example 32, **Compound 20-A** was confirmed to be **Compound 20-2.**

### Compound 20-A

LCMS m/z = 742.0 [M+1]⁺
¹H NMR (400 MHz, CF3COOD) δ 9.12 (s, 1H), 8.06 (d, 1H), 7.93 (s, 1H), 7.76 (d, 1H), 7.33-7.20 (m, 1H), 6.20-6.00 (m, 1H), 5.25-4.86 (m, 2H), 4.82- 4.65 (m, 2H), 4.60-4.42 (m, 1H), 4.41-4.24 (m, 2H), 4.16-3.57 (m, 4H), 3.50-3.31 (m, 1H), 3.07 (s, 3H), 2.99-2.70 (m, 4H), 2.42-2.23 (m, 2H) , 1.91(s, 6H).

### Compound 20-B

LCMS m/z = 742.0 [M+1]⁺
¹H NMR (400 MHz, CF3COOD) δ 9.11 (s, 1H), 8.06 (d, 1H), 7.93 (s, 1H), 7.76 (d, 1H), 7.33-7.20 (m, 1H), 6.14-5.94 (m, 1H), 5.16-4.83 (m, 2H), 4.82- 4.63 (m, 2H), 4.48-4.23 (m, 3H), 4.17-4.05 (m, 1H), 3.96-3.80 (m, 1H), 3.79-3.32 (m, 3H), 3.07 (s, 3H), 2.97-2.88 (m, 2H), 2.87-2.73 (m, 2H), 2.44-2.24 (m, 2H) , 1.90(s, 6H).

### Example 21: Synthesis of Compound 21

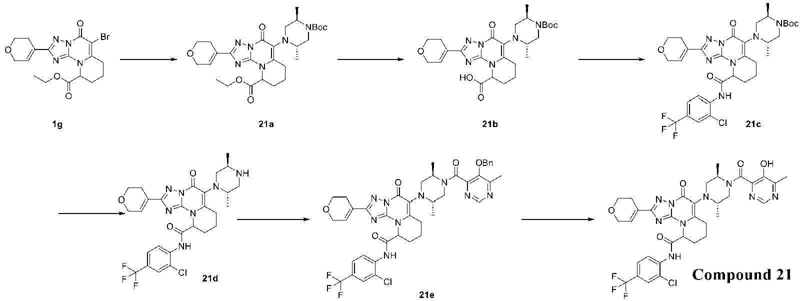

**Compound 21** was afforded with **1g** and ***tert-butyl* (2R,5S)-2,5-dimethylpiperazine-1-carboxylate** as the substrates by referring to the synthetic method in Example 1.
LCMS m/z = 742.3 [M+1]⁺
¹H NMR (400 MHz, CF3COOD) δ 9.17-9.08 (m, 1H), 8.08-8.00 (m, 1H), 7.93 (s, 1H), 7.80-7.71 (m, 1H), 7.33-7.22 (m, 1H), 6.17-5.99 (m, 1H), 5.68-5.21 (m, 1H), 5.10- 4.90 (m, 1H), 4.87-4.65 (m, 3H), 4.62-4.40 (m, 1H), 4.39-4.03 (m, 3H), 4.02-3.27 (m, 3H), 3.08 (s, 3H), 2.98-2.69 (m, 4H), 2.49-2.24 (m, 2H), 1.90-1.59 (m, 6H).

### Example 22: Synthesis of Compound 22

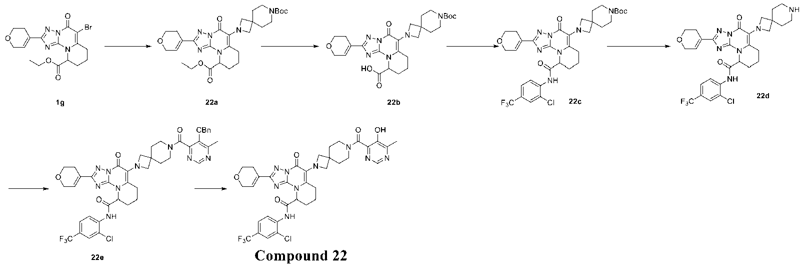

**Compound 22** was afforded with **1g** and ***tert-butyl 2,7-*diazaspiro[3.5]nonane-7-carboxylate** as the substrates by referring to the synthetic method in Example 1.
LCMS m/z = 754.1 [M+1]⁺
¹H NMR (400 MHz, CF3COOD) δ 9.13 (s, 1H), 8.04 (d, 1H), 7.93 (s, 1H), 7.75 (d, 1H), 7.31-7.22 (m, 1H), 6.08-5.99 (m, 1H), 5.48-5.01 (m, 4H), 4.81- 4.62 (m, 2H), 4.43-4.21 (m, 4H), 4.18-4.01 (m, 2H), 3.64-3.50 (m, 1H), 3.49-3.34 (m, 1H), 3.06 (s, 3H), 2.98-2.88 (m, 2H), 2.87-2.55 (m, 6H), 2.41-2.25 (m, 2H).

### Example 23: Synthesis of Compound 23

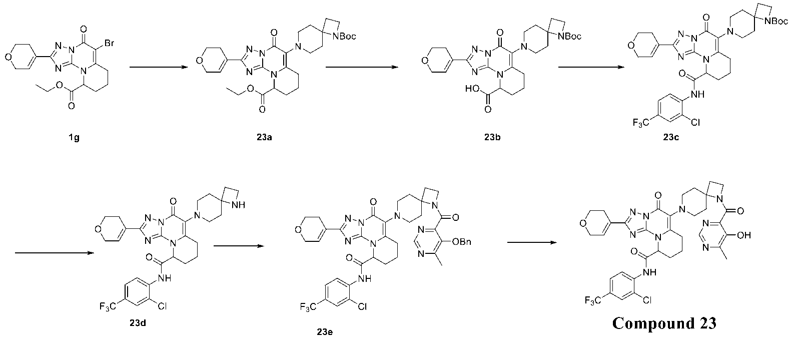

**Compound 23** was afforded with **1g** and ***tert-butyl 2,7-*diazaspiro[3.5]nonane-7-carboxylate** as the substrates by referring to the synthetic method in Example 1.
LCMS m/z **=** 754.3 [M+1]⁺

### Example 24: Synthesis of Compound 24

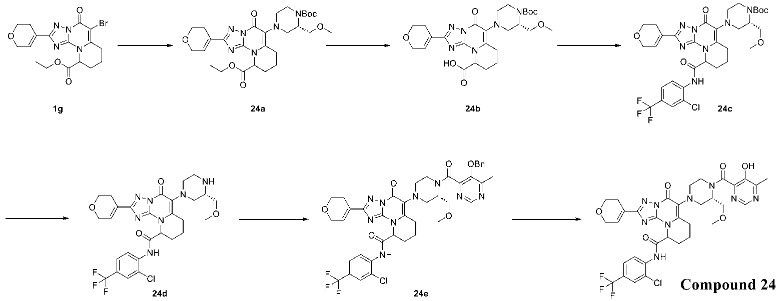

**Compound 24** was afforded with **1g** and ***tert-butyl* (2S)-2-(methoxymethyl)piperazine-1-carboxylate** as the substrates by referring to the synthetic method in Example 1.
LCMS m/z = 758.2 [M+1]⁺
¹H NMR (400 MHz, CF3COOD) δ 9.26-9.04 (m, 1H), 8.10-7.89 (m, 2H), 7.86-7.71 (m, 1H), 7.38-7.20 (m, 1H), 6.21-6.08 (m, 1H), 6.07-5.76 (m, 1H), 5.71-5.36 (m, 1H), 5.35- 4.92 (m, 2H), 4.87-4.52 (m, 4H), 4.48-4.05 (m, 5H), 3.96-3.83 (m, 3H), 3.73-3.42 (m, 2H), 3.10 (s, 3H), 2.99-2.76 (m, 4H), 2.57-2.38 (m, 2H).

### Example 25: Synthesis of Compound 25

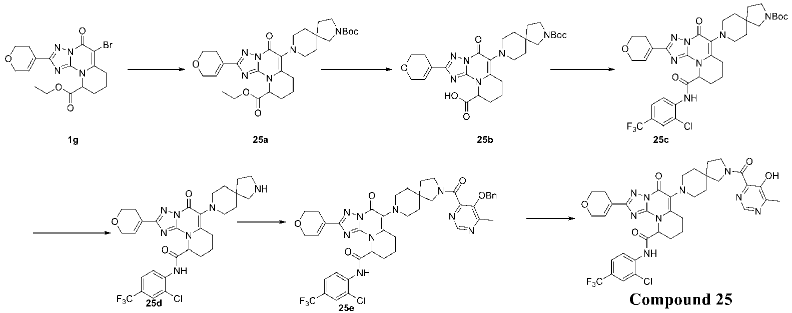

**Compound 25** was afforded with **1g** and **2-BOC-2,8-diazaspiro[4.5]decane** as the substrates by referring to the synthetic method in Example 1.
LCMS m/z = 768.2 [M+1]⁺
¹H NMR (400 MHz, CDCl3) δ 13.08-12.53(m, 1H), 8.74-8.57 (m, 1H), 8.51-8.39 (m, 2H), 7.68-7.62 (m, 1H), 7.57-7.48 (m, 1H), 6.91- 6.83(m, 1H), 5.52-5.45 (m, 1H), 4.42-4.21 (m, 3H), 4.09-3.49(m, 6H), 3.39-3.23(m, 1H), 3.16-2.97 (m, 1H), 2.80-2.50 (m, 7H), 2.39-2.08(m, 3H), 2.07-2.00 (m, 1H), 1.99-1.60 (m, 7H).

### Example 26: Synthesis of Compound 26-A and Compound 26-B

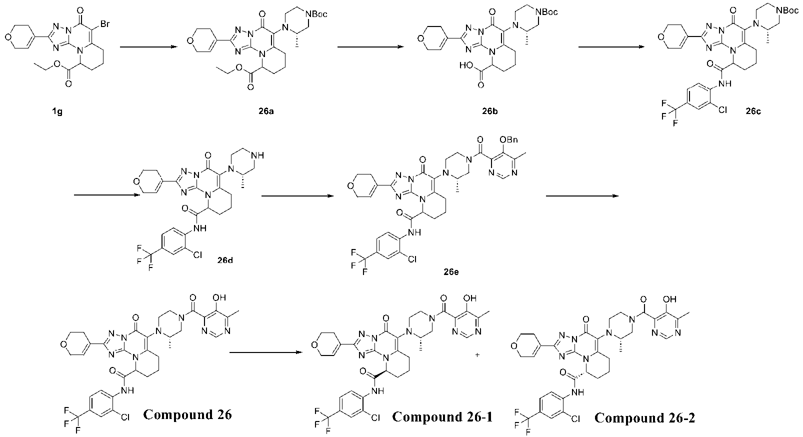

**Compound** 26 (crude) was afforded with 1g and **(S)-4-N-tert-butoxycarbonyl-2-methylpiperazine** as the substrates by referring to the synthetic method in Example 1. Compound 26 was purified by Pre-HPLC (the instrument and preparative column were as follows: Waters 2767 preparative liquid chromatograph was employed, and the preparative column model was Xbridge Prep C18 (19 mm × 250 mm) 5 um with an inner diameter * length of 19 mm * 150 mm). Preparation method: The crude product was dissolved in DMF and filtered with a 0.45 µm filter membrane to prepare into a sample solution. Mobile phase system: Acetonitrile/water (with 0.05% ammonium bicarbonate). Gradient elution method: An acetonitrile gradient from 20% to 55% (flow rate: 15 mL/min; elution time: 14 min). Compound 26-A (retention time T = 12.25 min) and Compound 26-B (retention time T = 13.51 min) were afforded after lyophilization (one of Compound 26-A and Compound 26-B is Compound 26-1, and the other is Compound 26-2).

### Compound 26-A

LCMS m/z = 728.0 [M+1]⁺
¹H NMR (400 MHz, CF3COOD) δ 9.17-9.05 (m, 1H), 8.10-7.96 (m, 1H), 7.94 (s, 1H), 7.82-7.70 (m, 1H), 7.38 -7.20 (m, 1H), 6.15-6.01 (m, 1H), 5.91-5.68 (m, 1H), 5.42-5.13 (m, 2H), 5.05-4.62 (m, 3H), 4.59-4.10 (m, 4H), 4.09 -3.67 (m, 2H), 3.55-3.34 (m, 1H), 3.09 (s, 3H), 2.98-2.74 (m, 4H), 2.51-2.29 (m, 2H), 1.76-1.54 (m, 3H).

### Compound 26-B

LCMS m/z = 727.9 [M+1]⁺
¹H NMR (400 MHz, CF3COOD) δ 9.16-9.05 (m, 1H), 8.10-7.98 (m, 1H), 7.94 (s, 1H), 7.82-7.70 (m, 1H), 7.38 -7.20(m, 1H), 6.15-6.01 (m, 1H), 5.91-5.68 (m, 1H), 5.39-5.09 (m, 2H), 5.02-4.62 (m, 3H), 4.59 - 4.13(m, 3H), 4.12 -3.74 (m, 2H), 3.73-3.47 (m, 2H), 3.09 (s, 3H), 2.98-2.72 (m, 4H), 2.56-2.29 (m, 2H), 1.78-1.55 (m, 3H).

### Example 27: Synthesis of Compound 27

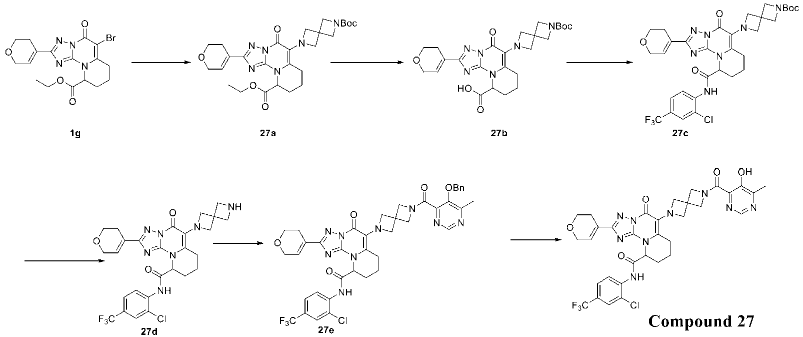

**Compound 27** was afforded with **1g** and ***tert-butyl 2,6-*diazaspiro[3.3]heptane-2-carboxylate** as the substrates by referring to the synthetic method in Example 1.
LCMS m/z = 726.0 [M+1]⁺
¹H NMR (400 MHz, CDCl₃) δ 12.34 (s, 1H), 8.58 (s, 1H), 8.45-8.32 (m, 2H), 7.70-7.62(m, 1H), 7.57-7.50 (m, 1H), 6.92-6.85(m, 1H), 5.55-5.43 (m, 1H), 5.20-5.05 (m, 2H), 4.53-4.24 (m, 8H), 3.96-3.83 (m, 2H), 3.48-3.31 (m, 1H), 3.20-3.05 (m, 1H), 2.78-2.64 (m, 2H), 2.60-2.46 (m, 4H), 2.33-2.23 (m, 1H), 2.22-2.08 (m, 1H), 2.00 - 1.90 (m, 1H).

### Example 28: Preparation of Compound 28

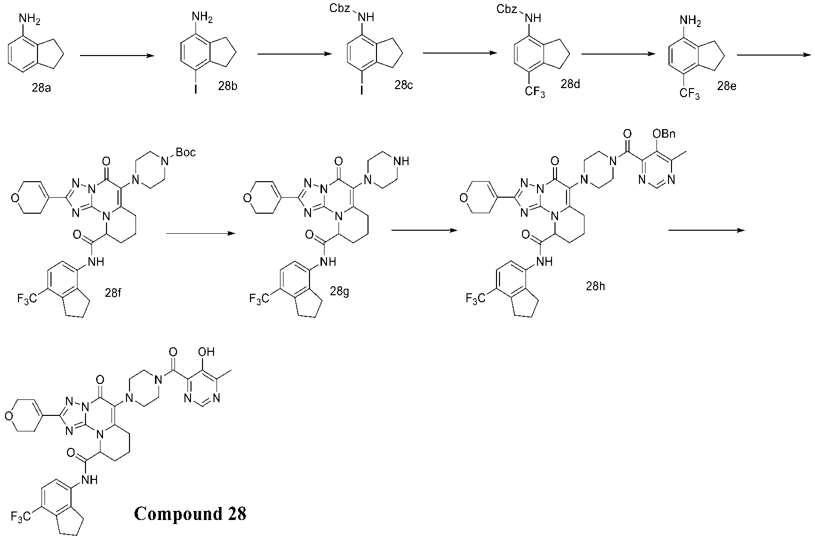

### Step 1: Preparation of 28b

N-Iodosuccinimide (3.90 g, 17.34 mmol) was added to **28a** (2.31 g, 17.34 mmol) in N,N-dimethylformamide (15 mL), and the mixture was reacted at room temperature for 16 h. At 0°C, water (50 mL) was added, and the mixture was extracted with ethyl acetate (20 mL x 2). The organic phases were combined, then washed with a saturated aqueous sodium chloride solution (100 mL), dried over anhydrous sodium sulfate, and then filtered. The filtrate was concentrated under reduced pressure to afford the crude product, which was purified by column chromatography (petroleum ether : ethyl acetate (v : v) = 92 : 8) to give 28b (4.15 g, yield: 92%).
LCMS m/z =260.0[M+H]⁺

### Step 2: Preparation of 28c

At 0°C, benzyl chloroformate (3.01 g, 17.62 mmol) in tetrahydrofuran (10 mL) was added dropwise to **28b** (4.15 g, 16.02 mmol) and sodium bicarbonate (2.96 g, 35.24 mmol) in tetrahydrofuran (40 mL), and the mixture was reacted at room temperature for 16 h. At 0°C, water (100 mL) was added, and the mixture was extracted with ethyl acetate (50 mL x 2). The organic phases were combined, then washed with a saturated aqueous sodium chloride solution (100 mL), dried over anhydrous sodium sulfate, and then filtered. The filtrate was concentrated under reduced pressure to afford the crude product, which was purified by column chromatography (petroleum ether : ethyl acetate (v : v) = 95 : 5) to give **28c** (5.47 g, yield: 87%).
LCMS m/z =394.0[M+H]⁺

### Step 3: Preparation of 28d

Copper(I) iodide (2.58 g, 13.57 mmol), methyl fluorosulfonyldifluoroacetate (10.43 g, 54.28 mmol), and hexamethylphosphoric triamide (9.73 g, 54.28 mmol) were added to **28c** (5.34 g, 13.58 mmol) in N,N-dimethylformamide (100 mL), and the mixture was reacted at 75°C for 16 h under nitrogen atmosphere. The mixture was cooled to room temperature and filtered, and the filter cake was washed with ethyl acetate. Water (500 mL) was added to the filtrate, and the mixture was extracted with ethyl acetate (100 mL x 2). The organic phases were combined, then washed with a saturated aqueous sodium chloride solution (200 mL), dried over anhydrous sodium sulfate, and then filtered. The filtrate was concentrated under reduced pressure to afford the crude product, which was purified by column chromatography (petroleum ether : ethyl acetate (v : v) = 95 : 5) to give **28d** (4.23 g, yield: 93%).
LCMS m/z =336.1[M+H]⁺

### Step 4: Preparation of 28e

Palladium hydroxide on carbon (10%, 1.2 g) was added to **28d** (3.96 g, 11.81 mmol) in absolute methanol (100 mL), and the mixture was reacted at 45°C for 16 h. The mixture was cooled to room temperature and filtered. The filtrate was concentrated under reduced pressure to afford **28e** (2.26 g, crude).
LCMS m/z =202.1[M+H]⁺

**Compound 28** was afforded with **1i** and **28e** as the substrates by referring to the synthetic method in Example **1.**
LCMS m/z = 720.3[M+1]⁺
¹H NMR (400 MHz, CDCl₃) δ 11.85 (s, 1H), 8.59 (s, 1H), 8.12 (s, 1H), 7.88(d, 1H), 7.42 (d, 1H), 6.93-6.80(m, 1H), 5.72-5.45 (m, 2H), 4.90-4.62 (m, 1H), 4.45-4.18 (m, 2H), 3.99-3.84 (m, 2H), 3.83-3.65 (m, 2H), 3.59-3.25 (m, 2H), 3.24-2.97 (m, 4H), 2.93-2.52 (m, 10H), 2.36-2.08 (m, 4H), 1.97-1.87 (m, 1H).

### Example 29: Preparation of Compound 29

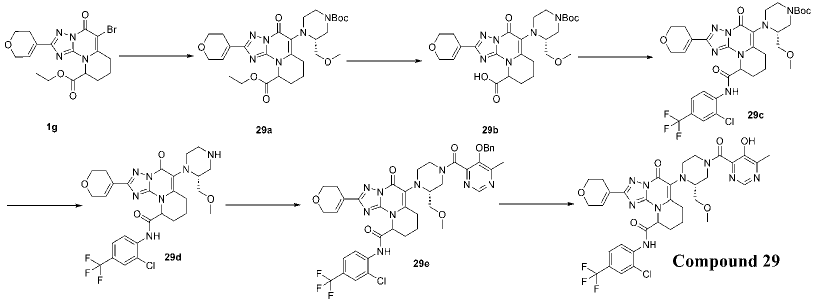

**Compound 29** was afforded with **1g** and ***tert-butyl* (R)-3-(methoxymethyl)piperazine-1-carboxylate** as the substrates by referring to the synthetic method in Example 1.
LCMS m/z = 758.2 [M+1]⁺
¹H NMR (400 MHz, CF₃COOD) δ 9.18-9.06 (m, 1H), 8.11-7.87 (m, 2H), 7.81-7.69(m, 1H), 7.36-7.22 (m, 1H), 6.23-5.96 (m, 1H), 5.91-5.60 (m, 1H), 5.42-5.08 (m, 2H), 4.96 -4.19 (m, 6H), 4.13 -3.35 (m, 9H), 3.09 (s, 3H), 2.99-2.70 (m, 4H), 2.54-2.28 (m, 2H).

### Example 30: Preparation of Compound 1-2

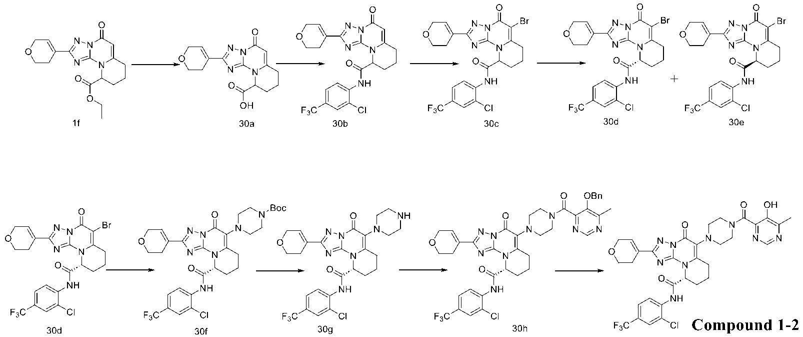

### Step 1: Preparation of 30a

Lithium hydroxide monohydrate (3.86 g, 92.04 mmol) was added to 1f (7.92 g, 23.01 mmol) in tetrahydrofuran (80 mL), water (20 mL), and methanol (40 mL), and the mixture was reacted at room temperature for 1 h. At 0°C, the pH was adjusted to 2-3 with hydrochloric acid (1 N). Saturated brine (20 mL) was added, followed by the addition of sodium chloride to saturate the aqueous phase. The mixture was extracted with ethyl acetate (30 mL x 15). The organic phases were combined, dried over anhydrous sodium sulfate, and filtered. The filtrate was concentrated under reduced pressure to afford **30a** (6.70 g, crude). LCMS m/z = 317.2[M+H]⁺

### Step 2: Preparation of 30b

At 0°C, oxalyl chloride (3.48 mL, 41.12 mmol) was slowly added to 30a (6.50 g, crude) in anhydrous dichloromethane (160 mL), N,N-dimethylformamide (0.47 mL) was added, and the mixture was reacted at room temperature for 1 h under nitrogen protection. Triethylamine (10.40 g, 102.75 mmol) and 2-chloro-4-(trifluoromethyl)aniline (12.06 g, 61.65 mmol) in dichloromethane (32 mL) was added, and the mixture was reacted at room temperature for 1 h. Silica gel was added, and the mixture was concentrated under reduced pressure to afford the residue, which was purified by column chromatography (dichloromethane : (dichloromethane/methanol = 10/1) (v : v) = 84 : 16) to give **30b** (8.47 g). LCMS m/z =494.1[M+H]⁺

### Step 3: Preparation of 30c

N-Bromosuccinimide (3.36 g, 18.86 mmol) was added to **30b** (8.47 g, 17.15 mmol) in N,N-dimethylformamide (150 mL), and the mixture was reacted at 50°C for 2 h. Ice water (1 L) was added, and the mixture was stirred for 30 min before being filtered to afford the crude product. The crude product was slurried with ethyl acetate (100 mL) to afford **30c** (7.90 g, yield: 80%). LCMS m/z = 572.0[M+H]⁺

**Compound 30c** was purified by SFC on IC column (the instrument and preparative column were as follows: Waters 150 Prep-SFC was employed, and the preparative column model was Chiral IC column). Preparation method: **Compound 1** was dissolved in acetonitrile and methanol, and filtered with a 0.45 µm filter membrane to prepare into a sample solution with a concentration of 50 mg/ml. Mobile phase system: A for CO₂ and B for isopropanol and acetonitrile. Gradient elution method: Isocratic elution with 60% mobile phase B (flow rate: 100 mL/min; elution time: 6 min). **30d** and **30e** were afforded after lyophilization.

Analytical method (instrument and preparative column: SHIMADZU LC-20AT; analytical column model: Chiralcel AD-H, 4.6 x 250 mm 5 µm; system: n-hexane : ethanol = 80 : 20; wavelength: 210 nm; column temperature: 35°C; flow rate: 1.0 mL/min). Retention time T = 17.481 min for **30d,** and retention time T = 24.569 min for **30e.**
**30d:** LCMS m/z = 572.0[M+H]⁺;
**30e:** LCMS m/z = 572.1[M+H]+;

### Step 5: Preparation of 30f

N-Boc-Piperazine (326 mg, 1.75 mmol) was added to 30d (200 mg, 0.35 mmol) in dimethyl sulfoxide (4 mL), and the mixture was reacted at 110°C for 1 h. The mixture was cooled to room temperature, water (30 mL) was added, and the mixture was extracted with ethyl acetate (20 mL x 2). The organic phases were combined, then washed with saturated brine, dried over anhydrous sodium sulfate, and filtered. The filtrate was concentrated under reduced pressure to afford the crude product, which was purified by column chromatography (dichloromethane : (dichloromethane/methanol = 10/1) (v : v) = 80 : 20) to give 30f (394 mg, crude).
LCMS m/z = 622.1[M-55]⁺

**Compound 1-2 was** afforded with **30f** as the substrate by referring to the synthetic method in **Example 1.**
LCMS m/z = 714.2[M+H]⁺;
¹H NMR (400 MHz, CDCl₃) δ 11.86 (s, 1H), 8.60 (s, 1H), 8.53-8.35 (m, 2H), 7.66 (s, 1H), 7.54 (d, 1H), 6.95-6.80 (m, 1H), 5.72-5.54 (m, 1H), 5.53-5.43(m, 1H), 4.94-4.62 (m, 1H), 4.44-4.19 (m, 2H), 4.00-3.66 (m, 4H), 3.60-3.28 (m, 2H),3.21-2.97 (m, 2H), 2.94-2.45 (m, 8H), 2.37-2.12 (m, 2H) ,2.03-1.89 (m, 1H).

Analytical method (instrument: SHIMADZU LC-20AT; preparative column model: CHIRALCEL OD-HOD-H, 4.6 x 250 mm, 5 µm; mobile phase system: n-hexane : ethanol = 60 : 40; wavelength: 210 nm; column temperature: 35°C; flow rate: 1.0 ml/min). Retention time T = 16.026 min.

**Compound 1-B** obtained from Example 1 was confirmed to be **Compound 1-2** by chiral HPLC.

### Example 31: Preparation of Compound 31

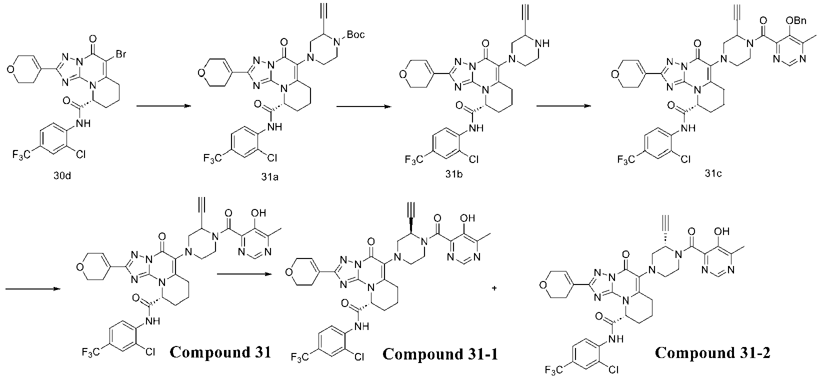

Anhydrous lithium chloride (70 mg, 1.64 mmol) was added to **31c** (340 mg, 0.41 mmol; **31c** was afforded by referring to the synthetic method of Example 30 using **30d** and *tert-butyl* 2-ethynylpiperazine-1-carboxylate as the starting materials) in N,N-dimethylformamide (4 mL), and the mixture was reacted under microwave irradiation at 150°C for 2 h. Water (40 mL) was added, and the mixture was extracted with dichloromethane (15 mL x 3). The crude product was purified by Pre-HPLC (the instrument and preparative column were as follows: Waters 2767 preparative liquid chromatograph was employed, and the preparative column model was XBridge@Prep C18 with an inner diameter x length of 19 mm x 250 mm). Preparation method: The crude product was dissolved in DMF and filtered with a 0.45 µm filter membrane to prepare into a sample solution. Mobile phase system: Acetonitrile/water (with 5 mmol/L of NH₄HCO₃). Gradient elution method: An acetonitrile gradient from 20% to 59% (flow rate: 15 mL/min; elution time: 13 min). **Compound 31** was afforded after lyophilization.
LCMS m/z = 738.1[M+H]⁺;

**Compound 31** was purified by SFC on Chiral IK column (the instrument and preparative column were as follows: Waters 150 Prep-SFC was employed, and the preparative column model was Chiral IK column). Preparation method: **Compound 31** was dissolved in acetonitrile and filtered with a 0.45 µm filter membrane to prepare into a sample solution. Mobile phase system: A for CO₂ and B for ethanol and acetonitrile. Gradient elution method: Isocratic elution with 50% mobile phase B (flow rate: 100 mL/min; elution time: 9.0 min). Compound **31-A** and Compound **31-B** were afforded after lyophilization. Analytical method (instrument and preparative column: SHIMADZU LC-20AD, and analytical column model: Chiral IK column; mobile phase system: A for CO2; B for 0.05% DEA in ethanol and acetonitrile; column temperature: 35°C; flow rate: 3.0 mL/min). Retention time T = 0.816 min for Compound **31-A,** and retention time T = 1.251 min for Compound **31-B** (one of Compound 31-A and Compound 31-B is Compound 31-1, and the other is Compound 31-2).

### Compound 31-A:

LCMS m/z = 738.2[M+H]⁺;

### Compound 31-B:

LCMS m/z = 738.2[M+H]⁺;

### Example 32: Preparation of Compound 20-2

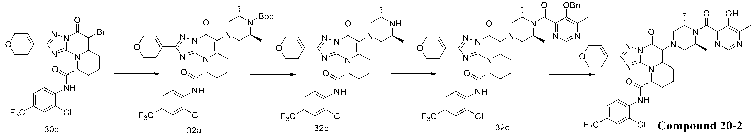

**Compound 20-2 was afforded with 30d and *tert-butyl* (2S,6S)-2,6-dimethylpiperazine-1-carboxylate as the substrates by referring to the synthetic method in Example 30.**

### Compound 20-2

LCMS m/z = 742.0 [M+1]⁺
Analytical method (instrument: SHIMADZU LC-20AT; preparative column model: CHIRALPAKAS-H, 4.6 x 150 mm, 5 µm; mobile phase system: n-hexane : ethanol = 70 : 30; wavelength: 210 nm; column temperature: 35°C; flow rate: 1.0 ml/min). Retention time T = 6.573 min.

**Compound 20-A** obtained from Example 20 was confirmed to be **Compound 20-2** by chiral HPLC.

### Biological Assay Examples

### 1. Assay for ATPase Activity of WRN Helicase

The assay buffer working solution was as follows: 30 mM Tris pH 7.5 + 0.02% BSA + 2 mM MgCl2 + 50 mM NaCl + 0.1% Pluronic^{®} F127 + 1 mM DTT. WRN protein (AA N517-P1238, ICE) was obtained through protein expression and purification *in vitro.* A single-stranded DNA substrate (TTTTTTTTTTTTTTTTTTTTTTCCAAGTAAAACGACGGCCAGTGC) was synthesized *in vitro.* 1 µL each of compounds at different concentrations, the WRN protein, and the DNA substrate, together with 2 µL of ATP, were added to a 384-well white plate. The working concentrations of the WRN protein, the DNA substrate, and the ATP were 0.3 nM, 0.2 nM, and 10 µM, respectively. The plate was incubated at room temperature for 20 minutes. After the incubation was completed, the detection was performed in accordance with the instructions of the ADP-Glo Assay Kit (Promega), and the chemiluminescence value was read.

The inhibition rate was calculated according to Formula 1, where RLUsample is the readout of the compound well, RLUmax is the readout of the DMSO control well, and RLUmin is the readout of the control well without the WRN protein. Curve fitting was performed via a four-parameter logistic model (log(inhibitor) vs. response -- Variable slope) using GraphPad Prism software, and the IC50 value was calculated. $Inhibition % = \left(1-\left(RLUsample-RLUmin\right)/\left(RLUmax-RLUmin\right)\right) \times 100 %$

**Table 1. Results of inhibitory activity of test compounds against ATPase activity of WRN helicase**

| Compound No. | IC₅₀ (µM) |
|---|---|
| Compound 1-B | A |
| Compound 2-B | A |

| | |
|---|---|
| Note: A < 0.02 µM, 0.02 µM ≤ B < 0.1 µM, 0.1 µM ≤ C < 0.5 µM, and 0.5 µM ≤ D < 1 µM | |

Conclusion: The compounds of the present invention, such as the above example compounds, exhibit excellent inhibitory effects on the ATPase activity of WRN helicase.

### 2. Assay for WRN Helicase Activity

The assay buffer working solution prepared with RNAase-free water was as follows: 25 mM Tris (pH 7.5), 2 mM MgCl2, 50 mM NaCl, 0.02% BSA, 0.01% Tween, and 1 mM DTT. WRN protein (AA N517-P1238, ICE, Cat. S2212T-H56HZ) was obtained through protein expression and purification *in vitro.* Two single-stranded DNAs were synthesized: TTTTTTTTTTTTTTTTTTTTTTTTTTTTTTCGTACCCGATGTGTTCGTTC-BHQ2 (quencher), and (fluorophore) TAMRA-GAACGAACACATCGGGTACGTTTTTTTTTTTTTTTTTTTTTTTTTTTTTT. dsDNA (FORKF) was obtained via annealing of the two single-stranded DNAs. When the WRN protein bound to FORKF, the dsDNA was unwound, exposing the fluorophore, thereby generating fluorescence. 2 µL of compounds at different concentrations and 4 µL of WRN protein were added to a 384-well black plate (Greiner, Cat#784075). The plate was pre-incubated at room temperature for 20 minutes. 2 µL each of ATP and the FORKF substrate were added. The working concentrations of the WRN protein, FORKF, and ATP were 10 nM, 10 nM, and 2 mM, respectively. The plate was incubated at room temperature for 20 minutes. After the incubation was completed, the fluorescence value was read at Ex 540 nm/Em 590 nm using a BMG microplate reader (PHERAstar FSX).

The inhibition rate was calculated according to Formula 2, where RLUₛₐₘₚₗₑ is the readout of the compound well, RLUₘₐₓ is the readout of the DMSO control well, and RLUₘᵢₙ is the readout of the control well without the WRN protein. Curve fitting was performed via a four-parameter logistic model using GraphPad Prism software, and the IC₅₀ value (the concentration of the compound when the inhibition rate was 50%) was calculated. $Inhibition % = \left(1-\left({RLU}_{sample}-{RLU}_{min}\right)/\left({RLU}_{max}-{RLU}_{min}\right)\right) \times 100 %$

**Table 2. Results of inhibitory activity of test compounds against WRN helicase activity**

| Compound No. | IC₅₀ (µM) |
|---|---|
| Compound 1 | B |
| Compound 1-B | A |
| Compound 2-B | A |
| Compound 3-B | A |
| Compound 5-B | A |
| Compound 6-B | A |
| Compound 7-B | A |
| Compound 8-B | A |
| Compound 15-B | A |
| Compound 20-A | A |
| Compound 26-A | A |
| Compound 28 | A |

| | |
|---|---|
| Note: A < 0.02 µM, and 0.02 µM ≤ B < 0.1 µM | |

Conclusion: The compounds of the present invention, such as the above example compounds, exhibit excellent inhibitory effects on the WRN helicase activity.

### 3. Cell Proliferation Inhibition Assay

### 3.1. HCT-116 Cell Proliferation Inhibition Assay

The culture conditions for HCT-116 cells were as follows: RPMI-1640 + 10% FBS + 1% penicillin-streptomycin, and the cells were cultured in an incubator with 5% CO₂ at 37°C. On the first day, the HCT-116 cells in the exponential growth phase were collected and plated in 96-well cell culture plates at a density of 800 cells/well (180 µL/well). The plates were cultured overnight in an incubator with 5% CO₂ at 37°C. T₀ wells were plated simultaneously during the plating process. The next day, 20 µL of compounds at different concentrations were added to each well (nine compound concentration gradients, one DMSO well, and one blank well; each compound concentration was plated in triplicate), and the final concentration of DMSO in each well was 0.1%. The plates were cultured in an incubator with 5% CO₂ at 37°C for 4 days. On the same day of compound addition, the T₀ plate was detected using the CellTiter-Glo Kit, and the detected value was recorded as RLU₀. After the culture was completed, 100 µL of detection solution (Cell Viability Assay, Promega) was added to each well. The plates were incubated at room temperature for 10 minutes with slow shaking. The chemiluminescence reading was read using a BMG microplate reader (PHERAstar FSX). The results were processed according to Formula (3) to calculate the proliferation inhibition rate of compounds at each concentration. Curve fitting was performed via a four-parameter logistic model using GraphPad Prism software, and the GI₅₀ value (the concentration of the compound when the proliferation inhibition rate was 50%) was calculated. In the formula, RLU_{compound} is the readout of the drug-treated group, RLU_{control} is the average value of the compound vehicle control group, and RLU_{Blank} is the average value of the blank control group. 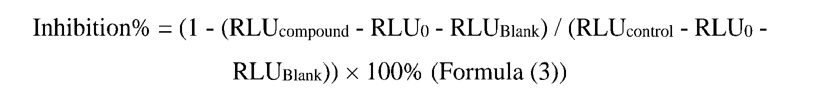 Inhibition% = (1 - (RLUcompound - RLU0 - RLUBlank) / (RLUcontrol - RLU0 - RLUBlank)) × 100% (Formula (3))

### 3.2. HT-29 Cell Proliferation Inhibition Assay

The culture conditions for HT-29 cells were as follows: DMEM + 10% FBS + 1% penicillin-streptomycin, and the cells were cultured in an incubator with 5% CO₂ at 37°C. On the first day, the HT-29 cells in the exponential growth phase were collected and plated in 96-well cell culture plates at a density of 1000 cells/well (180 µL/well). The plates were cultured overnight in an incubator with 5% CO₂ at 37°C. T₀ wells were plated simultaneously during the plating process. The next day, 20 µL of compounds at different concentrations were added to each well (nine compound concentration gradients, one DMSO well, and one blank well; each compound concentration was plated in triplicate), and the final concentration of DMSO in each well was 0.1%. The plates were cultured in an incubator with 5% CO₂ at 37°C for 4 days. On the same day of compound addition, the T₀ plate was detected using the CellTiter-Glo Kit, and the detected value was recorded as RLU₀. After the culture was completed, 100 µL of detection solution (Cell Viability Assay, Promega) was added to each well. The plates were incubated at room temperature for 10 minutes with slow shaking. The chemiluminescence reading was read using a BMG microplate reader (PHERAstar FSX). The results were processed according to Formula (4) to calculate the proliferation inhibition rate of compounds at each concentration. Curve fitting was performed via a four-parameter logistic model using GraphPad Prism software, and the GI₅₀ value (the concentration of the compound when the proliferation inhibition rate was 50%) was calculated. In the formula, RLU_{compound} is the readout of the drug-treated group, RLU_{control} is the average value of the compound vehicle control group, and RLU_{Blank} is the average value of the blank control group. 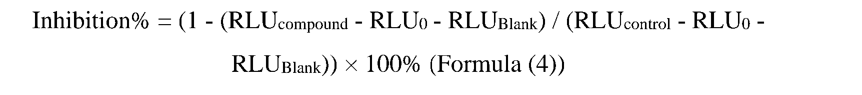 Inhibition% = (1 - (RLUcompound - RLU0 - RLUBlank) / (RLUcontrol - RLU0 - RLUBlank)) × 100% (Formula (4))

### 3.3. SW48 Cell Proliferation Inhibition Assay

The culture conditions for SW48 cells were as follows: Leibovitz L15 + 10% FBS + 1% penicillin-streptomycin, and the cells were cultured in an incubator without CO₂ at 37°C. On the first day, the SW48 cells in the exponential growth phase were collected and plated in 96-well cell culture plates at a density of 1000 cells/well (180 µL/well). The plates were cultured overnight in an incubator without CO₂ at 37°C. T₀ wells were plated simultaneously during the plating process. The next day, 20 µL of compounds at different concentrations were added to each well (nine compound concentration gradients, one DMSO well, and one blank well; each compound concentration was plated in triplicate), and the final concentration of DMSO in each well was 0.1%. The plates were cultured in an incubator without CO₂ at 37°C for 4 days. On the same day of compound addition, the T₀ plate was detected using the CellTiter-Glo Kit, and the detected value was recorded as RLU₀. After the culture was completed, 100 µL of detection solution (Cell Viability Assay, Promega) was added to each well. The plates were incubated at room temperature for 10 minutes with slow shaking. The chemiluminescence reading was read using a BMG microplate reader (PHERAstar FSX). The results were processed according to Formula (5) to calculate the proliferation inhibition rate of compounds at each concentration. Curve fitting was performed via a four-parameter logistic model using GraphPad Prism software, and the GI₅₀ value (the concentration of the compound when the proliferation inhibition rate was 50%) was calculated. In the formula, RLU_{compound} is the readout of the drug-treated group, RLU_{control} is the average value of the compound vehicle control group, and RLU_{Blank} is the average value of the blank control group. 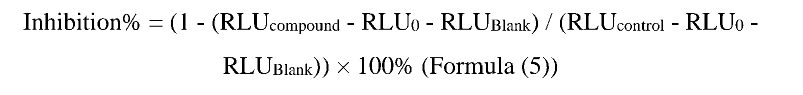 Inhibition% = (1 - (RLUcompound - RLU0 - RLUBlank) / (RLUcontrol - RLU0 - RLUBlank)) × 100% (Formula (5))

**Table 3. Results of inhibitory activity of test compounds against cell proliferation**

| Compound No. | Inhibitory activity against HCT-116 cell proliferation **IC₅₀** (µM) | Inhibitory activity against SW48 cell proliferation **IC₅₀** (µM) | Inhibitory activity against HT-29 cell proliferation **IC₅₀** (µM) |
|---|---|---|---|
| Compound 1-B | A | A | E |
| Compound 1 | / | A | / |
| Compound 2-B | / | A | / |
| Compound 7-B | / | A | / |
| Compound 8-B | / | A | / |
| Compound 20-A | / | A | E |
| Compound 26-A | / | A | E |

| | | | |
|---|---|---|---|
| Note: A < 0.1 µM, 0.1 µM ≤ B < 0.5 µM, 0.5 µM ≤ C < 1 µM, 1 µM ≤ D < 10 µM, and 10 µM ≤ E | | | |

**Conclusion:** The compounds of the present invention, such as the above example compounds, exhibit potent inhibitory effects on cell proliferation in HCT-116 and SW48 cells, but show poor inhibitory activity against cell proliferation in HT-29 cells.

### 4. Mouse Pharmacokinetic Assay

**4.1. Experimental animals:** Male Balb/c mice, about 22 g, 6-8 weeks old, 6 mice/compound. The animals were purchased from CHENGDU DOSSY EXPERIMENTAL ANIMALS CO., LTD.

**4.2. Experimental design:** On the day of the experiment, the Balb/c mice were randomly grouped according to their body weights. The animals were fasted with water available for 12-14 h one day before the administration, and were fed 4 h after the administration.

**Table 4. Administration information**

| Group | Number | Administration information | | | | | |
|---|---|---|---|---|---|---|---|
| | Male | Test substance | Administration dosage (mg/kg) | Administration concentration (mg/mL) | Administration volume (mL/kg) | Collected sample | Administration mode |
| G1 | 3 | Test compound | 2.5 | 0.5 | 5 | Plasma | Intravenous administration |
| G2 | 3 | | 10 | 1 | 10 | Plasma | Intragastric administration |

Vehicle for intravenous administration: 5% DMA + 5% Solutol + 90% Saline; and vehicle for intragastric administration: 5% DMSO + 95% (20% SBE-β-CD in Saline)

Before and after the administration, 0.03 ml of blood was taken from the orbit under isoflurane anesthesia and placed in an EDTAK2 centrifuge tube. The tube was centrifuged at 5000 rpm at 4°C for 10 min, and plasma was collected. The blood collection time points for the intravenous administration group and intragastric administration group were as follows: 0, 2 min, 5 min, 15 min, 30 min, 1 h, 2 h, 4 h, and 7 h. Before analysis and detection, all samples were stored at - 80°C.

**Table 5. Pharmacokinetic parameters of test compounds in mouse plasma**

| Test compound | Administration mode | AUC₀₋ₜ (hr*ng/mL) | F (%) | T1/2 (h) |
|---|---|---|---|---|
| HRO761 | i.g. (10 mg/kg) | 9208 | 74.1 | 2.32 |
| Compound 14-B | i.g. (10 mg/kg) | 24161 | / | 5.41 |
| Compound 20-A | i.g. (10 mg/kg) | 38492 | 93.4 | / |

Conclusion: The compounds of the present invention, such as Compound 1-B, Compound 8-B, and Compound 20-A, exhibit favorable pharmacokinetic properties. In particular, Compound 20-A shows high exposure level and good bioavailability in mice.

### 5. CYP450 Enzyme Activity Inhibition Assay

The purpose of this study was to evaluate the effects of the test compounds on the activities of five isoenzymes (CYP1A2, CYP2C9, CYP2C19, CYP2D6, and CYP3A4) of human liver microsomal cytochrome P450 (CYP) using an *in vitro* testing system. The specific probe substrates for each CYP450 isoenzyme were co-incubated with human liver microsomes and the test compounds at different concentrations, and reduced nicotinamide adenine dinucleotide phosphate (NADPH) was added to initiate the reaction. After the reactions were completed, the samples were treated, and liquid chromatography-tandem mass spectrometry (LC-MS/MS) was used to quantitatively detect metabolites produced by the specific substrates. The changes in CYP enzyme activities were determined, and the IC50 values were calculated to evaluate the inhibitory potential of the test compounds against each CYP enzyme subtype.

Conclusion: The compounds of the present invention, such as Compound 1-B, Compound 8-B, and Compound 20-A, exhibit inhibitory activities against CYP1A2, CYP2C9, CYP2C19, CYP2D6, and CYP3A4, all with > 30 µM.

### 6. hERG Potassium Channel Activity Assay

Experimental platform: Manual electrophysiological patch-clamp system

Cell line: Chinese hamster ovary (CHO) cell line stably expressing hERG potassium channel

Experimental method: In Chinese Hamster Ovary (CHO) cells stably expressing hERG potassium channel, whole-cell patch-clamp technique was used to record hERG potassium channel current at room temperature. The glass microelectrodes were pulled from glass electrode blanks (BF150-86-10, Sutter) by a glass microelectrode puller. The tip resistance of the microelectrodes after being filled with intrapipette solution was about 2-5 MΩ. The glass microelectrodes were connected to the patch-clamp amplifier by inserting them into an amplifier probe. The clamping voltage and data recording were controlled and recorded by the pClamp 10 software through a computer, with a sampling frequency of 10 kHz and a filtering frequency of 2 kHz. After the whole cell records were obtained, the cells were clamped at -80 mV, and the step voltage that elicited the hERG potassium current (I _{hERG}) was depolarized from -80 mV to +20 mV for 2 s, then repolarized to -50 mV, and returned to -80 mV after 1 s. This voltage stimulation was given every 10 s, and the administration process was initiated after the hERG potassium current was confirmed to be stable (at least 1 minute). The compound was administered for at least 1 minute at each test concentration, and at least 2 cells (n ≥ 2) were tested at each concentration.

Data processing: Data analysis and processing were performed using pClamp 10, GraphPad Prism 5, and Excel software. The inhibition degree of hERG potassium current (peak value of hERG tail current elicited at -50 mV) at different compound concentrations was calculated according to the following formula: $Inhibition % = \left[1-\left(I/Io\right)\right] \times 100 %$ where Inhibition% represents the percentage of inhibition of hERG potassium current by the compound, and I and Io represent the amplitude of hERG potassium current after and before the administration, respectively.

Compound IC₅₀ was calculated using GraphPad Prism 5 software by fitting according to the following equation: $Y = Bottom + \left(Top-Bottom\right) / \left(1+{10}^{∧}\left(\left(LogIC50-X\right)*HillSlope\right)\right)$ where X is the Log value of the test compound concentration, Y is the inhibition percentage at the corresponding concentration, and Bottom and Top are the minimum and maximum inhibition percentage, respectively.

Conclusion: The compounds of the present invention, such as Compound 1-B and Compound 8-B, exhibit inhibitory activity against hERG channels, with > 40 µM.

### 7. Beagle Dog Pharmacokinetic Assay

**7.1. Experimental animals:** Male Beagle dogs, about 8-13 kg, 6 Beagle dogs/compound.

**7.2. Experimental method:** On the day of the experiment, the Beagle dogs were randomly grouped according to their body weights. The animals were fasted with water available for 14-18 h one day before the administration, and were fed 4 h after the administration. The animals were administered according to Table 6.

**Table 6. Administration information**

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Administration mode |
| G1 | 3 | Test compound | 1 | 1 | 1 | Plasma | Intravenous administration |
| G2 | 3 | | 3 | 0.6 | 5 | Plasma | Intragastric administration |

| | | | | | | | |
|---|---|---|---|---|---|---|---|
| *Dosage is calculated based on free base. | | | | | | | |

Note: Vehicle for intravenous administration: 5% DMA + 5% Solutol + 90% Saline; and vehicle for intragastric administration: 5% DMSO + 95% (20% SBE-β-CD in Saline) (DMA: dimethylacetamide; Solutol: polyethylene glycol-15-hydroxystearate; Saline: normal saline; and MC: methylcellulose)

Before and after the administration, 1 ml of blood was taken from the jugular veins or limb veins and placed in an EDTAK2 centrifuge tube. The tube was centrifuged at 5000 rpm at 4°C for 10 min, and plasma was collected. The blood collection time points for the intravenous administration group and intragastric administration group were as follows: 0, 5 min, 15 min, 30 min, 1 h, 2 h, 4 h, 6 h, 8 h, 10 h, 12 h, 24 h, and 48 h. Before analysis and detection, all samples were stored at -80°C. The samples were analyzed quantitatively by LC-MS/MS.

**Table 7. Pharmacokinetic parameters of test compounds in Beagle dog plasma**

| Test compound | Administration mode | AUC₀₋ₜ (hr*ng/mL) |
|---|---|---|
| HRO761 | i.g. (3 mg/kg) | 10778 |
| Compound 1-B | i.g. (3 mg/kg) | 19885 |
| Compound 20-A | i.g. (3 mg/kg) | 19664 |

**Conclusion:** The compounds of the present invention, such as the above example compounds, show high exposure levels in dogs.

### 8. In Vivo Efficacy Assay of SW48 Xenograft Tumor in BALB/c Nude Mice

The culture conditions for SW48 cells were as follows: Leibovitz L15 + 10% FBS + 1% penicillin-streptomycin, and the cells were cultured in an incubator without CO₂ at 37°C. When the cell confluency reached 80%-90% and the cell number met the requirement, the cells were harvested and counted. 0.1 mL of SW48 cell suspension (5 × 10⁶ cells) was subcutaneously inoculated into the right posterior back of female BALB/c nude mice. When the average tumor volume reached 100-150 mm³, the mice were grouped according to their tumor volumes, with 10 animals per group. The test compounds at different dosages were administered to the animals in each group via intragastric administration once daily. The day of grouping was recorded as D0. During the experiment, the body weights and tumor diameters of the animals were measured three times a week. The tumor volume was calculated according to the formula V = 0.5 × a × b², where a and b represent the long diameter and short diameter of the tumor, respectively. The tumor inhibitory efficacy of the test compounds was evaluated by tumor growth inhibition rate (TGI%). 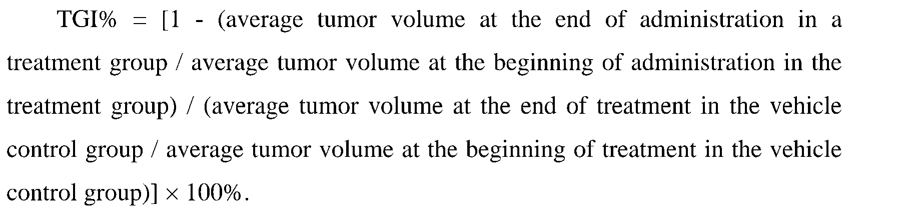 TGI% = [1 - (average tumor volume at the end of administration in a treatment group / average tumor volume at the beginning of administration in the treatment group) / (average tumor volume at the end of treatment in the vehicle control group / average tumor volume at the beginning of treatment in the vehicle control group)] × 100%.

Conclusion: After 14 days of administration, the test compounds, such as Compound 1-B, can significantly inhibit the growth of SW48 tumors, and tumor shrinkage can be observed in the dosage groups of 15 mg/kg and 50 mg/kg.

## Claims

1. A compound, or a stereoisomer, tautomer, deuterated compound, solvate, prodrug, metabolite, pharmaceutically acceptable salt, or co-crystal thereof, **characterized in that** the compound is a compound represented by general formula (I), wherein 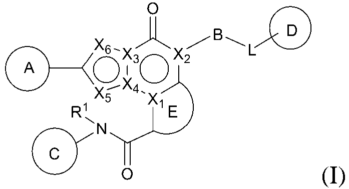
ring A is selected from C₃₋₁₅ carbocycle or 4- to 15-membered heterocycle, wherein the ring A is optionally substituted with 1 to 6 R^{a};
ring C is selected from C₃₋₁₅ carbocycle or 4- to 15-membered heterocycle, wherein the ring C is optionally substituted with 1 to 6 R^{c};
ring D is selected from C₃₋₁₅ carbocycle or 4- to 15-membered heterocycle, wherein the ring D is optionally substituted with 1 to 6 R^{d};
ring E is selected from C₄₋₁₀ carbocycle or 4- to 10-membered heterocycle, wherein the ring E is optionally substituted with 1 to 6 R^{e};
B is selected from B1 or B2, wherein
B1 is selected from 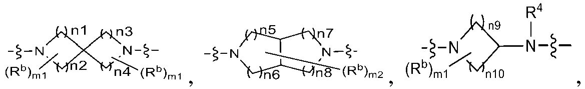 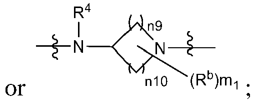 and
B2 is selected from 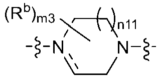 or 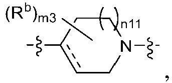 wherein
-̅ -̅ -̅ represents a single bond or a double bond;
n1, n2, n3, n4, n9, and n10 are each independently selected from 1, 2, 3, or 4;
or n1, n2, n3, and n4 are each independently selected from 0, and the sum of n1, n2, n3, and n4 is greater than 2;
n5, n6, n7, n8, and n11 are each independently selected from 0, 1, 2, 3, or 4; and
m1, m2, and m3 are each independently selected from 0, 1, 2, 3, 4, 5, 6, 7, or 8;
L is selected from -C(=O)-, -S(=O)-, -S(=O)(=NH)-, or -S(=O)₂-;
X₁, X₂, X₃, X₄, X₅, and X₆ are each independently selected from N, CH, or C, wherein at least two of X₃, X₄, X₅, and X₆ are N, and at least one of X₁ and X₂ is N;
R¹ is selected from H, deuterium, C₁₋₆ alkyl, C₃₋₆ carbocycle, or 3- to 7-membered heterocycle, wherein the alkyl, carbocycle, or heterocycle is optionally substituted with 1 to 4 R^{k};
R^{a}, R^{b}, R^{c}, R^{d}, and R^{e} are each independently selected from H, deuterium, halogen, OH, =O, CN, NH₂, SF₅, COOH, CONH₂, C₁₋₆ alkyl, OC₁₋₆ alkyl, SC₁₋₆ alkyl, C₂₋₆ alkenyl, C₂₋₆ alkynyl, NHC₁₋₆ alkyl, N(C₁₋₆ alkyl)₂, -C(=O)NH-C₁₋₆ alkyl, -C(=O)N(C₁₋₆ alkyl)₂, -O-C₃₋₆ carbocycle, -O-3- to 7-membered heterocycle, -NH-C₃₋₆ carbocycle, -NH-3- to 7-membered heterocycle, -C₀₋₄ alkylene-C₃₋₆ carbocycle, or -C₀₋₄ alkylene-3- to 7-membered heterocycle, wherein the alkyl, alkylene, alkenyl, alkynyl, carbocycle, or heterocycle is optionally substituted with 1 to 4 R^{k};
or R^{b} is selected from -C₁₋₄ alkylene-O-C₃₋₆ carbocycle, -C₁₋₄ alkylene-O-C₁₋₆ alkyl, -C₁₋₄ alkylene-O-C₁₋₄ alkylene-O-C₁₋₆ alkyl, or -C₁₋₄ alkylene-O-C₁₋₄ alkylene-O-C₃₋₆ carbocycle, wherein the alkyl, alkylene, or carbocycle is optionally substituted with 1 to 4 R^{k}; or
alternatively, two R^{b} in B2, together with the atoms or ring skeletons to which they are attached, form C₃₋₆ carbocycle or 4- to 8-membered heterocycle, wherein the carbocycle or heterocycle is optionally substituted with 1 to 4 R^{k}; and
alternatively, two R^{e}, together with the atoms to which they are attached, form C₃₋₆ carbocycle or 4- to 8-membered heterocycle, wherein the carbocycle or heterocycle is optionally substituted with 1 to 4 R^{k}; and
each R^{k} is independently selected from deuterium, halogen, =O, CN, OH, SH, NO₂, COOH, CONH₂, NH₂, SF₅, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocycle, -O-3- to 7-membered heterocycle, -NH-C₃₋₆ carbocycle, -NH-3- to 7-membered heterocycle, - C₁₋₄ alkylene-C₃₋₆ carbocycle, -C₁₋₄ alkylene-3- to 7-membered heterocycle, C₃₋₆ carbocycle, or 3- to 7-membered heterocycle, wherein the alkyl, alkylene, alkenyl, alkynyl, carbocycle, or heterocycle is optionally substituted with 1 to 4 substituents selected from deuterium, halogen, CN, OH, NH₂, C₁₋₆ alkyl, or C₁₋₆ alkoxy.

2. The compound, or the stereoisomer, tautomer, deuterated compound, solvate, prodrug, metabolite, pharmaceutically acceptable salt, or co-crystal thereof according to claim 1, **characterized in that**
ring A is selected from phenyl, 5- to 6-membered heteroaryl, C₃₋₆ monocyclic carbocycle, or 4- to 8-membered monocyclic heterocycle, wherein the ring A is optionally substituted with 1 to 5 R^{a};
ring C is selected from phenyl, naphthyl, benzo C₄₋₆ carbocycle, benzo 4- to 7-membered heterocyclyl, 5- to 6-membered heteroaryl, or 8- to 10-membered fused heteroaryl, wherein the ring C is optionally substituted with 1 to 5 R^{c};
ring D is selected from phenyl, naphthyl, benzo C₄₋₆ carbocycle, benzo 4- to 7-membered heterocyclyl, 5- to 6-membered heteroaryl, or 8- to 10-membered fused heteroaryl, wherein the ring D is optionally substituted with 1 to 5 R^{d}; 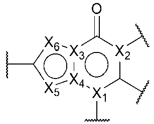
is selected from 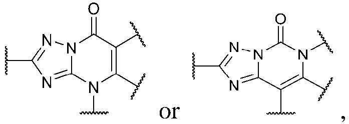 which is directly attached to ring A at the left end; 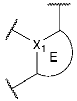
is selected from 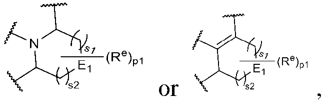 which is directly attached to X₂ at the position directly above, wherein s1 and s2 are each independently selected from 0, 1, 2, or 3;
each p1 is independently selected from 0, 1, 2, 3, or 4; and
each E₁ is independently selected from NR^{e}, C(R^{e})₂, O, S(=O)₂, or S;
R¹ is selected from H, deuterium, C₁₋₄ alkyl, or C₃₋₆ carbocycle, wherein the alkyl or carbocycle is optionally substituted with 1 to 4 R^{k}; and
alternatively, two R^{e}, together with the atoms to which they are attached, form C₃₋₆ carbocycle or 4- to 8-membered heterocycle, wherein the carbocycle or heterocycle is optionally substituted with 1 to 4 R^{k}.

3. The compound, or the stereoisomer, tautomer, deuterated compound, solvate, prodrug, metabolite, pharmaceutically acceptable salt, or co-crystal thereof according to claim 2, **characterized in that**
B2 is 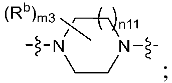
R^{a}, R^{b}, R^{c}, R^{d}, and R^{e} are each independently selected from H, deuterium, halogen, OH, =O, CN, NH₂, SF₅, COOH, CONH₂, C₁₋₄ alkyl, Oc₁₋₄ alkyl, SC₁₋₄ alkyl, C₂₋₄ alkenyl, C₂₋₄ alkynyl, NHC₁₋₄ alkyl, N(C₁₋₄ alkyl)₂, -C(=O)NH-C₁₋₄ alkyl, -C(=O)N(C₁₋₄ alkyl)₂, -O-C₃₋₆ carbocycle, -O-3- to 6-membered heterocycle, -NH-C₃₋₆ carbocycle, -NH-3- to 6-membered heterocycle, -C₀₋₂ alkylene-C₃₋₆ carbocycle, or -C₀₋₂ alkylene-3- to 6-membered heterocycle, wherein the alkyl, alkylene, alkenyl, alkynyl, carbocycle, or heterocycle is optionally substituted with 1 to 4 R^{k};
or R^{b} is selected from -C₁₋₂ alkylene-O-C₃₋₆ carbocycle, -C₁₋₂ alkylene-O-C₁₋₄ alkyl, -C₁₋₂ alkylene-O-C₁₋₂ alkylene-O-C₁₋₄ alkyl, or -C₁₋₂ alkylene-O-C₁₋₃ alkylene-O-C₃₋₆ carbocycle, wherein the alkyl, alkylene, or carbocycle is optionally substituted with 1 to 4 R^{k}; or
alternatively, two R^{b} in B2, together with the atoms or ring skeletons to which they are attached, form C₃₋₆ cycloalkyl or 4- to 7-membered heterocycloalkyl, wherein the cycloalkyl or heterocycloalkyl is optionally substituted with 1 to 4 R^{k}; and
each R^{k} is independently selected from deuterium, halogen, =O, CN, OH, SH, NO₂, COOH, CONH₂, NH₂, SF₅, C₁₋₄ alkyl, OC₁₋₄ alkyl, SC₁₋₄ alkyl, C₂₋₄ alkenyl, C₂₋₄ alkynyl, NHC₁₋₄ alkyl, N(C₁₋₄ alkyl)₂, -O-C₃₋₆ carbocycle, -O-3- to 6-membered heterocycle, -NH-C₃₋₆ carbocycle, -NH-3- to 6-membered heterocycle, - C₁₋₂ alkylene-C₃₋₆ carbocycle, -C₁₋₂ alkylene-3- to 6-membered heterocycle, C₃₋₆ carbocycle, or 3- to 6-membered heterocycle, wherein the alkyl, alkylene, alkenyl, alkynyl, carbocycle, or heterocycle is optionally substituted with 1 to 4 substituents selected from deuterium, halogen, CN, OH, NH₂, C₁₋₆ alkyl, or C₁₋₆ alkoxy.

4. The compound, or the stereoisomer, tautomer, deuterated compound, solvate, prodrug, metabolite, pharmaceutically acceptable salt, or co-crystal thereof according to claim 3, **characterized in that** 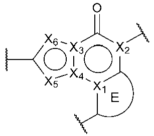
is 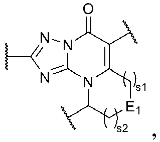 which is directly attached to ring A at the left end, wherein s1 and s2 are each independently selected from 0, 1, or 2;
L is -C(=O)-;
R¹ is selected from H, methyl, ethyl, or cyclopropyl;
R^{a}, R^{b}, R^{c}, R^{d}, and R^{e} are each independently selected from H, deuterium, F, Cl, Br, I, OH, =O, CN, NH₂, SF₅, COOH, CONH₂, NHCH₃, N(CH₃)₂, or one of the following groups optionally substituted with 1 to 4 R^{k}: methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl;
or R^{b} is selected from -methylene-O-cyclopropyl, -ethylene-O-methyl, - methylene-O-methyl, -methylene-O-ethyl, -methylene-O-ethylene-O-methyl, - methylene-O-ethylene-O-ethyl, -methylene-O-ethylene-O-propyl, -methylene-O-ethylene-O-cyclopropyl, -methylene-O-methylene-O-cyclopropyl, or -methylene-O-ethylene-O-cyclobutyl, wherein the methylene, ethylene, methyl, ethyl, propyl, cyclopropyl, or cyclobutyl is optionally substituted with 1 to 4 R^{k}; or
alternatively, two R^{b} in B2, together with the carbon atoms to which they are directly attached, form cyclopropyl, cyclobutyl, cyclopentyl, cyclohexyl, azetidinyl, azacyclopentyl, or azacyclohexyl, wherein the cyclopropyl, cyclobutyl, cyclopentyl, cyclohexyl, azetidinyl, azacyclopentyl, or azacyclohexyl is optionally substituted with 1 to 4 R^{k}; and
alternatively, two R^{e}, together with the carbon atoms to which they are attached, form cyclopropyl, cyclobutyl, cyclopentyl, cyclohexyl, azetidinyl, azacyclopentyl, azacyclohexyl, or oxetanyl, wherein the cyclopropyl, cyclobutyl, cyclopentyl, cyclohexyl, azetidinyl, azacyclopentyl, azacyclohexyl, or oxetanyl is optionally substituted with 1 to 4 R^{k}; and
each R^{k} is independently selected from deuterium, F, Cl, Br, I, =O, CN, OH, SH, NO₂, COOH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl, wherein the methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl is optionally substituted with 1 to 4 substituents selected from deuterium, F, Cl, Br, I, CN, OH, NH₂, C₁₋₄ alkyl, or C₁₋₄ alkoxy.

5. The compound, or the stereoisomer, tautomer, deuterated compound, solvate, prodrug, metabolite, pharmaceutically acceptable salt, or co-crystal thereof according to claim 4, **characterized in that**
ring A is selected from one of the following groups optionally substituted with 1 to 4 R^{a}: cyclopropyl, cyclobutyl, cyclopentyl, cyclohexyl, cyclopropenyl, cyclobutenyl, cyclopentenyl, cyclohexenyl, oxetanyl, azetidinyl, pyrrolidinyl, piperidinyl, piperazinyl, morpholinyl, tetrahydrofuryl, tetrahydropyranyl, dihydropyrrolyl, dihydropyridyl, dihydrofuryl, dihydropyranyl, phenyl, pyrrolyl, thienyl, furyl, pyrazolyl, imidazolyl, thiazolyl, isothiazolyl, oxazolyl, isoxazolyl, pyridyl, pyrimidinyl, pyrazinyl, or pyridazinyl;
ring C is selected from one of the following groups optionally substituted with 1 to 4 R^{c}: phenyl, pyrrolyl, thienyl, furyl, pyrazolyl, imidazolyl, thiazolyl, isothiazolyl, oxazolyl, isoxazolyl, pyridyl, pyrimidinyl, pyrazinyl, pyridazinyl, naphthyl, benzopyrrolyl, benzothienyl, benzofuryl, benzopyrazolyl, benzimidazolyl, benzothiazolyl, benzisothiazolyl, benzoxazolyl, benzisoxazolyl, benzopyridyl, benzopyrimidinyl, benzopyrazinyl, benzopyridazinyl, pyridopyrrolyl, pyridothienyl, pyridofuryl, pyridopyrazolyl, pyridoimidazolyl, pyridothiazolyl, pyridoisothiazolyl, pyridooxazolyl, pyridoisoxazolyl, pyridopyridyl, pyridopyrimidinyl, pyridopyrazinyl, pyridopyridazinyl, 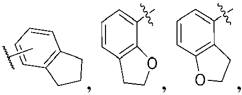
or 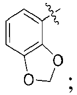
ring D is selected from one of the following groups optionally substituted with 1 to 4 R^{d}: phenyl, pyrrolyl, thienyl, furyl, pyrazolyl, imidazolyl, thiazolyl, isothiazolyl, oxazolyl, isoxazolyl, pyridyl, pyrimidinyl, pyrazinyl, pyridazinyl, triazinyl, benzopyrrolyl, benzothienyl, benzofuryl, benzopyrazolyl, benzimidazolyl, benzothiazolyl, pyridopyrrolyl, pyridothienyl, pyridofuryl, pyridopyrazolyl, pyridoimidazolyl, or pyridothiazolyl;
B2 is selected from 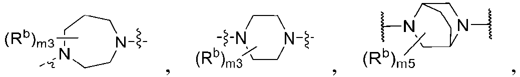 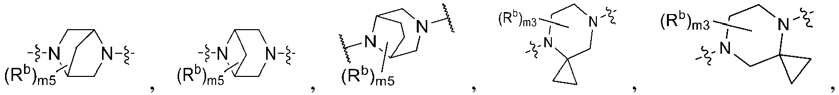 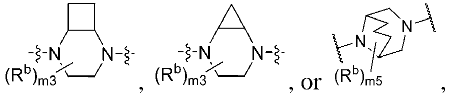 wherein
m5 is selected from 0, 1, 2, 3, 4, 5, or 6;
B1 is selected from 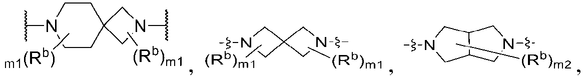 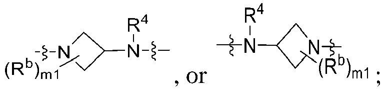
or B1 is selected from 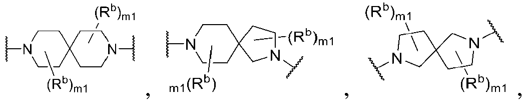 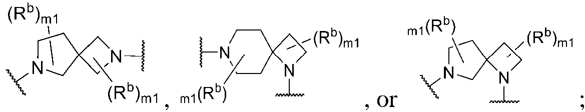 and
each R^{k} is independently selected from deuterium, F, Cl, Br, I, =O, CN, OH, SH, NO₂, COOH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl, wherein the methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, or morpholinyl is optionally substituted with 1 to 4 substituents selected from deuterium, F, Cl, Br, I, CN, OH, NH₂, methyl, ethyl, methoxy, or ethoxy.

6. The compound, or the stereoisomer, tautomer, deuterated compound, solvate, prodrug, metabolite, pharmaceutically acceptable salt, or co-crystal thereof according to claim 5, **characterized in that** 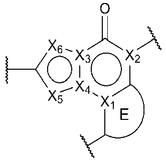
is selected from 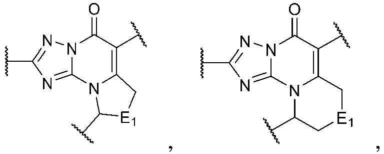 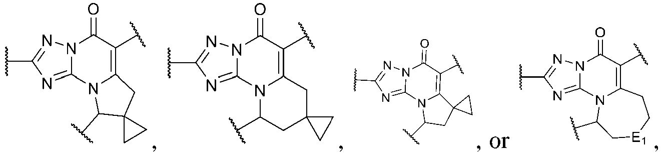 which is directly attached to ring A at the left end, wherein
each E₁ is independently selected from NH, CH₂, C(R^{e})₂, O, S(=O)₂, or S;
ring A is selected from one of the following groups optionally substituted with 1 to 3 R^{a}: 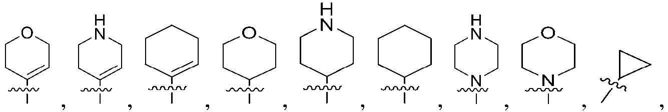 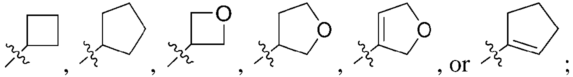
ring C is selected from one of the following groups optionally substituted with 1 to 4 R^{c}: 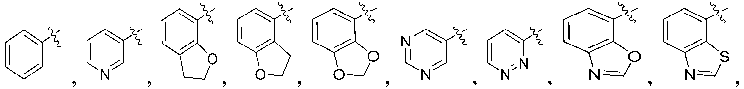 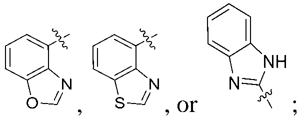
or ring C is selected from one of the following groups optionally substituted with 1 to 3 R^{c}: 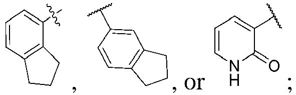
ring D is selected from one of the following groups optionally substituted with 1 to 3 R^{d}: 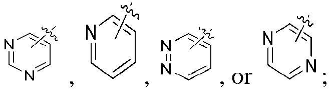
B is selected from 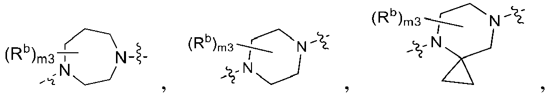 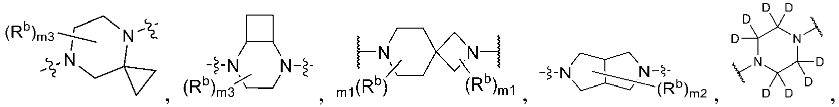 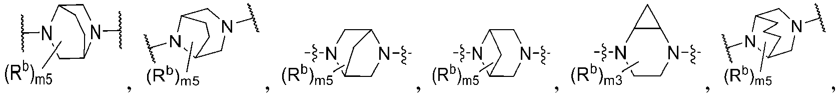 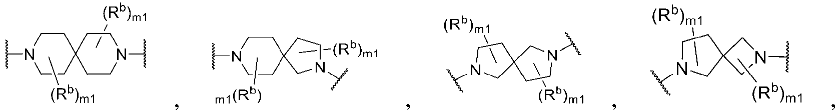 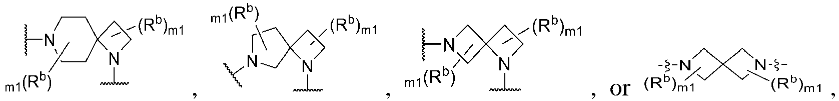 wherein m3 is selected from 0, 1, 2, 3, or 4;
m5 is selected from 0, 1, 2, 3, or 4; and
m1 and m2 are each independently selected from 0, 1, 2, 3, or 4;
each R^{e} is independently selected from H, deuterium, F, Cl, Br, I, OH, CN, NH₂, SF₅, COOH, CONH₂, NHCH₃, N(CH₃)₂, CD₃, CHF₂, CF₃, CH₂F, OCHF₂, OCF₃, SCF₃, OCH₂F, CH₂CN, methyl, ethyl, propyl, isopropyl, methoxy, ethoxy, methylthio, vinyl, ethynyl, propynyl, propargyl, cyclopropyl, cyclobutyl, azetidinyl, or oxetanyl; and
R^{a}, R^{b}, R^{c}, and R^{d} are each independently selected from H, deuterium, F, Cl, Br, I, OH, =O, CN, NH₂, SF₅, COOH, CONH₂, NHCH₃, N(CH₃)₂, CD₃, CHF₂, CF₃, CH₂F, CHF₂CF₃, CHF₂CH₃, OCHF₂, OCF₃, SCF₃, OCH₂F, CH₂CN, CH₂OH, methyl, ethyl, propyl, isopropyl, methoxy, ethoxy, methylthio, vinyl, ethynyl, propynyl, propargyl, cyclopropyl, cyclobutyl, azetidinyl, oxetanyl, pyrrolidinyl, piperidinyl, pyrazolyl, pyrrolyl, morpholinyl, 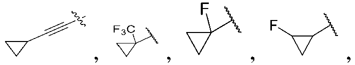 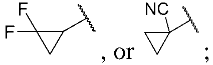
or R^{b} is selected from -CH₂-O-cyclopropyl, -CH₂-OCH₃, -CH₂-O-CH₂CH₂OCH₃, -CH₂OCH₂CH₂OCH₂CH₂CH₂OCH₃, or -CH₂-O-CH₂CH₂O-cyclopropyl.

7. The compound, or the stereoisomer, tautomer, deuterated compound, solvate, prodrug, metabolite, pharmaceutically acceptable salt, or co-crystal thereof according to claim 1, **characterized in that** the compound has a structure selected from one of those in Table E-1.

8. A pharmaceutical composition comprising the compound, or the stereoisomer, tautomer, deuterated compound, solvate, prodrug, metabolite, pharmaceutically acceptable salt, or co-crystal thereof according to any one of claims 1-7, and a pharmaceutically acceptable carrier, **characterized in that** preferably, the pharmaceutical composition comprises 1-1500 mg of the compound, or the stereoisomer, tautomer, deuterated compound, solvate, prodrug, metabolite, pharmaceutically acceptable salt, or co-crystal thereof according to any one of claims 1-5.

9. Use of the compound, or the stereoisomer, tautomer, deuterated compound, solvate, prodrug, metabolite, pharmaceutically acceptable salt, or co-crystal thereof according to any one of claims 1-7, or the pharmaceutical composition according to claim 8 in the preparation of a medicament for treating a disease associated with WRN.

10. Use of the compound, or the stereoisomer, tautomer, deuterated compound, solvate, prodrug, metabolite, pharmaceutically acceptable salt, or co-crystal thereof according to any one of claims 1-7, or the pharmaceutical composition according to claim 8 in the preparation of a medicament for treating a tumor, preferably an MSI-H solid tumor or an MMRD solid tumor.

11. A method for treating a disease in a mammal, comprising administering to the subject a therapeutically effective amount of the compound, or the stereoisomer, tautomer, deuterated compound, solvate, prodrug, metabolite, pharmaceutically acceptable salt, or co-crystal thereof according to any one of claims 1-7, **characterized in that** the therapeutically effective amount is preferably 1-1500 mg, and the disease is preferably a tumor, further preferably an MSI-H solid tumor or an MMRD solid tumor.
